# EUROPEAN PATENT SPECIFICATION

(11) **EP 2 794 862 B1**
(45) Date of publication and mention of the grant of the patent: **07.03.2018**
(21) Application number: 12808653.5
(22) Date of filing: 19.12.2012
(51) Int. Cl.: C12N 9/00, C12N 9/04, C12N 9/10, C12N 9/12, C12N 9/92, C12P 7/06, C12P 7/10, C12N 1/21, C12N 15/74

(54) **GENE INACTIVATION ALLOWING IMMEDIATE GROWTH ON XYLOSE MEDIUM BY ENGINEERED ZYMOMONAS**
GENINAKTIVIERUNG FÜR UNMITTELBARES WACHSTUM AUF XYLOSE-MEDIUM DURCH MANIPULIERTE ZYMOMONA-BAKTERIEN
INACTIVATION DE GÈNES PERMETTANT LA CROISSANCE IMMÉDIATE SUR MILIEU XYLOSÉ PAR DES ZYMOMONAS GÉNÉTIQUEMENT MODIFIÉS

(30) Priority: 20.12.2011 US 201161577879 P
(43) Date of publication of application: 29.10.2014
(73) Proprietor: E. I. du Pont de Nemours and Company, Wilmington, DE 19805 (US)
(72) Inventor: HITZ, William, D., Wilmington, Delaware 19807 (US); QI, Min, Hockessin, Delaware 19707 (US); TAO, Luan, Wallingford, Pennsylvania 19086 (US); VIITANEN, Paul, V., West Chester, Pennsylvania 19382 (US); YANG, Jianjun, Hockessin, Delaware 19707 (US)
(74) Representative: Jones, Elizabeth Louise
(86) International application number: PCT/US2012/070460
(87) International publication number: WO 2013/096366

(56) References cited:
- WO-A2-01/83786
- WO-A2-2008/133638
- WO-A2-2009/058938
- WO-A2-2009/120730
- WO-A2-2009/120731
- WO-A2-2009/132201
- US-A1- 2011 318 801
- US-A1- 2012 196 342
- MANOJ AGRAWAL ET AL: "Discovery and characterization of a xylose reductase fromZM4", BIOTECHNOLOGY LETTERS, SPRINGER NETHERLANDS, DORDRECHT, vol. 33, no. 11, 1 July 2011 (2011-07-01), pages 2127-2133, XP019957903, ISSN: 1573-6776, DOI: 10.1007/S10529-011-0677-6 cited in the application
- MANOJ AGRAWAL ET AL: "Adaptation yields a highly efficient xylose-fermenting Zymomonas mobilis strain", BIOTECHNOLOGY AND BIOENGINEERING, vol. 108, no. 4, 1 April 2011 (2011-04-01) , pages 777-785, XP055037845, ISSN: 0006-3592, DOI: 10.1002/bit.23021
- AMORE R ET AL: "THE FERMENTATION OF XYLOSE - AN ANALYSIS OF THE EXPRESSION OF BACILLUS AND ACTINOPLANES XYLOSE ISOMERASE GENES IN YEAST", APPLIED MICROBIOLOGY AND BIOTECHNOLOGY, SPRINGER VERLAG, BERLIN, DE, vol. 30, 1 January 1989 (1989-01-01), pages 351-357, XP001011493, ISSN: 0175-7598, DOI: 10.1007/BF00296623
- DATABASE UniProt [Online] 1 February 2005 (2005-02-01), "SubName: Full=Aldo/keto reductase;", XP002683524, retrieved from EBI accession no. UNIPROT:Q5NNW0 Database accession no. Q5NNW0 -& SEO JEONG-SUN ET AL: "The genome sequence of the ethanologenic bacterium Zymomonas mobilis ZM4", NATURE BIOTECHNOLOGY, NATURE PUBLISHING GROUP, NEW YORK, NY, US, vol. 23, no. 1, 1 January 2005 (2005-01-01), pages 63-68, XP002561270, ISSN: 1087-0156, DOI: 10.1038/NBT1045
- MANOJ AGRAWAL ET AL: "Engineering efficient xylose metabolism into an acetic acid-tolerant strain by introducing adaptation-induced mutations", BIOTECHNOLOGY LETTERS, SPRINGER NETHERLANDS, DORDRECHT, vol. 34, no. 10, 6 June 2012 (2012-06-06), pages 1825-1832, XP035131441, ISSN: 1573-6776, DOI: 10.1007/S10529-012-0970-Z

## Description

This application claims the benefit of United States Provisional Application 61/577879, filed December 20, 2011.

### FIELD OF THE INVENTION

The invention relates to the fields of microbiology and genetic engineering. More specifically, inactivation of a gene in the *Zymomonas* genome that is annotated as encoding an aldo/keto reductase was found to allow immediate growth on xylose by *Zymomonas* engineered to express a xylose utilization pathway without a xylose-adaptation step.

### BACKGROUND OF THE INVENTION

Production of ethanol by microorganisms provides an alternative energy source to fossil fuels and is therefore an important area of current research. It is desirable that microorganisms producing ethanol, as well as other useful products, be capable of using xylose as a carbon source since xylose is the major pentose in hydrolyzed lignocellulosic biomass. Biomass can provide an abundantly available, low cost carbon substrate. *Zymomonas mobilis* and other bacterial ethanologens which do not naturally utilize xylose have been genetically engineered for xylose utilization by introduction of genes encoding 1) xylose isomerase, which catalyses the conversion of xylose to xylulose; 2) xylulokinase, which phosphorylates xylulose to form xylulose 5-phosphate; 3) transketolase; and 4) transaldolase (US 5514583, US 5712133, US 6566107, WO 95/28476, Feldmann et al. (1992) Appl. Microbiol. Biotechnol. 38: 354-361, Zhang et al. (1995) Science 267:240-243; Yanase et al. (2007) Appl. Environ. Mirobiol. 73:2592-2599). Typically the coding regions used were from *E. coli* genes.

Even with expression of this xylose utilization pathway however, engineered strains of *Zymomonas* usually require an adaptation period in xylose-containing medium before they are able to grow on xylose when it is the sole carbon source. Strains engineered for expression of the xylose utilization pathway have been adapted by serial passage on xylose-containing medium, resulting in strains with improved xylose utilization as described in U. S. Pat. 7,223,575 and U. S. Pat. 7,741,119. A similar technique was also used in WO 2009/132201 to produce mutant *Zymomonas mobilis* strains capable of increased xylose fermentation. Disclosed in U.S. Pat. 7,989,206 is the finding that during adaptation, the *Zymomonas mobilis* glyceraldehyde-3-phosphate dehydrogenase gene promoter expressing *E. coli* xylose isomerase was mutated to a more active form that increased the level of xylose isomerase activity, which had previously been the rate-limiting enzyme for xylose metabolism.

U. S. Pat. 7,741,119 also discloses improved xylose utilization by inactivation of the *gfor* locus encoding glucose-fructose oxidoreductase, an enzyme that is able to generate xylitol when glucose and xylulose are both available. Thus, in growth media that contained glucose and xylose, about 3-fold more xylitol was produced by *Zymomonas* cells that express xylose isomerase, which converts xylose to xylulose, as compared to cells lacking xylose isomerase (US 7,741,119). Moreover, this increase did not occur when the GFOR gene was inactivated, thus demonstrating the importance of this enzyme in the production of xylitol *in vivo.*

It has been established using cell-free extracts that a wild type strain of *Z. mobilis* (CP4) has NADPH-dependent aldose reductase activity that can directly convert D-xylose to xylitol (Feldmann et al. Appl. Microbiol. Biotechnol. (1992) 38:354-361). Another wild type strain of *Z. mobilis* (ZM4) was also reported to have NADPH-dependent aldose reductase activity (Agrawal et al. 2011 108:777-785). In that study a plasmid that contained all four genes that are required for xylose metabolism was introduced into ZM4, and the resulting transformants could *not* grow on xylose without an adaptation step. The adapted strain that resulted from this procedure (A1) was able to grow on xylose, but only very slowly. To improve xylose utilization, A1 was further adapted in a process that took 80 days and 30 serial transfers with xylose as sole carbon source. The new adapted strain (A3) grew better on xylose than the A1 parent. It also produced less xylitol, was more resistant to xylitol, and had higher xylose isomerase activity. Thus at least three different mutations took place during the evolution of the A3 strain. Although the number of mutations that occurred during the first adaptation period that resulted in the A1 strain, (which was able to grow on xylose, albeit poorly), was not determined, the authors noted that both adapted strains, A1 and A3, had "barely detectable" NADPH-dependent aldose reductase activities compared to wild type ZM4 carrying an empty plasmid. It was subsequently reported that the A3 strain has a point mutation in the coding region of the ZMO0976 gene, which codes for an enzyme that has NADPH-dependent xylose reductase activity that is able to convert xylose to xylitol (Agrawal and Chen (2011) Biotechnol. Lett. 33:2127-2133). The purified mutant protein has < 5% of the activity of the wild type enzyme, based on expression in *E. coli.* It was also shown in the same study that benzaldehyde and furfural are better substrates for the ZMO0976 gene product than xylose.

There remains a need for engineered strains of *Zymomonas* and other bacterial ethanolagens that contain the genes for xylose utilization and are able to grow on media that contains xylose as sole carbon source without a preliminary adaptation step, and processes for using these strains to produce ethanol.

### SUMMARY OF THE INVENTION

Disclosed herein are *Zymomonas* cells that are genetically engineered to be competent to grow on medium containing xylose as the only sugar without adaptation, and methods for making said cells and for using said cells for ethanol production.

Accordingly, the invention provides a method of making a xylose-competent *Zymomonas* cell comprising:
a) providing a *Zymomonas* host cell;
b) creating a genetic modification in said cell in at least one endogenous gene encoding an aldose reductase enzyme of EC 1.1.1.21 having the ability to convert xylose to xylitol in the presence of NADPH; and
c) introducing into said cell a xylose utilization metabolic pathway; wherein the order of steps (b) and (c) is not specified, and optionally occur concurrently, wherein said xylose-competent *Zymomonas* cell has aldose reductase activity reduced by greater than 90% as compared with a *Zymomonas* cell lacking the genetic modification of step (b), wherein said genetic modification is not created in a step of adaptation for growth in xylose by prolonged growth in medium containing xylose, and wherein said xylose-competent *Zymomonas* cell is able to grow in medium containing xylose as the only sugar.

Additionally the invention provides a method for producing ethanol comprising growing a xylose-competent and ethanol-producing *Zymomonas* cell produced by the method of the invention under conditions wherein ethanol is produced and optionally collecting said ethanol.

### BRIEF DESCRIPTION OF THE DRAWINGS AND SEQUENCE DESCRIPTIONS

Figure 1 shows a diagram of metabolic pathways for xylose utilization and ethanol production.
Figure 2 shows a diagram of the first two steps of the engineered xylose utilization pathway (boxed), xylitol synthesis, xylitol 5-phosphate formation (a toxic dead-end intermediate), and inhibition of xylose isomerase by xylitol.
Figure 3 shows plasmid maps of pZX21 (A), pZX52 (B), and pZX6 (C).
Figure 4 shows graphs of growth, xylose used, and ethanol produced for cultures grown in mRM3-X10 of ZW1-109 (A), ZW1-210 (B), and control ZW1 (C).
Figure 5 shows a plasmid map of pMODlinker-Cm.
Figure 6 (A) shows a plasmid map of pAR-cm and (B) shows a diagram of primer binding sites in a portion of the ZW658 chromosome that includes the *ZMO0976* ORF.
Figure 7 shows a diagram of a region of the ZW1 chromosome that includes the *ZMO0976* ORF following a double-crossover event with the pAR-cm suicide construct, with primer binding sites and PCR products marked.
Figure 8 shows a graph of xylitol production by two ZMO0976 knockout mutants (AR1, AR2) and the control wild type *Z. mobilis* ZW1.
Figure 9 shows a graph of growth in medium containing 100 g/L of xylose as the only sugar, monitored by OD600, of *Z. mobilis* strains engineered to express a xylose utilization pathway (on pZB4) either with knockout of the *ZMO0976* gene (AR1; three isolates: -1, -2, -3) or with the native *ZMO0976* gene (ZW1; three isolates: -A, -B, -C). The filled symbols are all overlapping.
Figure 10 shows a graph of growth in medium containing 100 g/L of xylose as the only sugar, monitored by OD600, of eight *Z. mobilis* isolates engineered to express a xylose utilization pathway (on pZB4) after adaptation in medium containing 45 g/L xylose and 5 g/L glucose, and then in medium that contained 100 g/L xylose as the only sugar. The three types of adapted mutant strains that were isolated (Groups #1-3) are indicated by the circles.

The invention can be more fully understood from the following detailed description and the accompanying sequence descriptions which form a part of this application.

The following sequences conform with 37 C.F.R. 1.821-1.825 ("Requirements for Patent Applications Containing Nucleotide Sequences and/or Amino Acid Sequence Disclosures - the Sequence Rules") and are consistent with World Intellectual Property Organization (WIPO) Standard ST.25 (2009) and the sequence listing requirements of the EPO and PCT (Rules 5.2 and 49.5(a-bis), and Section 208 and Annex C of the Administrative Instructions). The symbols and format used for nucleotide and amino acid sequence data comply with the rules set forth in 37 C.F.R. §1.822.
SEQ ID NO:1 is the nucleotide sequence of the *ZMO0976* coding region.
SEQ ID NO:2 is the amino acid sequence of the protein encoded by the *ZMO0976* coding region.
SEQ ID NO:3 is the nucleotide sequence of the *Zm*P*gap* from the CP4 strain of Z. mobilis.
SEQ ID NO:4 is the nucleotide sequence of the improved P*gap* from strain ZW658 which is called the 801 GAP promoter or the Super GAP promoter or P_{gapS}.
SEQ ID NO:5 is the nucleotide sequence of the improved P*gap* from strain 8b.
SEQ ID NO:6 is the nucleotide sequence of an improved P*gap* with both position 116 (ZW658) and position 217 (8b) mutations in the pZB4 variant of *Pgap.*
SEQ ID NO:7 is the nucleotide sequence of an improved P*gap* with the position 116 mutation from ZW658 in the CP4 variant of P*gap.*
SEQ ID NO:8 is the nucleotide sequence of an improved P*gap* with the position 217 mutation from 8b in the CP4 variant of P*gap.*
SEQ ID NO:9 is the nucleotide sequence of an improved *Pgap* with both position 116 (ZW658) and position 217 (8b) mutations in the CP4 variant of P*gap.*
SEQ ID NO:10 is the nucleotide sequence of an improved P*gap* with the position 116 mutation from ZW658 in the ZM4 variant of *Pgap.*
SEQ ID NO:11 is the nucleotide sequence of an improved P*gap* with the position 217 mutation from 8b in the ZM4 variant of P*gap.*
SEQ ID NO:12 is the nucleotide sequence of an improved P*gap* with both position 116 (ZW658) and position 217 (8b) mutations in the ZM4 variant of P*gap.*
SEQ ID NO:13 is the coding region for the *Actinoplanes missourinesis* xylose isomerase that was codon optimized for *Zymomonas.*
SEQ ID NO:14 is the nucleotide sequence of the *ZMO0976* coding region with a 78 nucleotide deletion from strains ZW641 and ZW658.
SEQ ID NO:15 is the nucleotide sequence of plasmid pZX21.
SEQ ID NO:16 is the nucleotide sequence of the GFO-L fragment.
SEQ ID NO:17 is the nucleotide sequence of the gfor coding sequence.
SEQ ID NO:18 is the nucleotide sequence of the GFO-R fragment.
SEQ ID NO:19 is the nucleotide sequence of a 1,661-bp chimeric xylA gene containing the 304-bp *Z. mobilis* Super GAP promoter, a 1,185-bp *A. missouriensis* xylA coding sequence, and a 166-bp *E. coli* araD 3'UTR with a 5' Xbal site.
SEQ ID NO:20 is the nucleotide sequence of a 1,960-bp chimeric xylB gene containing a 191 bp Pₑₙₒ, a 1,455-bp *E. coli xyl*B coding sequence and a 314-bp *E.coli* xylB 3'UTR.
SEQ ID NO:21 is the nucleotide sequence of a 1,014 bp aadA marker (for spectinomycin resistance; Spec-R) bounded by lox sites.
SEQ ID NO:22 is the nucleotide sequence of shuttle vector pZX52.
SEQ ID NO:23 is the nucleotide sequence of the LDH-L fragment.
SEQ ID NO:24 is the nucleotide sequence of the LDH-R fragment.
SEQ ID NO:25 is the nucleotide sequence of the IdhA coding sequence.
SEQ ID NO:26 is the nucleotide sequence of a 3,339 bp P_{gapT}-Tal-Tkt operon containing a 304-bp T-mutant of the *Z. mobilis* GAP promoter (P_{gapT}), a 954-bp *E. coli* Tal coding region, a 1,992-bp *E. coli Tkt* coding region, and a 68-bp *E. coli Tkt* 3'UTR.
SEQ ID NO:27 is the nucleotide sequence of the P_{gapT} promoter.
SEQ ID NO:28 is the nucleotide sequence of a 1,443 bp Pₑₙₒ-Rpi-Rpe operon containing a 191 bp Pₑₙₒ, a 471 bp *Z. mobilis* Rpi coding sequence, a 663 bp *Z. mobilis* Rpe coding sequence, and a 35 bp *E.coli* xylA 3'UTR.
SEQ ID NO:29 is the nucleotide sequence of the DCO shuttle vector pZX6.
SEQ ID NO:30 is the nucleotide sequence of the PNP-L fragment.
SEQ ID NO:31 is the nucleotide sequence of the PNP-R fragment.
SEQ ID NOs:32 to 41 are PCR primers.
SEQ ID NO:42 is the nucleotide sequence of the pnp coding region from *Zymomonas mobilis* strain ZM4.
SEQ ID NO:43 is the nucleotide sequence of the coding region for the *Z. mobilis* RPI protein with the start codon mutated to ATG.
SEQ ID NOs:44 and 45 are primers.
SEQ ID NO:46 is the nucleotide sequence of Fragment 2 containing a Cm^{r}-cassette that is flanked by two wild type loxP sites
SEQ ID NOs:47 to 72 are primers.

### DETAILED DESCRIPTION

The following definitions may be used for the interpretation of the claims and specification:
As used herein, the terms "comprises," "comprising," "includes," "including," "has," "having," "contains" or "containing," or any other variation thereof, are intended to cover a non-exclusive inclusion. For example, a composition, a mixture, process, method, article, or apparatus that comprises a list of elements is not necessarily limited to only those elements but may include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

The term "invention" or "present invention" as used herein is a nonlimiting term and is not intended to refer to any single embodiment of the particular invention but encompasses all possible embodiments as described in the specification and the claims.

As used herein, the term "about" modifying the quantity of an ingredient or reactant of the invention employed refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the compositions or carry out the methods; and the like. The term "about" also encompasses amounts that differ due to different equilibrium conditions for a composition resulting from a particular initial mixture. Whether or not modified by the term "about", the claims include equivalents to the quantities. In one embodiment, the term "about" means within 10% of the reported numerical value, preferably within 5% of the reported numerical value.

"Gene" refers to a nucleic acid fragment that expresses a specific protein or functional RNA molecule, which may optionally include regulatory sequences preceding (5' non-coding sequences) and following (3' non-coding sequences) the coding sequence. "Native gene" or "wild type gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign" gene refers to a gene not normally found in the host organism, but that is introduced into the host organism by gene transfer. Foreign genes can comprise native genes inserted into a non-native organism, or chimeric genes.

"Promoter" or "Initiation control regions" refers to a DNA sequence capable of controlling the expression of a coding sequence or functional RNA. In general, a coding sequence is located 3' to a promoter sequence. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions. Promoters which cause a gene to be expressed in most cell types at most times are commonly referred to as "constitutive promoters".

The term "expression", as used herein, refers to the transcription and stable accumulation of coding (mRNA) or functional RNA derived from a gene. Expression may also refer to translation of mRNA into a polypeptide. "Overexpression" refers to the production of a gene product in transgenic organisms that exceeds levels of production in normal or non-transformed organisms.

The term "transformation" as used herein, refers to the transfer of a nucleic acid fragment into a host organism, resulting in genetically stable inheritance. The transferred nucleic acid may be in the form of a plasmid maintained in the host cell, or some transferred nucleic acid may be integrated into the genome of the host cell. Host organisms containing the transformed nucleic acid fragments are referred to as "transgenic" or "recombinant" or "transformed" organisms.

The terms "plasmid" and "vector" as used herein, refer to an extra chromosomal element often carrying genes which are not part of the central metabolism of the cell, and usually in the form of circular double-stranded DNA molecules. Such elements may be autonomously replicating sequences, genome integrating sequences, phage or nucleotide sequences, linear or circular, of a single- or double-stranded DNA or RNA, derived from any source, in which a number of nucleotide sequences have been joined or recombined into a unique construction which is capable of introducing a promoter fragment and DNA sequence for a selected gene product along with appropriate 3' untranslated sequence into a cell.

The term "operably linked" refers to the association of nucleic acid sequences on a single nucleic acid fragment so that the function of one is affected by the other. For example, a promoter is operably linked with a coding sequence when it is capable of affecting the expression of that coding sequence (i.e., that the coding sequence is under the transcriptional control of the promoter). Coding sequences can be operably linked to regulatory sequences in sense or antisense orientation.

The term "selectable marker" means an identifying factor, usually an antibiotic or chemical resistance gene, that is able to be selected for based upon the marker gene's effect, i.e., resistance to an antibiotic, wherein the effect is used to track the inheritance of a nucleic acid of interest and/or to identify a cell or organism that has inherited the nucleic acid of interest.

As used herein the term "codon degeneracy" refers to the nature in the genetic code permitting variation of the nucleotide sequence without affecting the amino acid sequence of an encoded polypeptide. The skilled artisan is well aware of the "codon-bias" exhibited by a specific host cell in usage of nucleotide codons to specify a given amino acid. Therefore, when synthesizing a gene for improved expression in a host cell, it is desirable to design the gene such that its frequency of codon usage approaches the frequency of preferred codon usage of the host cell.

The term "codon-optimized" as it refers to genes or coding regions of nucleic acid molecules for transformation of various hosts, refers to the alteration of codons in the gene or coding regions of the nucleic acid molecules to reflect the typical codon usage of the host organism without altering the polypeptide encoded by the DNA.

The term "genetic modification" refers, non-inclusively, to any modification, mutation, base deletion, base addition, codon modification, gene over-expression, gene suppression, promoter modification or substitution, gene addition (either single or multicopy), antisense expression or suppression, or any other change to the genetic elements of a host cell or bacterial strain, whether they produce a change in phenotype or not.

The term "recombinant bacterial host cell" refers to a bacterial cell that comprises at least one heterologus gene or genetic construct or nucleic acid fragment.

The term "NADPH-dependent xylose reductase activity" refers to aldose reductase activity for conversion of xylose to xylitol in the presence of NADPH.

The term "xylose-competent *Zymomonas* cell" refers to a *Zymomonas* cell that is able to grow in medium containing xylose as the only sugar, without being adapted for growth on xylose.

The term "adapted for growth on xylose" refers to a cell or strain isolated after prolonged growth in medium containing xylose. Adaptation may include a period of growth in medium containing xylose and glucose, and then a period of growth in medium containing only xylose, each medium being a xylose-containing medium. Typically the prolonged period of growth is at least about four days.

The term "xylose metabolic pathway" or "xylose utilization metabolic pathway" refers to a series of enzymes (encoded by genes) that metabolize xylose through to fructose-6-phosphate and/or glyceraldehyde- 6- phosphate and include 1) xylose isomerase, which catalyses the conversion of xylose to xylulose; 2) xylulokinase, which phosphorylates xylulose to form xylulose 5-phosphate; 3) transketolase; and 4) transaldolase.

The term "xylose isomerase" refers to an enzyme that catalyzes the interconversion of D-xylose and D-xylulose. Xylose isomerases (XI) belong to the group of enzymes classified as EC 5.3.1.5.

The term "ribose-5-phosphate isomerase" or "RPI" refers to an enzyme that catalyzes the interconversion of ribulose-5-phosphate and ribose-5-phosphate. Ribose-5-phosphate isomerases belong to the group of enzymes classified as EC 5.3.1.6.

The term "ribulose-phosphate 3-epimerase" or "RPE" refers to an enzyme catalyzes the interconversion of D-ribulose 5-phosphate and D-xylulose 5-phosphate and is classified as EC 5.1.3.1.

The term "carbon substrate" or "fermentable carbon substrate" refers to a carbon source capable of being metabolized by microorganisms. A type of carbon substrate is "fermentable sugars" which refers to oligosaccharides and monosaccharides that can be used as a carbon source by a microorganism in a fermentation process.

The term "lignocellulosic" refers to a composition comprising both lignin and cellulose. Lignocellulosic material may also comprise hemicellulose.

The term "cellulosic" refers to a composition comprising cellulose and additional components, including hemicellulose.

The term "saccharification" refers to the production of fermentable sugars from polysaccharides.

The term "pretreated biomass" means biomass that has been subjected to thermal, physical and/or chemical pretreatment to increase the availability of polysaccharides in the biomass to saccharification enzymes.

"Biomass" refers to any cellulosic or lignocellulosic material and includes materials comprising cellulose, and optionally further comprising hemicellulose, lignin, starch, oligosaccharides and/or monosaccharides. Biomass may also comprise additional components, such as protein and/or lipid. Biomass may be derived from a single source, or biomass can comprise a mixture derived from more than one source; for example, biomass could comprise a mixture of corn cobs and corn stover, or a mixture of grass and leaves. Biomass includes, but is not limited to, bioenergy crops, agricultural residues, municipal solid waste, industrial solid waste, sludge from paper manufacture, yard waste, wood and forestry waste. Examples of biomass include, but are not limited to, corn cobs, crop residues such as corn husks, corn stover, grasses, wheat, wheat straw, barley straw, hay, rice straw, switchgrass, waste paper, sugar cane bagasse, sorghum, components obtained from milling of grains, trees, branches, roots, leaves, wood chips, sawdust, shrubs and bushes, vegetables, fruits, flowers and animal manure.

"Biomass hydrolysate" refers to the product resulting from saccharification of biomass. The biomass may also be pretreated or preprocessed prior to saccharification.

The term "heterologous" means not naturally found in the location of interest. For example, a heterologous gene refers to a gene that is not naturally found in the host organism, but that is introduced into the host organism by gene transfer. For example, a heterologous nucleic acid molecule that is present in a chimeric gene is a nucleic acid molecule that is not naturally found associated with the other segments of the chimeric gene, such as the nucleic acid molecules having the coding region and promoter segments not naturally being associated with each other.

As used herein, an "isolated nucleic acid molecule" is a polymer of RNA or DNA that is single- or double-stranded, optionally containing synthetic, non-natural or altered nucleotide bases. An isolated nucleic acid molecule in the form of a polymer of DNA may be comprised of one or more segments of cDNA, genomic DNA or synthetic DNA.

A nucleic acid fragment is "hybridizable" to another nucleic acid fragment, such as a cDNA, genomic DNA, or RNA molecule, when a single-stranded form of the nucleic acid fragment can anneal to the other nucleic acid fragment under the appropriate conditions of temperature and solution ionic strength. Hybridization and washing conditions are well known and exemplified in Sambrook, J., Fritsch, E. F. and Maniatis, T. Molecular Cloning: A Laboratory Manual, 2nd ed., Cold Spring Harbor Laboratory: Cold Spring Harbor, NY (1989), particularly Chapter 11 and Table 11.1 therein. The conditions of temperature and ionic strength determine the "stringency" of the hybridization. Stringency conditions can be adjusted to screen for moderately similar fragments (such as homologous sequences from distantly related organisms), to highly similar fragments (such as genes that duplicate functional enzymes from closely related organisms). Post-hybridization washes determine stringency conditions. One set of preferred conditions uses a series of washes starting with 6X SSC, 0.5% SDS at room temperature for 15 min, then repeated with 2X SSC, 0.5% SDS at 45 °C for 30 min, and then repeated twice with 0.2X SSC, 0.5% SDS at 50 °C for 30 min. A more preferred set of stringent conditions uses higher temperatures in which the washes are identical to those above except for the temperature of the final two 30 min washes in 0.2X SSC, 0.5% SDS was increased to 60 °C. Another preferred set of highly stringent conditions uses two final washes in 0.1X SSC, 0.1% SDS at 65 °C. An additional set of stringent conditions include hybridization at 0.1X SSC, 0.1% SDS, 65 °C and washes with 2X SSC, 0.1% SDS followed by 0.1X SSC, 0.1% SDS, for example.

Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., supra, 9.50-9.51). For hybridizations with shorter nucleic acids, i.e., oligonucleotides, the position of mismatches becomes more important, and the length of the oligonucleotide determines its specificity (see Sambrook et al., supra, 11.7-11.8). The length for a hybridizable nucleic acid may be at least about 10 nucleotides. Preferably a minimum length for a hybridizable nucleic acid is at least about 15 nucleotides; more preferably at least about 20 nucleotides; and most preferably the length is at least about 30 nucleotides. Furthermore, the skilled artisan will recognize that the temperature and wash solution salt concentration may be adjusted as necessary according to factors such as length of the probe.
The term "complementary" is used to describe the relationship between nucleotide bases that are capable of hybridizing to one another. For example, with respect to DNA, adenosine is complementary to thymine and cytosine is complementary to guanine.

The term "percent identity", as known in the art, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. "Identity" and "similarity" can be readily calculated by known methods, including but not limited to those described in: 1.) Computational Molecular Biology (Lesk, A. M., Ed.) Oxford University: NY (1988); 2.) Biocomputing: Informatics and Genome Projects (Smith, D. W., Ed.) Academic: NY (1993); 3.) Computer Analysis of Sequence Data, Part I (Griffin, A. M., and Griffin, H. G., Eds.) Humania: NJ (1994); 4.) Sequence Analysis in Molecular Biology (von Heinje, G., Ed.) Academic (1987); and 5.) Sequence Analysis Primer (Gribskov, M. and Devereux, J., Eds.) Stockton: NY (1991).

Preferred methods to determine identity are designed to give the best match between the sequences tested. Methods to determine identity and similarity are codified in publicly available computer programs. Sequence alignments and percent identity calculations may be performed using the MegAlign program of the LASERGENE bioinformatics computing suite (DNASTAR Inc., Madison, WI).

Multiple alignment of the sequences is performed using the "Clustal method of alignment" which encompasses several varieties of the algorithm including the "Clustal V method of alignment" corresponding to the alignment method labeled Clustal V (described by Higgins and Sharp, CABIOS. 5:151-153 (1989); Higgins, D.G. et al., Comput. Appl. Biosci., 8:189-191 (1992)) and found in the MegAlign v8.0 program of the LASERGENE bioinformatics computing suite (DNASTAR Inc.). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program.

Additionally the "Clustal W method of alignment" is available and corresponds to the alignment method labeled Clustal W (described by Higgins and Sharp, CABIOS. 5:151-153 (1989); Higgins, D.G. et al., Comput. Appl. Biosci. 8:189-191(1992); Thompson, J.D. et al, Nucleic Acid Research, 22 (22): 4673-4680, 1994) and found in the MegAlign v8.0 program of the LASERGENE bioinformatics computing suite (DNASTAR Inc.). Default parameters for multiple alignment (stated as protein/nucleic acid (GAP PENALTY=10/15, GAP LENGTH PENALTY=0.2/6.66, Delay Divergen Seqs(%)=30/30, DNA Transition Weight=0.5, Protein Weight Matrix=Gonnet Series, DNA Weight Matrix=IUB). After alignment of the sequences using the Clustal W program, it is possible to obtain a "percent identity" by viewing the "sequence distances" table in the same program.

It is well understood by one skilled in the art that many levels of sequence identity are useful in identifying polypeptides, from other species, wherein such polypeptides have the same or similar function or activity. Useful examples of percent identities include, but are not limited to: 50%, 55%, 60%, 65%, 70%, 75%, 80%, 85%, 90%, or 95%, or any integer percentage from 50% to 100% may be useful in identifying polypeptides of interest, such as 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%. Suitable nucleic acid fragments not only have the above identities but typically encode a polypeptide having at least 50 amino acids, preferably at least 100 amino acids, and more preferably at least 125 amino acids.

The term "sequence analysis software" refers to any computer algorithm or software program that is useful for the analysis of nucleotide or amino acid sequences. "Sequence analysis software" may be commercially available or independently developed. Typical sequence analysis software will include, but is not limited to: 1.) the GCG suite of programs (Wisconsin Package Version 9.0, Genetics Computer Group (GCG), Madison, WI); 2.) BLASTP, BLASTN, BLASTX (Altschul et al., J. Mol. Biol., 215:403-410 (1990)); 3.) DNASTAR (DNASTAR, Inc. Madison, WI); 4.) Sequencher (Gene Codes Corporation, Ann Arbor, MI); and 5.) the FASTA program incorporating the Smith-Waterman algorithm (W. R. Pearson, Comput. Methods Genome Res., [Proc. Int. Symp.] (1994), Meeting Date 1992, 111-20. Editor(s): Suhai, Sandor. Plenum: New York, NY). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

Standard recombinant DNA and molecular cloning techniques used herein are well known in the art and are described by Sambrook, J. and Russell, D., Molecular Cloning: A Laboratory Manual, Third Edition, Cold Spring Harbor Laboratory Press, Cold Spring Harbor, NY (2001); and by Silhavy, T. J., Bennan, M. L. and Enquist, L. W., Experiments with Gene Fusions, Cold Spring Harbor Laboratory Press, Cold Spring Harbor, NY (1984); and by Ausubel, F. M. et. al., Short Protocols in Molecular Biology, 5th Ed. Current Protocols, John Wiley and Sons, Inc., N.Y., 2002.

The present invention relates to methods of creating strains of *Zymomonas* that can grow in medium containing xylose as the only sugar directly following introduction of a xylose utilization pathway, without an adaptation period in xylose-containing medium. Xylose is one of the predominant pentose sugars in hydrolyzed lignocellulosic materials, making utilization of xylose desirable for fermentation of biomass hydrolysate. Biomass hydrolysate can provide an abundant, renewable carbohydrate resource for biocatalytic production of target products. Eliminating an adaptation step in xylose-containing medium, which is time-consuming and where multiple undefined mutations may occur, greatly facilitates development of a xylose-utilizing biocatalyst.

### Engineered xylose utilizing Zymomonas

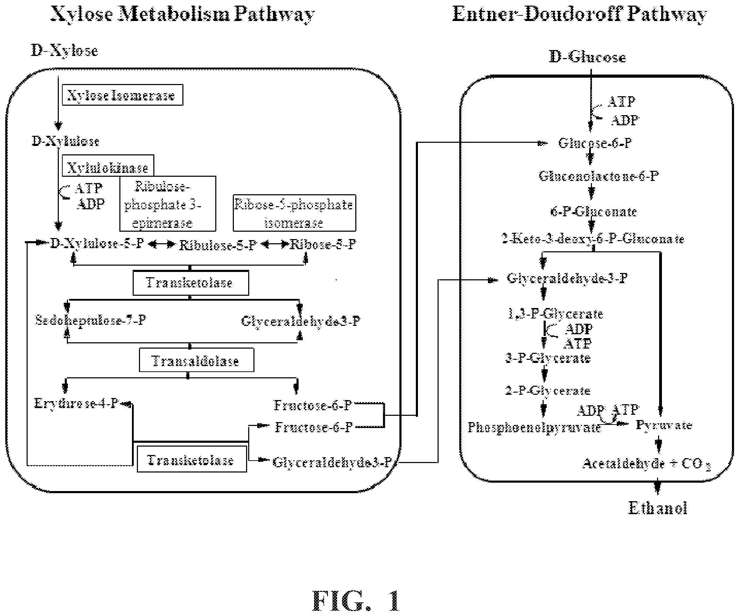

*Zymomonas* cells naturally produce ethanol using glucose, fructose and/or sucrose as fermentation substrates, but xylose is not metabolized. Strains of ethanol-producing *Zymomonas,* such as *Z. mobilis* have been engineered for xylose fermentation to ethanol. Typically four genes have been introduced into *Z. mobilis* for expression of four enzymes involved in xylose metabolism to create a xylose utilization metabolic pathway (Figure 1) as described in U.S. Pat. No. 5,514,583, U.S. Pat. No. 5,712,133, U.S. Pat. No. 6,566,107, WO 95/28476, Feldmann et al. ((1992) Appl Microbiol Biotechnol 38: 354-361), and Zhang et al. ((1995) Science 267:240-243). These include genes encoding xylose isomerase which catalyzes the conversion of xylose to xylulose, and xylulokinase which phosphorylates xylulose to form xylulose 5-phosphate. Additionally expressed are transketolase and transaldolase, two enzymes of the pentose phosphate pathway that convert xylulose 5-phosphate to intermediates that couple pentose metabolism to the glycolytic Entner-Douderoff pathway permitting the metabolism of xylose to ethanol (see Figure 1). DNA sequences encoding these enzymes may be obtained from any of numerous microorganisms that are able to metabolize xylose, such as enteric bacteria, and some yeasts and fungi. Sources for the coding regions may include *Xanthomonas, Klebsiella, Escherichia, Rhodobacter, Flavobacterium, Acetobacter, Gluconobacter, Rhizobium, Agrobacterium, Salmonella, Pseudomonads,* and *Zymomonas.* The coding regions of *E. coli* are typically used.

The encoding DNA sequences are operably linked to promoters that are expressed in *Zymomonas* cells such as the promoter *of Z. mobilis* glyceraldehyde-3-phosphate dehydrogenase (GAP promoter), and *Z. mobilis* enolase (ENO promoter). A mutant GAP promoter with increased expression as disclosed in US 7,989,206 is also useful for expression in *Zymomonas.* The coding regions may individually be expressed from promoters, or two or more coding regions may be joined in an operon with expression from the same promoter. The resulting chimeric genes may be introduced into *Zymomonas* cells and maintained on a plasmid, or integrated into the genome using, for example, homologous recombination, site-directed integration, or random integration. Examples of strains engineered to express a xylose utilization metabolic pathway include CP4(pZB5) (US 5,514,583), ATCC31821/pZB5 (US 6,566,107), 8b (US 20030162271; Mohagheghi et al., (2004) Biotechnol. Lett. 25; 321-325), and ZW658 (ATTCC # PTA-7858).

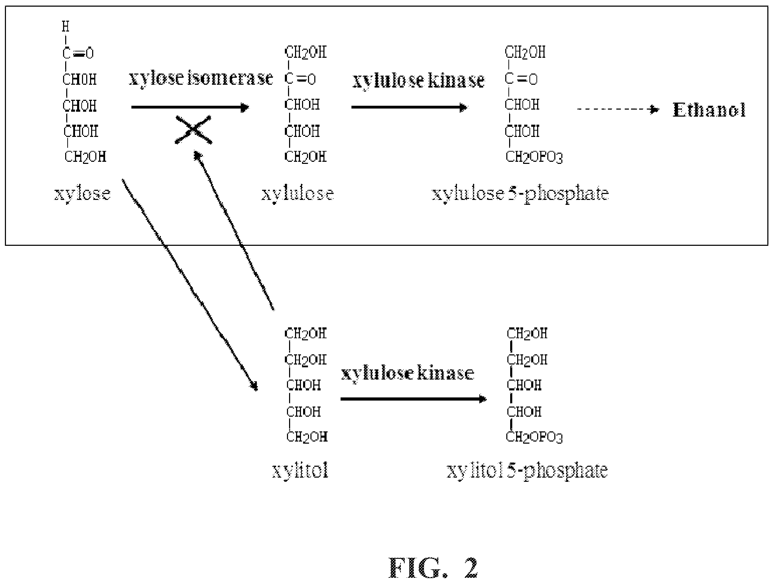

Strains engineered to express the xylose utilization metabolic pathway as described above typically are not able to immediately grow in medium containing xylose as the only sugar. One metabolic issue for these strains is a side pathway shown in Figure 2 (outside the box) that not only reduces efficiency of ethanol production from xylose (boxed section), but may also have a detrimental effect on cell viability in the presence of xylose, especially when xylose is the sole carbon source. In this pathway, xylose is converted to xylitol, which in turn is converted to xylitol 5-phosphate by xylulose kinase which is the second enzyme in the engineered xylose utilization metabolic pathway. Xylitol 5-phosphate is a toxic compound that inhibits bacterial growth, and it has clearly been demonstrated that *E. coli* xylulokinase is directly responsible for the inhibitory effect of xylitol through its conversion to xylitol 5-phosphate (Akinterinwa et al. (2009) Metabolic Engineering 11: 48-55). In addition, conversion of xylose to xylitol also reduces substrate flow to ethanol, and xylitol is also an inhibitor of xylose isomerase, the first enzyme in the engineered pathway for xylose utilization. Experiments have established that there are at least two different pathways for xylitol formation in *Z. mobilis:* 1) by glucose-fructose oxidoreductase (US 7,741,119), and 2) by NADPH-dependent aldose reductase activity (Feldmann et al. *supra;* Agrawal et al. (2011) Biotechnology and Bioengineering 108:777-785).

Cells of *Zymomonas* that are engineered for expression of the xylose utilization metabolic pathway generally require a period of adaptation in xylose-containing medium prior to being able to grow in medium that contains xylose as the only sugar. During the adaptation step, which is typically a prolonged period of serial transfers in xylose-containing medium, multiple mutations can occur in the genome. A number of specific mutations which have occurred during adaptation of different strains that were engineered for xylose utilization have been identified, but it is not clear which ones are important and/or necessary for the initial ability to grow on xylose as sole carbon source. Indeed, prior to this work, no single mutation had been identified that allows immediate growth of a *Zymomonas* strain expressing the xylose utilization metabolic pathway in medium containing xylose as the only sugar.

### Engineering immediate growth in xylose medium

The present invention relates to a new finding that genetic modification affecting expression of a single enzyme activity can be made in cells of *Zymomonas* that also are engineered for xylose utilization, which allows immediate growth in medium containing xylose as the only sugar, without an adaptation step. These cells are xylose-competent *Zymomonas* cells. The genetic modification disrupts the expression of at least one endogenous gene encoding an NADPH-dependent aldose reductase that is able to convert xylose to xylitol, thereby causing a reduction of NADPH-dependent xylose reductase activity in cell-free extracts by greater than 90%. The activity may be reduced by greater than 90%, 91%, 92%, 93%, 94%, 95%, 96%, 97%, 98%, or 99%. NADPH-dependent xylose reductase activity is measured in cell-free extracts by monitoring the conversion of NADPH to NADP at 340 nm in the presence of xylose as described in the General Methods herein.

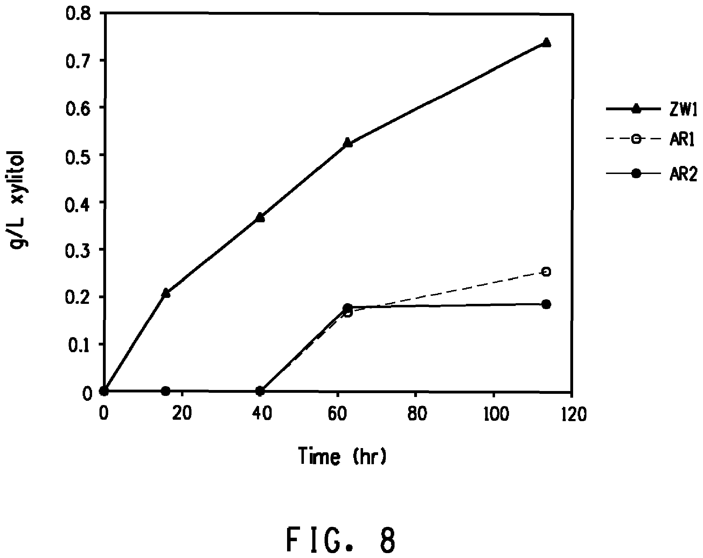

Reducing NADPH-dependent xylose reductase acitvity by greater than 90% in otherwise wild type *Zymomonas* cells, by disrupting expression of an endogenous gene encoding a putative aldo/keto reductase (as designated by Seo et al. (2005) Nat. Biotechnol. 23: 63-68) was found herein to reduce production of xylitol by the cells when grown in xylose-containing medium. Even with this genetic manipulation, however, cellular production of xylitol was not completely eliminated, although it was reduced by about a factor of three (shown in Figure 8).

Cells with this disruption, that were subsequently provided with the four enzymes that are required for xylose utilization (a xylose utilization metabolic pathway), were shown herein (Example 6) to be able to immediately grow in medium containing xylose as the only sugar without the need for an adaptation step. Thus these are xylose-competent *Zymomonas* cells which can be selected for using medium containing only xylose as the carbon source. Alternatively, in a Zymomonas cell that already has the four enzymes that are required for xylose-utilization but has not been adapted for growth on xylose, intentional inactivation of NADPH-dependent xylose reductase activity can be selected for using medium that only contains xylose as the carbon source.

Immediate growth on media that contained only xylose without an adaptation step was enabled even in the presence of wild type glucose-fructose oxidoreductase (GFOR) activity. GFOR strongly contributes to xylitol production in *Zymomonas* cells engineered to express xylose isomerase, the first enzyme in the xylose metabolism pathway, since it converts xylose to xylulose. In contrast to the direct reduction of xylose to xylitol by NADPH-dependent xylose reductases, xylitol production by GFOR only occurs in the presence of glucose-containing media when a source of xylulose is also present as disclosed in US 7,741,119.
In the present method, expression of one or more endogenous aldose reductase encoding genes of the *Zymomonas* genome is disrupted. In the fully sequenced genome of *Zymomonas* there are multiple genes annotated as encoding aldo/keto reductases. For example, in the sequenced genome of the wild type *Zymomonas mobilis* ZM4 strain (GenBank accession number AE008692; Seo et al., Nat. Biotechnol. 23 (1), 63-68 (2005)), there are at least four genes that code for putative aldo/keto reductases, which are named *ZMO0976, ZMO1344, ZMO1673,* and *ZMO1773.* The proteins encoded by the *ZMO1344, ZMO1673,* and *ZMO1773* genes have 24%, 29% and 38% amino acid sequence identities, respectively, to the *ZMO0976* encoded protein (SEQ ID NO:2). Two additional genes recently re-annotated as aldo/ketose reductases (*ZMO062* and *ZMO1984*) encode proteins with about 32% identity to the *ZMO976* encoded protein. Aldo/keto reductases belong to a superfamily of soluble NAD(P)H oxidoreductases whose chief purpose is to reduce aldehydes and ketones to primary and secondary alcohols. Aldose reductases, also called aldehyde reductases, are classified as EC 1.1.1.21. The present method and strains concern aldose reductase enzymes that are able to convert xylose to xylitol in the presence of NADPH (which is converted to NADP), an enzyme activity which is referred to herein as NADPH-dependent xylose reductase activity.

A genetic modification causing disruption of only the *ZMO0976* gene in *Z. mobilis* strain ZW1 is shown herein in Example 5 to reduce NADPH-dependent xylose reductase activity by greater than 90%. Thus in the present method, the wild type *Z. mobilis* strain ZM4 (ATCC #31821; ZW1 is another name for ZM4) and derivatives thereof are engineered with a genetic modification that disrupts the *ZMO0976* gene, which is identified in the ZM4 genomic sequence of GenBank accession number AE008692. This is the only genetic modification needed to provide competency for growth on medium containing xylose as the only sugar when the four xylose pathway enzymes are introduced into wild type ZM4, eliminating the need for a xylose-adaptation step. The other genes annotated as encoding aldo/keto reductases in the ZM4 genome (GenBank accession number AE008692), *ZMO1344, ZMO1673, ZMO177, ZM0062,* and *ZMO1984* did not significantly contribute to NADPH-dependent aldose reductase activity in the presence of xylose in cell-free extracts, as shown in Example 5 herein.

The *ZMO0976* gene has the coding sequence of SEQ ID NO:1, which encodes a protein of SEQ ID NO:2 shown herein to have NADPH-dependent xylose reductase activity. Wild type strains *Z. mobilis* NCIMB 11163 and ATCC 10988 have genes with coding regions that have 100% nucleotide sequence identity to SEQ ID NO:1, and encode proteins that have 100% amino acid sequence identity to SEQ ID NO:2. These genes with identical nucleotide sequences in *Z. mobilis* strains NCIMB 11163 and ATCC 10988 are disrupted in the present method to provide competency for growth on medium containing xylose as the only sugar, with no adaptation for growth on xylose.

There may be variation in sequences of the genes that are equivalent to *ZMO0976* in different strains of *Zymomonas,* which may be genetically modified in the present method. Thus coding sequences with nucleotide sequence identity of at least about 95%, 96%, 97%, 98%, or 99% to SEQ ID NO:1 may represent the *ZMO0976* gene in different strains of *Zymomonas.* In addition, proteins with amino acid sequence identity of at least about 95%, 96%, 97%, 98%, or 99% to SEQ ID NO:2 may represent the *ZMO0976* gene product in different strains of *Zymomonas.* In the present methods and strains, genes having coding regions with these identities, or genes encoding proteins with these identities, are disrupted.

In other *Zymomonas* species or strains it may be necessary to inactivate multiple genes that code for proteins that have NADPH-dependent xylose reductase activity to achieve the greater than 90% reduction of total NADPH-dependent aldose reductase enzyme activity that allows immediate growth on xylose. One of skill in the art can readily determine by experimentation, using genetic modification methods described below and others known in the art, which gene or genes annotated as aldo/keto reductase in a *Zymomonas* genome sequence should be disrupted to obtain greater than 90% decrease in NADPH-dependent xylose reductase activity.

Genetic modification to disrupt an NADPH-dependent xylose reductase encoding gene described above, that is a target gene, in the present method may be using any method known to one skilled in the art such as methods that affect its expression of mRNA or protein, or the function or stability of the encoded protein. Genetic modifications may be, for example, mutation, insertion, or deletion in the coding region, or other region of the gene such as the promoter. Methods include, but are not limited to, deletion of the entire or a portion of the gene, inserting a DNA fragment into the gene (in either the promoter or coding region) so that the encoded protein cannot be expressed, introducing a mutation into the coding region which adds a stop codon or frame shift such that a functional protein is not expressed, and introducing one or more mutations into the coding region to alter amino acids so that a non-functional protein is expressed. All of these methods may be readily practiced by one skilled in the art making use of the known target NADPH-dependent xylose reductase coding sequence (such as SEQ ID NO:1), as well as the *Zymomonas* DNA sequence that surrounds the target NADPH-dependent xylose reductase coding sequence, such as that which is available in the complete *Z. mobilis* genome sequence (GenBank Accession AE008692).

A particularly suitable method for creating a genetic modification in an NADPH-dependent xylose reductase encoding target gene, as exemplified herein in Examples 3 and 4, is using double-crossover homologous recombination mediated by *ZMO0976* flanking DNA sequences bounding a chloramphenicol resistance or other marker gene, leading to insertion of the marker gene in the target aldose reductase coding region such that a functional protein is not expressed. In addition, the marker gene may be bounded by site-specific recombination sites, so that following expression of the corresponding site-specific recombinase, the resistance gene is excised from the gene. The site-specific recombination leaves behind a recombination site which disrupts expression of the aldose reductase encoding gene. The homologous recombination vector may be constructed to also leave a deletion in the aldose reductase encoding gene following excision of the marker, as is well known to one skilled in the art.

In one embodiment the present method steps (b) and (c) are performed concurrently by integration of one or more genes of the xylose utilization metabolic pathway into the endogenous target gene encoding NADPH-dependent xylose reductase, thereby disrupting its expression. In other embodiments integration of one or more genes of the xylose utilization metabolic pathway may be in another gene, thereby creating an additional genetic modification as described above.

For any of the host cells and during any of the steps of the present method there is no need for a preliminary adaptation process to achieve immediate growth on medium that contains xylose as the sole carbon source, provided the engineered xylose metabolism pathway provides sufficient carbon flux to support growth. No additional mutations, other than disruption of at least one endogenous gene encoding NADPH-dependent xylose reductase acitivity that reduces activity by greater than 90%, are required in a host cell engineered with a xylose utilization metabolic pathway to provide said growth property. However, even with said disruption, further adaptation in xylose-containing media can result in better growth on xylose through natural selection of other mutations that increase carbon flux through the engineered xylose pathway. In one embodiment said disruption is only in the *ZMO0976* gene (such as in GenBank accession number AE008692; such as in SEQ ID NO:1).

### Additional Genetic Modifications

In one embodiment a wild type strain of *Zymomonas* is used as the host cell for introduction of a xylose utilization metabolic pathway and a genetic modification disrupting expression of at least one endogenous gene encoding an NADPH-dependent xylose reductase. In various embodiments the host cell or xylose-competent *Zymomonas* cell has one or more additional genetic modifications that are performed prior to, concurrently with, or after steps (b) and/or (c) of the present method. Additional genetic modifications may include any modification that improves the strain such as one that increases growth rate and/or cell mass, increases utilization of xylose and/or allows the use of other sugars, increases tolerance to inhibitory compounds such as acetate, or increases production of ethanol. These genetic modifications can be introduced through rational design or they can occur spontaneously through random mutations and natural selection by further adaptation of the strain in various xylose-containing growth media.

In one embodiment *Zymomonas* cells may be additionally engineered for arabinose utilization which is described in US 5,843,760. To allow arabinose utilization, genes expressed in addition to genes of the xylose utilization pathway include:1) L-arabinose isomerase to convert L-arabinose to L-ribulose, 2) L-ribulokinase to convert L-ribulose to L-ribulose-5-phosphate, and 3) L-ribulose-5-phosphate-4-epimerase to convert L-ribulose-5-phosphate to D-xylulose (US 5,843,760). As disclosed in US 2011/0143408, improved arabinose utilization may be achieved by additionally expressing an arabinose-proton symporter, such as by expressing a coding region from an araE gene.

In another embodiment the endogenous *himA* gene, which encodes the alpha subunit of the integration host factor, is genetically modified to reduce its expression which improves growth in medium containing acetate as described in US 7,897,396. Acetate is present in biomass hydrolysate, thus when using medium containing biomass hydrolysate, increased tolerance to this component is desired.

In another embodiment a genetic modification is made that reduces glucose-fructose oxidoreductase (GFOR) activity as described in US 7,741,119. Reduced expression of GFOR, as well as of the himA gene, may be by any method such as those described above for reducing aldose reductase activity.

In another embodiment a genetic modification is made which increases ribose-5-phosphate isomerase (RPI) activity, as disclosed in commonly owned and co-pending US20120156746A1. Increased RPI expression may be accomplished by increasing expression of the endogenous RPI encoding gene, such as with a promoter that is more highly active than the native promoter, or by expressing a heterologous gene encoding any protein or polypeptide with ribose-5-phosphate isomerase activity in *Zymomonas.* There are two groups of ribose-5-phosphate isomerase enzymes that are called RPI-A and RPI-B, as described in US20120156746A1, either of which may be expressed.

In another embodiment, the xylose isomerase that is expressed as part of the xylose utilization metabolic pathway is expressed using a mutant, highly active promoter that is disclosed in US 7,989,206 and US 7,998,722. Mutant promoters disclosed therein are promoters of the *Zymomonas mobilis* glyceraldehyde-3-phosphate dehydrogenase gene (*Pgap*) having a mutation of G to T at position 116 or of C to T at position 217 of the promoter of SEQ ID NO:3. Mutant, more highly active promoters include SEQ ID NOs:4, 5, 6, 7, 8, 9, 10, 11, and 12. These include variant promoter sequences from different strains of *Z. mobilis* with one or both of the mutations at positions equivalent to 116 and 217 in SEQ ID NO:3.

In another embodiment a xylose isomerase that is expressed as part of the xylose utilization metabolic pathway is a Group I xylose isomerase included in the class of enzymes identified by EC 5.3.1.5 as disclosed in commonly owned and co-pending US2011-0318801. It is disclosed therein that Group I xylose isomerases, such as one expressed from a coding region isolated from *Actinoplanes missouriensis* (coding region with codon optimization for *Zymomonas:* SEQ ID NO:13), have higher activity in *Zymomonas* than Group 2 xylose isomerases. Group I xylose isomerases are defined therein by molecular phylogenetic bioinformatics analysis (using PHYLIP neighbor joining algorithm as implemented in PHYLIP (Phylogeny Inference Package version 3.5c; Felsenstein (1989) Cladistics 5:164-166), GroupSim analysis (Capra and Singh (2008) Bioinformatics 24: 1473-1480), and a Profile Hidden Markov Model (using the hmmsearch algorithm of the HMMER software package; Janelia Farm Research Campus, Ashburn, VA).

Although no mutation other than one conferring reduction of aldose reductase activity for conversion of xylose to xylitol in the presence of NADPH by greater than 90% is required for immediate growth on xylose, in one embodiment serial transfers of the xylose-competent cells described herein in media containing xylose as a carbon source (adapting in xylose-containing media) may be used to produce new strains that grow better on xylose as a consequence of natural selection through the accumulation of mutations in other genes that are beneficial to xylose metabolism. Adaptation on xylose-containing medium is described in U. S. Pat. 7,223,575 and U. S. Pat. 7,741,119.

### Characteristics of the present cells

The *Zymomonas* cells described herein have the natural ability to produce ethanol. In addition the cells, which are grown to provide strains, have greater than 90% reduction in native aldose reductase activity for conversion of xylose to xylitol in the presence of NADPH. In addition the cells have a xylose utilization metabolic pathway comprising a series of polynucleotides encoding polypeptides, each having xylose isomerase, xylulokinase, transketolase, or transaldolase enzymatic activity.

The strains disclosed herein may additionally have one or more characteristic that is an improvement in the strain such as increased growth rate or cell mass production, increased utilization of xylose and/or use of other sugars, increased tolerance to inhibitory compounds such as acetate, or increased production of ethanol. These characteristics are conferred by genetic modifications such as those described above.

### Fermentation for Ethanol Production

An engineered *Zymomonas* strain having a xylose utilization pathway and at least one disrupted gene encoding aldose reductase, having aldose reductase activity for conversion of xylose to xylitol in the presence of NADPH reduced by greater than 90%, may be used in fermentation to produce ethanol. *Zymomonas mobilis* is a natural ethanolagen. As an example, production of ethanol by a Z. *mobilis* strain produced by a method of the invention is described.

For production of ethanol, recombinant xylose-utilizing *Z. mobilis* is brought in contact with medium that contains mixed sugars including xylose. Typically the medium contains mixed sugars including arabinose, xylose, and glucose. The medium may contain biomass hydrolysate that includes these sugars that are derived from treated cellulosic or lignocellulosic biomass.

When the mixed sugars concentration is high such that growth is inhibited, the medium includes sorbitol, mannitol, or a mixture thereof as disclosed in US 7,629,156. Galactitol or ribitol may replace or be combined with sorbitol or mannitol. The *Z. mobilis* grows in the medium where fermentation occurs and ethanol is produced. The fermentation is run without supplemented air, oxygen, or other gases (which may include conditions such as anaerobic, microaerobic, or microaerophilic fermentation), for at least about 24 hours, and may be run for 30 or more hours. The timing to reach maximal ethanol production is variable, depending on the fermentation conditions. Typically, if inhibitors are present in the medium, a longer fermentation period is required. The fermentations may be run at temperatures that are between about 30 ° C and about 37 ° C, at a pH of about 4.5 to about 7.5.

The present *Z. mobilis* cells may be grown in medium containing mixed sugars including xylose in laboratory scale fermenters, and in scaled up fermentation where commercial quantities of ethanol are produced. Where commercial production of ethanol is desired, a variety of culture methodologies may be applied. For example, large-scale production from the present *Z. mobilis* strains may be produced by both batch and continuous culture methodologies. A classical batch culturing method is a closed system where the composition of the medium is set at the beginning of the culture and not subjected to artificial alterations during the culturing process. Thus, at the beginning of the culturing process the medium is inoculated with the desired organism and growth or metabolic activity is permitted to occur adding nothing to the system. Typically, however, a "batch" culture is batch with respect to the addition of carbon source and attempts are often made at controlling factors such as pH and oxygen concentration. In batch systems the metabolite and biomass compositions of the system change constantly up to the time the culture is terminated. Within batch cultures cells moderate through a static lag phase to a high growth log phase and finally to a stationary phase where growth rate is diminished or halted. If untreated, cells in the stationary phase will eventually die. Cells in log phase are often responsible for the bulk of production of end product or intermediate in some systems. Stationary or post-exponential phase production can be obtained in other systems.

A variation on the standard batch system is the Fed-Batch system. Fed-Batch culture processes are also suitable for growth of the present *Z. mobilis* strains and comprise a typical batch system with the exception that the substrate is added in increments as the culture progresses. Fed-Batch systems are useful when catabolite repression is apt to inhibit the metabolism of the cells and where it is desirable to have limited amounts of substrate in the medium. Measurement of the actual substrate concentration in Fed-Batch systems is difficult and is therefore estimated on the basis of the changes of measurable factors such as pH and the partial pressure of waste gases such as CO₂. Batch and Fed-Batch culturing methods are common and well known in the art and examples may be found in Biotechnology: A Textbook of Industrial Microbiology, Crueger, Crueger, and Brock, Second Edition (1989) Sinauer Associates, Inc., Sunderland, MA, or Deshpande, Mukund V., Appl. Biochem. Biotechnol., 36, 227, (1992).

Commercial production of ethanol may also be accomplished with a continuous culture. Continuous cultures are open systems where a defined culture medium is added continuously to a bioreactor and an equal amount of conditioned medium is removed simultaneously for processing. Continuous cultures generally maintain the cells at a constant high liquid phase density where cells are primarily in log phase growth. Alternatively, continuous culture may be practiced with immobilized cells where carbon and nutrients are continuously added, and valuable products, by-products or waste products are continuously removed from the cell mass. Cell immobilization may be performed using a wide range of solid supports composed of natural and/or synthetic materials as is known to one skilled in the art.

Continuous or semi-continuous culture allows for the modulation of one factor or any number of factors that affect cell growth or end product concentration. For example, one method will maintain a limiting nutrient such as the carbon source or nitrogen level at a fixed rate and allow all other parameters to moderate. In other systems a number of factors affecting growth can be altered continuously while the cell concentration, measured by medium turbidity, is kept constant. Continuous systems strive to maintain steady state growth conditions and thus the cell loss due to medium being drawn off must be balanced against the cell growth rate in the culture. Methods of modulating nutrients and growth factors for continuous culture processes as well as techniques for maximizing the rate of product formation are well known in the art of industrial microbiology and a variety of methods are detailed by Brock, *supra.*

Particularly suitable for ethanol production is a fermentation regime as follows. The desired *Z. mobilis* strain prepared according to the present invention is grown in shake flasks in semi-complex medium at about 30 °C to about 37 °C with shaking at about 150 rpm in orbital shakers and then transferred to a 10 L seed fermentor containing similar medium. The seed culture is grown in the seed fermentor anaerobically until OD₆₀₀ is between 3 and 6, when it is transferred to the production fermentor where the fermentation parameters are optimized for ethanol production. Typical inoculum volumes transferred from the seed tank to the production tank range from about 2% to about 20% v/v. Typical fermentation medium contains minimal medium components such as potassium phosphate (1.0 - 10.0 g/l), ammonium sulfate (0- 2.0 g/l), magnesium sulfate (0 - 5.0 g/l), a complex nitrogen source such as yeast extract or soy based products (0 - 10 g/l). A final concentration of about 5 mM sorbitol or mannitol is present in the medium. Mixed sugars including xylose and at least one additional sugar such as glucose (or sucrose), providing a carbon source, are continually added to the fermentation vessel on depletion of the initial batched carbon source (50-200 g/l) to maximize ethanol rate and titer. Carbon source feed rates are adjusted dynamically to ensure that the culture is not accumulating glucose in excess, which could lead to build up of toxic byproducts such as acetic acid. In order to maximize yield of ethanol produced from substrate utilized, biomass growth is restricted by the amount of phosphate that is either batched initially or that is fed during the course of the fermentation. The fermentation is controlled at pH 5.0 - 6.0 using caustic solution (such as ammonium hydroxide, potassium hydroxide, or sodium hydroxide) and either sulfuric or phosphoric acid. The temperature of the fermentor is controlled at 30 °C - 35 °C. In order to minimize foaming, antifoam agents (any class- silicone based, organic based etc) are added to the vessel as needed. An antibiotic, for which there is an antibiotic resistant marker in the strain, such as kanamycin, may be used optionally to minimize contamination.

Any set of conditions described above, and additionally variations in these conditions that are well known in the art, are suitable conditions for production of ethanol by a xylose-utilizing recombinant *Zymomonas* strain.

### EXAMPLES

### GENERAL METHODS

### Transformation of Z. mobilis

Replicating and non-replicating plasmid DNA was introduced into *Z. mobilis* using electroporation, essentially as described in US 5,514,583. Briefly, the 50-µl transformation reactions contained ∼10¹⁰ cells/ml in 10% (v/v) glycerol and 1-2 µg of non-methylated plasmid DNA that was isolated from *E. coli* SCS110. Control reactions were treated identically, but did not receive any plasmid DNA. The settings for the electroporator were 1.6 kv/cm, 200 Ω, and 25 µF, and the gap width of the cuvette was 0.1 cm. Following electroporation, the transformation reactions were diluted with MMG medum (50 g/L glucose, 10 g/L yeast extract, 5 g/L of tryptone, 2.5 g/L of (NH₄)₂SO₄, 0.2 g/L K₂HPO₄, and 1 mM MgS0₄) and the cells were allowed to recover at 30 °C before they were plated on MMG medium that contained 1.5% agar (MMG agar plates) with or without antibiotics as indicated. MMX agar plates are identical to MMG agar plates but contain 50 g/L xylose instead of glucose. Plates were incubated in an anaerobic chamber at 30-33 °C, until colonies appeared. Additional details are described in the Examples section.

### Zymomonas mobilis ZW641 and ZW658 strain construction

A detailed description of the construction of the xylose-utilizing recombinant strain, ZW658, starting from the wild type parent strain, ZW1, is provided in US 7,741,084. ZW658 was constructed by integrating two operons, P*_{gap}xylAB* and P*_{gap}taltkt,* containing four xylose-utilizing genes encoding xylose isomerase (xylA), xylulokinase (xylB), transaldolase (tal), and transketolase (tkt), with coding regions from the *E. coli* genes, into the genome of ZW1 (rename of strain ZM4; ATCC 31821) via sequential transposition events followed by adaptation on xylose-containing growth media to produce strain X13L3, which was subsequently renamed ZW641 as disclosed in US 7,989,206. Further adaptation of ZW641 on xylose-containing growth media gave rise to ZW658, which grows much better in xylose and was deposited under the Budapest Treaty as ATCC PTA-7858. As further disclosed in US 7,989,206, ZW658 has higher xylose isomerase activity than ZW641 due to a point mutation in the *Z*. *mobilis* P_{gap} promoter that drives the chromosomally integrated *E. coli* XylA/B operon. This mutant promoter (SEQ ID NO:4), herein called either the 801 GAP promoter or the Super GAP promoter or P_{gapS}, has a "T" instead of "G" in position 116, when compared to the native P_{gap} in ZW641 (the 641GAP promoter). The 801 Gap promoter is 3- to 4-fold stronger than the 641Gap promoter, and the rate-limiting step for xylose metabolism in ZW641 is xylose isomerase.

### Shake flask experiments

Unless otherwise noted, all experiments described below were conducted at 33 °C in shake flasks (15-ml loosely-capped, conical shaped test tubes) using synthetic media that contained glucose and/or xylose as carbon sources. mRM3-G10 medium contains 10 g/L yeast extract, 2 g/L KH₂PO₄, 1 g/L MgSO₄ (7H₂0) and 100 g/L glucose. mRM3-X10 medium is identical but it contains 100 g/L xylose instead of glucose. Cell growth was monitored spectrophometrically by following changes in optical density at 600 nm as a function of time. In the text and figure legends "OD" or "OD600" means optical density at 600 nm. At indicated times during shake flask growth experiments, 1.0-ml aliquots of the cultures were removed for HPLC analysis using an HP 1100 equipped with a refractive index detector (Hewlett-Packard, Palo Alto, CA) to determine the concentrations of glucose, xylose, xylitol, ribulose, glycerol, acetate and ethanol that were present in the fermentation broth. Prior to HPLC analysis, cells were removed by centrifugation and the supernatant was filtered through a 0.22 µm cellulose acetate Spin-X centrifuge tube filter (Costar, catalog number 8160) to remove small particles. Compounds were separated on an Aminex HPX-87H column (Bio-Rad) that was run at 55 °C under isocratic conditions using a flow rate of 0.6 ml/min and 0.01 N H₂SO₄ as the mobile phase. Authentic standards of known concentration were used to quantify the peaks of interest and all results are expressed in g/L.

### Preparation of cell-free extracts

*Z. mobilis* strains ZW1 and ZW1-AR1 were grown in 25 ml of mRM3-G10 to OD -2.8, harvested by centrifugation at 3,000 x g (4 °C), and cell pellets were rapidly frozen and stored at -80 °C for subsequent use. Cell-free extracts were prepared by mechanical disruption using a Bio101 FastPrep FP120 Cell Disrupter (ThermoSavant); all steps were performed at 0-4 °C except the mechanical disruption step, which was done at room temperature as described below. Frozen cell pellets were thawed, washed three times with 1.0 ml of Lysis Buffer that contained 12.5 mM triethanolamine hydrochloride (adjusted to pH 7.5 with KOH), 0.5 mM EDTA, 1 mM dithiothreitol, and were finally resuspended in 0.8 ml of the same solution. The resuspended cells were transferred to 2.0-ml Lysing Matrix B tubes that contained 0.1 mm silica spheres (MP Biomedicals, Catalog No. 9911-100) and were subjected to three cyles of cell disruption in the FastPrep FP120; each cycle consisted of a 20-sec agitation period at 6 m/sec followed by a 3-min chilling period on ice. The lysate was then centrifuged for 10 min at 20, 800 x g and the resulting supernatant was transferred to a fresh tube and re-centrifuged for another 60 min at the same speed. The supernatant from the second centrifugation step is referred to below as the "cell-free extract" in the NADPH-dependent xylose reductase activity assay described below. Protein concentration of the cell-free extracts was measured using the Coomassie (Bradford) Protein Assay Kit (Pierce Biotechnology, Catalog No. 23200) with bovine serum albumin as a standard.

### Measurement of NADPH-dependent Xylose Reductase Activity

NADPH-dependent aldose reductase activity in cell-free extracts with xylose as the substrate was measured essentially as described (Feldmann et al (1992) Appl Microbial Biotechnol. 38:354-361) with minor modifications. The assay was conducted at 32 °C in a 0.5-ml quartz cuvette that contained the following components: 400 µl of Reaction Buffer (50 mM triethanolamine hydrochloride (pH 7.5) and 5 mM MgSO₄), 40 µl of cell-free extract (that contained 8-10 mg of protein/ml), 10 µl of 10 mM NADPH tetra(cyclohexylammonium) salt and 50 µl of 2M D-xylose. The "no xylose control" reactions were identical, but xylose was omitted and 50 µl of Reaction Buffer was added instead. Enzyme activity was measured spectrophotometrically as a function of time at 340 nm using an extinction coefficient of 6220 M⁻¹cm⁻¹ to monitor the conversion of NADPH to NADP. Enzyme activities are expressed as mU/mg of cell-free extract protein after correcting for the rate of NADPH oxidation in the absence of xylose; 1 mU = 1 nanomole of xylitol formed per minute. The signal-to-noise ratio for NADPH-dependent xylose reductase activity was -3.5 for the wild type strain, ZW1 (i.e. the rate of NADPH disappearance in the presence of xylose compared to the "no xylose control" reaction).

### Example 1

### Discovery of ZMO0976 mutation in adapted xylose-utilizing Zymomonas strains

Comparative genomic sequence analysis of *Zymomonas mobilis* wild type strain ZW1, which is genetically identical to wild type strain ZM4 (ATCC#31821), and two adapted xylose-utilizing ZW1 derivatives, ZW641 and ZW658 (see General Methods for strain construction) was performed to identify genetic modifications (mutations) that occurred during adaptation on xylose. Whole genome sequence analysis showed that multiple mutations occurred during adaptation of ZW641 and ZW658 strains. Among these mutations was a 78 nucleotide deletion in a coding region of ZW1, which was present in both ZW641 and ZW658. The coding region having the deletion is designated as *ZMO0976* in the genomic sequence of the ZM4 strain (GenBank accession number AE008692). This *ZMO0976* coding region is annotated as encoding an aldo/keto reductase. The deletion removes a large portion (nucleotides 547 to 624 relative to the start of the initiation codon) of the 1023 nucleotide full coding region (including the stop codon) of *ZMO0976* (SEQ ID NO:1), resulting in SEQ ID NO:14.

### Example 2

### Additional xylose-utilizing Zymomonas strains with ZMO0976 mutations

Two additional xylose-utilizing strains were obtained by introduction of genes for the xylose utilization pathway and adaptation on xylose-containing medium as follows.

### Vector constructs for building xylose utilizing Z. mobilis strains using targeted integration

A new xylose utilizing *Z*. *mobilis* strain was constructed by introducing chimeric xylA, xylB, tal, and tkt genes into the ZW1 strain. The xylB, tal, and tkt coding regions were from *E. coli* genes as in the ZW658 strain described in General Methods. The xylA coding region was from *Actinoplanes missouriensis* (AMxylA) which is disclosed in commonly owned and co-pending US2011-0318801, as encoding an enzyme having higher activity than the *E. coli* xylose isomerase in *Z. mobilis.* The coding region for the AMxylA was codon optimized for expression in *Z. mobilis* (SEQ ID NO:13). Additional copies of *Z. mobilis* rpi and rpe genes were also introduced in order to increase ribose-5-phosphate-isomerase (RPI) and ribulose-phosphate 3-epimerase (RPE) activities. Double crossover (DCO) transformation vectors were designed to specifically integrate the chimeric genes into target regions in *Z. mobilis* genome.

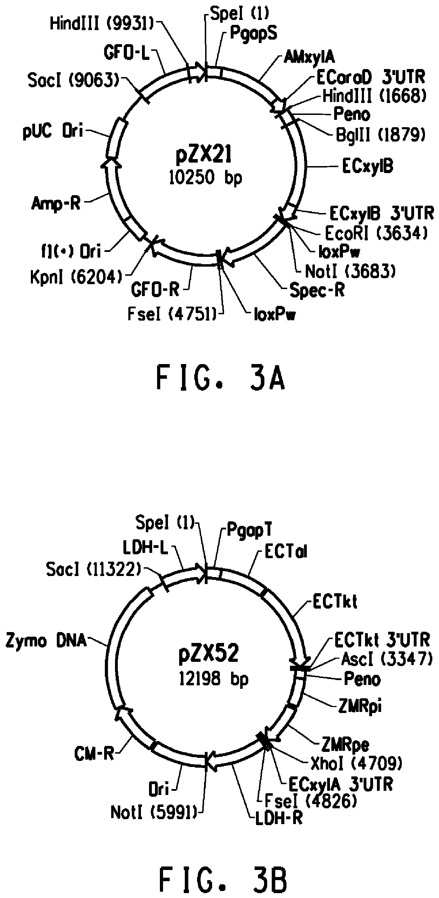

Standard molecular recombination methods were used to construct DCO (double cross over) suicide integration vectors. To express xylose isomerase and xylulose kinase in *Z. mobilis,* a 10,250-bp DCO suicide vector pZX21 (SEQ ID NO:15; Figure 3A) was constructed. This vector has a pBluescript backbone which contains a replication origin for *E. coli* but no *Z. mobilis* replication origin, thus it cannot be propagated in *Z. mobilis* making it a suicide vector. It contains DNA sequences from the *Z mobilis* gene encoding glucose-fructose oxidoreductase, GFO-L and GFO-R, flanking the sequences to be integrated. Both fragments were synthesized by PCR, using *Z. mobilis* genomic DNA as template. The 1,186-bp GFO-L fragment (SEQ ID NO:16) includes the first 654 bp (from nt-1 to nt-653) of the gfor coding sequence (SEQ ID NO:17) and 533 bp of upstream genomic sequence. The 1,446-bp GFO-R fragment (SEQ ID NO:18) includes the last 480 bp (from nt-823 to nt-1302) of the GFOR coding sequence and 966 bp of downstream genomic sequence. The GFO-L and GFO-R sequences direct integration into the gfor locus, replacing a segment of the gfor coding sequence (from nt-655 to nt-822) in the *Z. mobilis* genome. This disrupts expression of glucose-fructose oxidoreductase, which reduces xylitol production and increases ethanol production as disclosed in US 7,741,119.

The region in pZX21 between GFO-L and GFO-R includes three chimeric genes. One is a 1,661-bp chimeric xylA gene (SEQ ID NO:19) containing the 304-bp *Z. mobilis* Super GAP promoter (P_{gapS}; described in US 7,989,206), a 1,185-bp *A. missouriensis* xylA coding sequence (AMxylA) and a 166-bp *E. coli ara*D 3'UTR with a 5' Xbal site (ECaraD 3'UTR). The AMxylA coding region was optimized for expression in *Z. mobilis* according to codon bias of *Z. mobilis* ZM4 (SEQ ID NO:13). The ECaraD 3'UTR was from the *E. coli* araBAD operon. The second gene is a 1,960-bp chimeric xylB gene (SEQ ID NO:20) containing a 191 bp Pₑₙₒ, a 1,455-bp *E*. *coli* xylB coding sequence (ECxylB) and a 314-bp *E.coli* xylB 3'UTR (ECxylB 3'UTR). Pₑₙₒ is a strong constitutive promoter from the *Z. mobilis* genomic DNA having approximately 28% activity of P_{gap}. The third gene is a 1,014 bp aadA marker (for spectinomycin resistance; Spec-R) bounded by lox sites (SEQ ID NO:21). The marker can be removed after integration by expressing Cre recombinase.

To express transaldolase, transketolase, ribose-5-P-isomerase, and D-ribulose-P-3-epimerase in *Z. mobilis,* a 12,198-bp DCO shuttle vector pZX52 (SEQ ID NO:22; Figure 3B) was constructed. This vector is a *Zymomonas-E. coli* shuttle vector which is based on the vector pZB188 (Zhang et al. (1995) Science 267:240-243; US 5,514,583), which includes a 2,582 bp *Z. mobilis* genomic DNA fragment containing a replication origin allowing the vector to replicate in *Zymomonas* cells, and a 909-bp *E*. *coli* replication origin (Ori). It has a 911 bp chloramphenicol resistance marker (Cm-R) for selection of either *E*. *coli or Z. mobilis* transformants. pZX52 contains DNA sequences from the *Z mobilis* IdhA gene encoding lactate dehydrogenase, LDH-L (875 bp; SEQ ID NO:23) and LDH-R (1,149 bp; SEQ ID NO:24), flanking the sequences to be integrated. These sequences direct integration into the IdhA coding sequence (SEQ ID NO:25) in the *Z. mobilis* genome between nucleotides #493 and #494, thereby disrupting expression of lactate dehydrogenase.

The region in pZX52 between LDH-L and LDH-R includes two chimeric operons. The first one is a 3,339 bp P_{gapT}-Tal-Tkt operon (SEQ ID NO:26) containing a 304-bp T-mutant of the *Z. mobilis* GAP promoter (P_{gapT}), a 954-bp *E. coli* Tal coding region (ECTal), a 1,992-bp *E. coli Tkt* coding region, and a 68-bp *E. coli Tkt* 3'UTR (ECTkt 3'UTR). This operon is identical to the naturally existing *E. coli* Tal-Tkt operon except for the P_{gapT} promoter (SEQ ID NO:27), which is a P_{gap} with a "G" to an "A" change at position 83 and a "T" missing at position 285 as compared to the native P_{gap} (SEQ ID NO:3). The other chimeric operon is a 1,443 bp Pₑₙₒ-Rpi-Rpe operon (SEQ ID NO:28), containing a 191 bp Pₑₙₒ, a 471 bp *Z. mobilis* Rpi coding sequence with first codon changed to ATG (SEQ ID NO:43) (ZMRpi), a 663 bp *Z. mobilis* Rpe coding sequence (ZMRpe), and a 35 bp *E.coli* xylA 3'UTR (ECxylA 3'UTR).

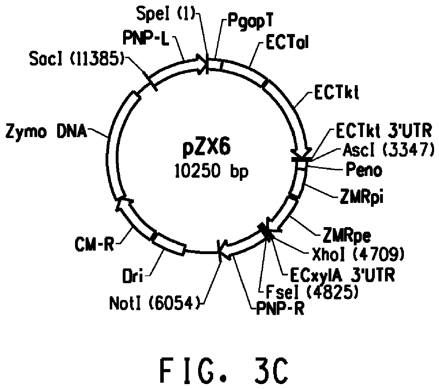

Another DCO shuttle vector named pZX6 (SEQ ID NO:29; Figure 3C) was constructed. This 12,704 bp vector is a modification of pZX52 having LDH-L and LDH-R sequences replaced with sequences from the *Z mobilis* pnp gene encoding polynucleotide phosphorylase. The 1,318 bp PNP-L fragment (SEQ ID NO:30) is a segment of the pnp coding sequence (SEQ ID NO:42) from nt-767 to nt-2,084, while the 1,225 bp PNP-R fragment (SEQ ID NO:31) includes the last 59 bp (from nt-2189 to nt-2247) of the pnp coding sequence and 1,166 bp of downstream genomic sequence. Therefore, pZX6 is able to direct integration of the P_{gapT}-Tal-Tkt operon and the Pₑₙₒ-Rpi-Rpe operon into the endogenous pnp gene near the end of the pnp coding sequence and replace a segment of the pnp coding sequence (from nt-2,084 to nt-2,188) in the *Z. mobilis* genome.

### Development of xylose utilizing Z. mobilis strains

The ZW1 strain was transformed with two plasmids in two steps. Competent cells of ZW1 were prepared by growing seed cells overnight in mRM3-G5 (1% yeast extract, 15 mM KH₂PO₄, 4 mM MgSO₄, and 50 g/L glucose) at 30 °C with 150 rpm shaking, to an OD₆₀₀ value near 5. Cells were harvested and resuspended in fresh medium to an OD₆₀₀ value of 0.05. The cells were grown under the same conditions to early to middle log phase (OD₆₀₀ near 0.5). Cells were harvested and washed twice with ice-cold water and then once with ice-cold 10% glycerol. The resulting competent cells were collected and resuspended in ice-cold 10% glycerol to an OD₆₀₀ value near 100. Since transformation of *Z. mobilis* requires non-methylated DNA, DCO plasmids pZX21, pZX52, and pZX6 were each transformed into *E. coli* SCS110 competent cells (Stratagene, La Jolla, CA). For each transformation, one colony of transformed cells was grown in 10 mL LB-Amp100 (LB broth containing 100 mg/L ampicillin) overnight at 37 °C. DNA was prepared from the 10 mL culture, using QIAprep Spin DNA Miniprep Kit (Qiagen).

Approximately 1 µg non-methylated pZX21 DNA was mixed with 50 µL ZW1 competent cells in a 1 MM Electroporation Cuvette (VWR, West Chester, PA). The plasmid DNA was electroporated into the cells at 2.0 KV using a BT720 Transporater Plus (BTX-Genetronics, San Diego, CA). Transformed cells were recovered in 1 mL MMG5 medium (10 g/L glucose, 10 g/L yeast extract, 5 g/L tryptone, 2.5 g/L (NH₄)₂SO₄, 2 g/L K₂HPO₄, and 1 mM MgSO₄) for 4 hours at 30 °C and grown on MMG5-Spec250 plates (MMG5 with 250 mg/L spectinomycin and 15 g/L agar) for 3 days at 30 °C, inside an anaerobic jar with an AnaeroPack (Mitsubishi Gas Chemical, New York, NY).

Since pZX21 is a DCO suicide vector, surviving Spec^{R} colonies had the P_{gapS}-AMxylA::Pₑₙₒ-ECxylB::Spec-R segment integrated into the gfor locus. The colonies were streaked and grown on a fresh MMG5-Spec250 plate, and then subjected to PCR to inspect chimeric gene integration. The first PCR used forward primer ara285 (SEQ ID NO:32) and reverse primer ara120 (SEQ ID NO:33) to inspect double crossover recombination mediated by the GFO-L fragment in pZX21. The ara285 primer matches a segment of *Z. mobilis* genomic sequence that is 494 bp upstream of the GFO-L fragment in the genome, while ara120 complements the last 18 bp of P_{gapS} and the first 17 bp of AMxylA in pZX21. If integration had occurred as designed, PCR would amplify a 1,903 bp fragment from the transformants. The 2nd PCR used forward primer ara46 (SEQ ID NO:34) and reverse primer ara274 (SEQ ID NO:35) to inspect double crossover recombination mediated by the GFO-R fragment in pZX21. The ara46 primer is a sequence near the end of the Spec^{R} gene in pZX21, while ara274 complements a segment of *Z. mobilis* genomic DNA that is 83 bp downstream of the GFO-R fragment. This PCR would amplify a 1,864-bp fragment from the colonies having successful integration. Both inspections produced the expected PCR products and thus confirmed accurate transgene integration. The resulting strain was named ZW1-pZX21.

In the second step, ZW1-pZX21 was transformed with pZX52 and selected on a MMG5-Spec250-CM120 (MMG5-Spec250 with 120 mg/L of chloramphenicol) plate. Because pZX52 is a DCO shuttle vector having the Cm^{R} marker for plasmid selection and a markerless integration segment (P_{gapT}-ECTal-ECTkt::Pₑₙₒ-ZMRpi-ZMRpe), the recovered colonies should contain not only the previously integrated construct P_{gapS}-AMxylA::Pₑₙₒ-ECxylB::Spec-R in the *Z. mobilis* genome, but also the non-integrated construct P_{gapT}-ECTal-ECTkt::Pₑₙₒ-ZMRpi-ZMRpe in the propagated pZX52 plasmid. These transformants should have all required genes for the xylose utilization pathway. To demonstrate that all transgenes were functional in *Z. mobilis,* ten selected colonies were subjected to a 48-hour growth assay in xylose. In the assay, 2 mL of mRM3-G5-Spec200-CM120 (mRM3-G5 with 200 mg/L spectinomycin and 120 mg/L chloramphenicol) in a 14 mL Falcon polypropylene round-bottom tube was inoculated with a selected colony and cultured overnight at 30 °C with 150 rpm shaking. Tubes were tightly capped, but a hole was punched in the top of the cap using a 23G1 needle for pressure release during cell growth and fermentation. Cells were harvested, washed with MRM3X10 (MRM3 with 100 g/L xylose), and resuspended in mRM3-X10-Spec200-CM120 (mRM3-X10 containing 200 mg/L spectinomycin and 120 mg/L chloramphenicol) to have a starting OD₆₀₀ of 0.1. Five mL of the suspension was placed in a new 14 mL Falcon polypropylene round-bottom tube. Tubes were capped with a hole on the top. Cells were grown for 48 hrs at 30 °C with 150 rpm shaking and OD₆₀₀ was measured on a Shimadzu UV-1201 Spectrophotometer. Then, 1 mL of culture was centrifuged at 10,000x g to remove cells. The supernatant was filtered through a 0.22 µm Costar Spin-X Centrifuge Tube Filter (Corning Inc, Corning, NY) and analyzed for xylose and ethanol by running through a BioRad Aminex HPX-A7H ion exclusion column (BioRad, Hercules, CA) with 0.01 N H₂SO₄ at a speed of 0.6 mL/min at 55 °C on an Agilent 1100 HPLC system (Agilent Technologies, Santa Clara, CA). Results indicated that all 10 of the transformants had acquired the xylose utilization pathway for ethanol production. The new strain was named ZW1-pZX21-pZX52 and one of the cultures was used in further experiments.

ZW1-pZX21-pZX52 then went through three post-transformation procedures sequentially for integration of the P_{gapT}-ECTal-ECTkt::Pₑₙₒ-ZMRpi-ZMRpe construct.
(1) The strain was adapted on xylose. In this procedure, ZW1-pZX21-pZX52 was suspended in a 5-mL mRM3-G1X9-Spec200-CM120 medium (MRM3 with 10 g/L glucose, 90 g/L xylose, 200 mg/L spectinomycin and 120 mg/L chloramphenicol) with a starting OD₆₀₀ value of 0.2 and grown for 3 to 4 doublings at 30°C (OD₆₀₀ value from 0.2 to 2; one passage). The culture was then diluted to the starting OD₆₀₀ value and grown for another passage. Totally, 4 passages (approximately 15 doublings) were completed.
(2) Plasmid curing and integration of the P_{gapT}-ECTal-ECTkt::Pₑₙₒ-ZMRpi-ZMRpe construct were carried out by growing 10 µL of the adaptation cell pool in 2 mL mRM3-G5-Spec200 medium at higher temperature (37 °C) for overnight. The 10 µL culture was then diluted in 2 mL mRM3-G5-Spec200 medium and grown for another passage. Totally, 5 passages were performed at 37 °C in glucose medium. As a result of the high temperature growth, the majority of the population should not host the pZP52 plasmid any more, but the P_{gapT}-ECTal-ECTkt::Pₑₙₒ-ZMRpi-ZMRpe construct (lacking a selective marker) should have been integrated into the IdhA gene of the *Z. mobilis* genome. A minority of the population may maintain pZX52, without integration.
(3) The population was enriched by growing 50 µL of the cell pool in 2 mL mRM3-X10-Spec200 at 30 °C for overnight. The enriched population was grown on a MMG5-Spec250 plate at 30 °C for overnight. Individual colonies were selected and streaked on MMG5 plates and MMG5-CM120 plates in replica. After incubating at 30 °C for overnight, those colonies that grew on MMG5 but not on MMG5-CM120 were selected for further PCR inspection. The first PCR used forward primer ara45 (SEQ ID NO:36) and reverse primer ara356 (SEQ ID NO:37) to inspect double crossover recombination mediated by the LDH-L fragment in pZX52. The ara45 primer matches a segment of *Z. mobilis* genomic DNA that is 86 bp upstream of the LDH-L fragment in the genome, and ara356 complements a fragment (from nt-91 to nt-112) of the ECTal coding region in pZX52. The PCR would amplify a 1,383-bp fragment from the colonies if integration had occurred as designed. The 2nd PCR used forward primer ara354 (SEQ ID NO:38) and reverse primer ara43 (SEQ ID NO:39) to inspect double crossover recombination mediated by the LDH-R fragment in pZX52. The ara354 primer is a sequence near the 3' end of ZMRpe in pZX52. The ara43 primer complements a segment of *Z. mobilis* genomic DNA that is 122 bp downstream of the LDH-R fragment. This PCR would amplify a 1,468 bp fragment from the colonies when recombination was as expected. Both PCRs produced DNA fragments with the expected sizes, which confirmed that the P_{gapT}-ECTal-ECTkt::Pₑₙₒ-ZMRpi-ZMRpe construct had been accurately integrated as designed in all inspected colonies. The resulting colonies were named ZW1-X109.

In a second approach, the ZW1-pZX21 strain was transformed with the pZX6 DCO shuttle vector and the three post-transformation procedures including the xylose-adaptation step were performed as described above for ZW1-X109, except that adaptation was for 10 passages rather than 4 passages. Therefore, the P_{gapT}-ECTal-ECTkt::Pₑₙₒ-ZMRpi-ZMRpe construct was targeted to the endogenous pnp gene. As described for construction of ZW1-X109, the 48-hour growth assay was preformed prior to the three post-transformation procedures to make sure that all transgenes were functioning as expected. After the three post-transformation procedures, the integration was also inspected by PCR. The first PCR used forward primer ara340 (SEQ ID NO:40) and reverse primer ara356 (SEQ ID NO:37) to inspect double crossover recombination mediated by the PNP-L fragment in pZX6. The ara340 primer matches *Z. mobilis* genomic DNA that is 75 bp upstream of the PNP-L fragment. The ara356 primer used here complements a fragment (from nt-91 to nt-112) of ECTal in pZX6. The PCR produced a 1,815-bp fragment from the transformants, as expected for an accurate integration event. The 2nd PCR used forward primer ara354 (SEQ ID NO:38) and reverse primer ara339 (SEQ ID NO:41) to inspect double crossover recombination mediated by PNP-R fragment in pZX6. In this case, the ara354 primer matches a sequence near the 3' end of ZMRpe in pZX6, and the ara339 primer complements a segment of *Z. mobilis* genomic DNA that is 59 bp downstream of the PNP-R fragment sequence. This PCR amplified a 1,549 bp fragment from the transformants, a size that was expected for successful integration. Therefore, PCR inspection confirmed that the P_{gapT}-ECTal-ECTkt::Pₑₙₒ-ZMRpi-ZMRpe construct had been accurately integrated in all inspected colonies. This new strain was named ZW1-X210.

In summary, two xylose utilizing *Z. mobilis* strains were rebuilt de novo from wild type ZW1. They both had a P_{gapS}-AMxylA::Pₑₙₒ-ECxylB::Spec-R construct integrated into the gfor locus. The ZW1-X109 strain had a P_{gapT}-ECTal-ECTkt::Pₑₙₒ-ZMRpi-ZMRpe construct integrated into the IdhA locus, while the ZW1-X210 strain had the same construct integrated in the endogenous pnp gene. Both strains had one marker gene in the integrated P_{gapS}-AMxylA::Pₑₙₒ-ECxylB::Spec-R construct, which could be removed by introduction of Cre recombinase.

### Characterization of new xylose utilizing Z. mobilis strains

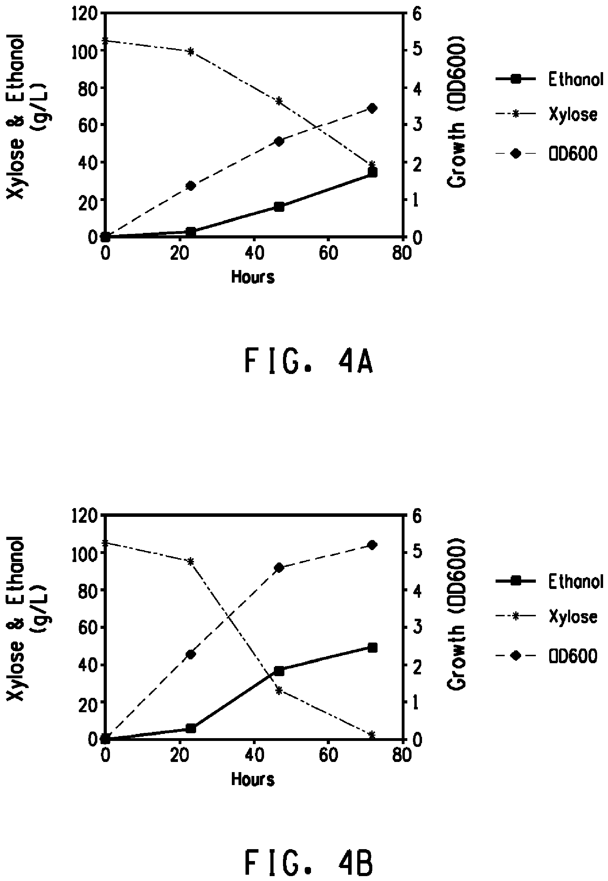

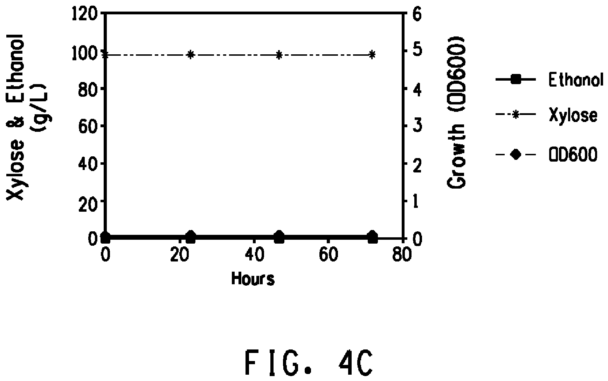

Shake flask fermentation was carried out in 20 mL mRM3-X10 in order to determine each strain's ability to use xylose. OD₆₀₀ value and both xylose and ethanol concentrations were measured at 0, 24, 48, and 72 hours. Figure 4 is a summary of the results for ZW1-X109 (A), ZW1-210 (B) and ZW1 (C). The results confirm that both new strains were able to ferment xylose. After 72 hours of fermentation, ZW1-X109 had utilized approximately 64.2% of xylose (a reduction from 105.6 g/L to 37.8 g/L) to support an ethanol titer of 31.5 g/L and biomass growth to OD₆₀₀ value of 3.51; ZW1-X210 had utilized almost all available xylose (a reduction from 105.6 g/L to 1.6 g/L) to support an ethanol titer of 48.5 g/L and a biomass growth to OD₆₀₀ value of 5.22. However, ZW1 could not grow in mRM3-X10 due to lacking the xylose metabolic pathway. Therefore, among new strains, ZW1-X210 could ferment xylose faster than ZW1-X109, in the xylose-containing single sugar medium. The major difference between ZW1-X109 and ZW1-X210 is that the P_{gapT}-ECTal-ECTkt::Pₑₙₒ-ZMRpi-ZMRpe construct was inserted into the IdhA locus in ZW1-X109, and into the endogenous pnp gene in ZW1-X210. This result indicates that interruption of the pnp gene may benefit xylose metabolism in *Z. mobilis.*

The genomes of the ZW1-X109 and ZW1-X210 strains were sequenced and compared to the ZW1 genomic sequence as described in Example 1. Among the differences were sequence changes in the *ZMO0976* coding region in both strains. In ZW1-X109 there was a G to A mutation at position 917 of the coding region sequence (in SEQ ID NO:1 native *ZMO0976* seq). This mutation changed codon 306 from TGG (encoding tryptophan) to the TAG stop codon, which shortens the normally 340 amino acid encoded protein (SEQ ID NO:2) by eliminating 35 amino acids at the C-terminus. In the ZW1-X210 strain there is a large deletion including 125 nucleotides of the *ZMO0976* promoter region and extending through 475 nucleotides of the coding region.

### Example 3

### Generation of a suicide construct to knockout the ZMO0976 gene in ZW1

To directly assess the effect of a *ZMO0976* mutation alone on xylitol production in the wild type ZW1 strain, a suicide construct that can insertionally inactivate this gene was generated as described below.

### Construction of pMODlinker-Cm

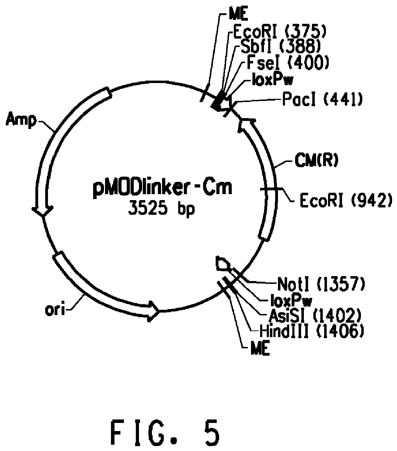

pMODlinker-Cm (Figure 5) was an important plasmid intermediate in the generation of a suicide construct (pAR-cm) that was designed to insertionally-inactivate ("knock out") the *ZMO0976* gene (Seo et al (2005) Nature Biotechnol. 23:63-68) in *Z. mobilis.* A DNA fragment that confers resistance to chloramphenicol (Cm^{r}) was inserted between the Notl and Pacl sites of the pMOD-Linker-Spec plasmid, which is described in detail in U.S. 7,989,206, replacing the DNA fragment that confers resistance to spectinomycin (Spec^{r}) as follows. pMOD-Linker-Spec was sequentially digested with Pacl and Notl, and the 2.6 kb vector DNA fragment was purified from a 1% agarose gel. The Cm^{r} gene with its associated promoter was then PCR amplified from the commercially available plasmid pACYC184 (Boca Scientific, Boca Raton, FL), using primer Cm-F-Notl (SEQ ID NO. 44; Notl site underlined) and primer Cm-R-Pacl (SEQ ID NO: 2; Pacl site underlined).
Primer Cm-F-Notl (SEQ ID NO: 44): CATCTTACTGCGGCCGCGTGACGGAAGATCACTTCGCAG
Primer Cm-R-Pacl (SEQ ID NO: 45): TCACTCATTTAATTAACTTATTCAGGCGTAGCACCAG
The 0.9 kb PCR product was also cut with Pacl and Notl, and the purified DNA fragment was ligated to the Pacl/Notl-cut vector fragment described above to yield pMODlinker-Cm (Fig. 5). This 3525 bp plasmid has a loxP-flanked, Cm^{r}-cassette that is located between the two mosaic ends (ME) that Tn5 transposase interacts with to form transposomes.

### Construction of plasmid pAR-cm

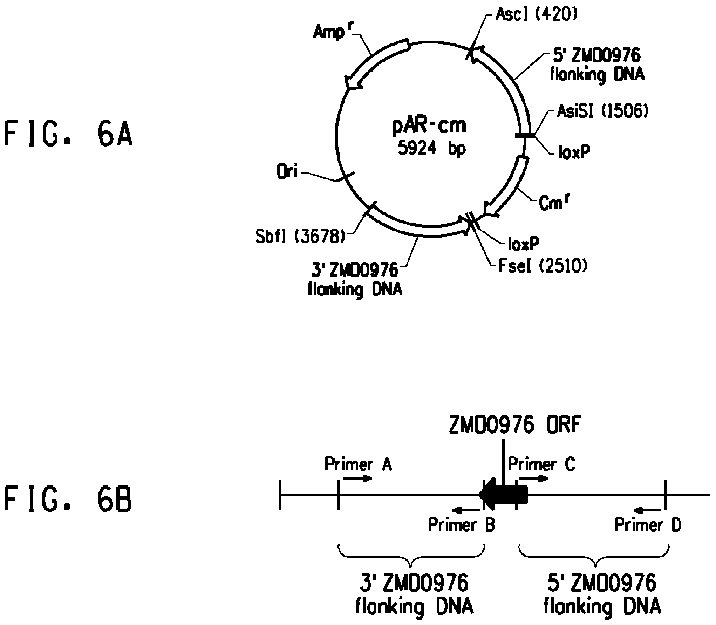

A suicide construct (pAR-cm) was used to knockout the *ZMO0976* gene in ZW1, via host-mediated double-crossover homologous recombination. The plasmid that was used for this manipulation is analogous to the suicide construct (pHimA) that was previously used to knockout the himA gene in ZW801-4. The construction of pHimA was described in detail in US 7,897,396. To generate the pAR-cm suicide construct (Figure 6A) that was used, the 5'- and 3'-himA flanking DNA fragments in pHimA were replaced with 5'- and 3'-*ZMO0976* flanking DNA fragments to target the selectable marker and double-crossover event to the chromosomal *ZMO0976* gene. Additionally, the loxP-flanked spectinomycin-resistance (Spec^{r}) cassette in pHimA was replaced with a loxP-flanked chloramphenicol-resistance (Cm^{r}) cassette. Four purified DNA fragments were required to the generate pAR-cm as described below.

Fragment 1 was obtained from pLDHSp-9WW that was previously described in US 7,897,396 by cutting the plasmid with four different restriction enzymes: Notl, Bsal, Sbfl and Ascl. Notl cuts pLDHSp-9WW at nt 2647 and Bsal cuts the plasmid at nt 1816. After the plasmid DNA was completely digested with the four restriction enzymes, the 2666 bp Sbfl-Ascl DNA fragment was purified by electrophoresis using a 1% agarose gel and the Zymoclean Gel DNA Recovery Kit (catalog #D4001, Zymo Research). This fragment, named Fragment 1, contains an *E. coli* origin of replication that is not functional in *Z. mobilis* and a gene that confers ampicillin-resistance in *E. coli.*

Fragment 2 corresponds to the 1002 bp stretch of DNA that is located between the Fsel and AsiSI sites in pMODlinker-Cm (Fig. 5). As indicated above, this DNA fragment contains a Cm^{r}-cassette that is flanked by two wild type loxP sites, one at each end. The complete DNA sequence of Fragment 2 is given in SEQ ID NO:46.

Fragment 3 contains 3'-*ZMO0976* flanking DNA. This ∼ 1.2 Kb DNA fragment was generated by PCR using Primers A (SEQ ID NO:47) and B (SEQ ID NO:48). The template for PCR-amplification was genomic DNA that was isolated from ZW658 (ATCC #PTA-7858) using the Wizard Genomic DNA Purification Kit (catalog #A1125, Promega).
Primer A (SEQ ID NO:47):
   CTACTCATcctgcaggCTTCTCGGTGATCGTGTTGC
Primer B (SEQ ID NO:48):
   TCACTCA TggccggccGAACAGA TCGACGGTATTGATG

The underlined bases of Primer A (forward primer) binds downstream from the *ZMO0976* coding region in the middle of a coding region for a hypothetical protein (product of the *ZMO0975* gene), while the lower case letters correspond to an Sbfl site that was added to the 5' end of the primer. The underlined bases of Primer B (reverse primer) hybridize to the 3' end of the *ZMO0976* open reading frame, while the lower case letters correspond to an Fsel site that was added to the 5' end of the primer. The chromosomal binding sites for Primers A and B and the PCR product that was generated are shown in Figure 6B. The PCR product was digested with Sbfl and Fsel, and the resulting 1168 bp fragment was then purified by agarose gel electrophoresis as described above.

Fragment 4 contains 5'-*ZMO0976* flanking DNA. This ∼1.1 kb DNA fragment was generated by PCR using Primers C (SEQ ID NO:49) and D (SEQ ID NO:50). The template for PCR-amplification was genomic DNA that was isolated from ZW658 (ATCC #PTA-7858) using the Wizard Genomic DNA Purification Kit (catalog #A1125, Promega).
Primer C (SEQ ID NO:49):
   CATCTTACTgcgatcgcGATCAATCGCCCGATGAATG
Primer D (SEQ ID NO:50):
   CATCTTACTggcgcgccTCGCCGTATTGTATCGCTG

The underlined bases of Primer C (forward primer) hybridize at the 5' end the of the *ZMO0976* open reading frame, while the lower case letters correspond to an AsiSI site that was added to the 5' end of the primer. The underlined bases of Primer D (reverse primer) hybridize upstream from the *ZMO0976* gene in the middle of the open reading frame of a gene that codes for a putative "periplasmic binding protein", while the lower case letters correspond to an Ascl site that was added to the 5' end of the primer. The chromosomal binding sites for Primers C and D and the PCR product that was generated are shown in Figure 6B. The PCR product was digested with AsiSI and Ascl, and the resulting 1086 bp fragment was purified by electrophoresis using a 1% agarose gel.

The four DNA fragments described above were then subjected to a 4-way ligation reaction to assemble the *ZMO0976* knockout construct (pAR-cm), which is shown in Figure 6A. The molar ratio of Fragments #1-#4 that was used for this reaction was approximately 1:1:1:1. An aliquot of the ligation reaction mixture was electroporated into *E. coli* DH10B and the transformed cells were plated on LB media that contained chloramphenicol (25 µg/ml). The plates were then incubated at 37 °C. Chloramphenicol-resistant tranformants that contained plasmids with the correct size inserts were initially identified by colony PCR using Primer A / Primer D. Subsequent confirmation of positive clones came from DNA sequence analysis of the pAR-cm plasmid DNA from the PCR positive clones.

To obtain non-methylated plasmid DNA needed for transformation of Z. *mobilis,* pAR-cm was introduced into *E. coli* SCS110 (dcm⁻, dam⁻), and the transformed cells were plated on LB medium that contained chloramphenicol (25 µg/ml); growth was at 37°C. The chemically competent cells that were used for this manipulation were obtained from Stratagene (Cat. No. 200247) and the vendor's protocol was followed. It is important to note that the use of non-methylated plasmid DNA for transformation of *Z. mobilis* stains that are derived from ZM4 is critical for success, since methylated plasmid DNA that is isolated from wild type strains of *E. coli,* like DH10B, is readily destroyed by the host's restriction/modification systems. In the last step, plasmid DNA was isolated from one of the SCS110 transformants using the QIAGEN Plasmid Plus Midi Kit (Cat. No. 12943), and the final concentration of DNA was ∼1.5 µg/µl.

### Example 4

### Generation of the ZW1 ZMO0976 knockout mutant

To inactivate the *ZMO0976* gene in ZW1, non-methylated pAR-cm plasmid DNA (which does not replicate in *Z. mobilis*) was introduced into ZW1 competent cells using electroporation as described in the GENERAL METHODS section. After a 3-hr recovery period in 1.0 ml MMG medium, the transformed cells were harvested by centrifugation (13,000 x g for 1 min) and cell pellets were resuspended in 300 µL of MMG media. Aliquots of the cell suspension were then plated onto MMG agar plates that contained 120 µg/ml chloramphenicol (MMG/Cm120 plates), and the plates were incubated for 3 days at 33 °C under anaerobic conditions. Two of the resulting chloramphenicol-resistant colonies were randomly selected for further manipulation. Both colonies were streaked onto an MMG/Cm120 plate and incubated for 24 hr as described above. The new patches were then re-streaked onto a fresh MMG/Cm120 plate, and after a 24-hr growth period under the same conditions the resulting cell patches were subjected to further analysis as described below.

As described in US 7,897,396, the initial interaction between the *Z. mobilis* chromosome and a suicide construct is a single-crossover event that takes place at one of the two flanking DNA sequences, and the single-crossover event eventually gives rise to double-crossover events. Transition to a double-crossover is normally very rapid (unless this event is lethal or results in a slower growth rate) and usually occurs after a few serial transfers in liquid or solid media that contains the selective agent for the suicide construct, in this case chloramphenicol. To confirm that the pAR-cm suicide construct had indeed undergone a double-crossover event at the correct location in the ZW1 chromosome in the two strains that were selected, colony PCR experiments were carried out using three different pairs of primers (Primer E/Primer F, Primer G/Primer H, Primer I/Primer J).
Primer E (SEQ ID NO:51):
   CTACTTCACTTCATGACCGG
Primer F (SEQ ID NO:52):
   AGTCATGCaggcctCTGATGAATGCTCATCCGGAA
Primer G (SEQ ID NO:53):
   GTCTGACGTTGATCCTGATC
Primer H (SEQ ID NO:54):
   TCACTCATggccggccTGCGTATAATATTTGCCCATGG
Primer I (SEQ ID NO:55):
   GTTCCTGCTTTGCTTTTGTGG
Primer J (SEQ ID NO:56):
   CCCGGAAGCTATCAAAATTTTG

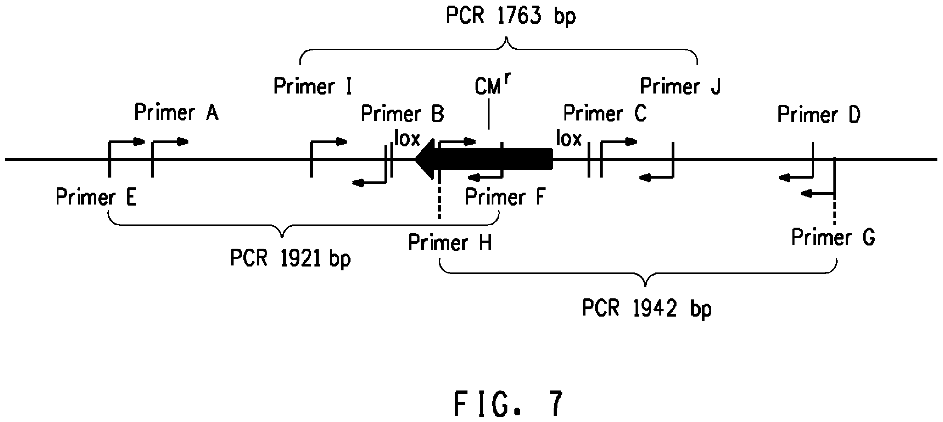

Primers E, I, J, and G hybridize to the *Z. mobilis* chromosome at the locations shown in Figure 7. The underlined bases of Primer F and H hybridize to the chloramphenicol-resistance (Cm^{r}) cassette that is inserted into the chromosome after the desired double-crossover event has occurred (Fig. 7). A 1921 bp PCR product using Primer E and F indicates that the correct single-crossover event occurred at one end of the *ZMO0976* gene, while a 1942 bp PCR product using Primer G and H indicates that the correct single-crossover event occurred at other end. Primers I and J can only generate a single 1763 bp PCR product if the correct double-crossover event has occurred. In contrast, this pair of primers would generate a 1468 bp PCR product with wild type ZW1 strain or a mixed population of transformants that had not yet completed the transition from the single-crossover event to the double-crossover event. The presence of the 1763 bp PCR product and the absence of the 1468 bp PCR product with Primers I and J indicate a homogeneous population of *ZMO0976* knockout mutants that have the desired double crossover event. Using the PCR strategy described above, both colonies that were selected for further analysis were shown to be true double-crossover events, and these two strains were named AR1 and AR2.

### Example 5

### Effect of insertional-inactivation of ZMO0976 on xylitol production in vivo and NADPH-dependent xylose reductase activity in cell-free extracts

To test the possibility that the wild type *ZMO976* gene encodes an enzyme that is able to convert D-xylose to xylitol in living cells, xylitol formation was monitored for wild type ZW1 and the two ZW1/*ZMO0976* knockout mutants (AR1 and AR2) during growth in a synthetic medium that contained both glucose and xylose. The seed cultures for this experiment were grown at 33 °C in 7 ml of 80 g/L glucose, 10 g/L yeast extract, 2 g/L KH₂PO₄, and 1 g/L MgSO₂(7H₂0) to an OD600 of ∼1.8. Aliquots of the seed cultures were then used to inoculate 10-ml cultures that contained 60 g/L glucose, 20 g/L xylose, 10 g/L yeast extract, 6 g/L KH₂PO₄, 1 g/L MgSO₂(7H₂0), and 2.5 g/L (NH₄)₂SO₄; the final pH was adjusted to pH 5.9 with concentrated potassium hydroxide. The cultures were grown at 33 °C and the initial OD₆₀₀ values were -0.11. After 0, 15.5, 39, 60, and 112 hours of growth, 1.0-ml aliquots of the cultures were removed for HPLC analysis using an HP 1100 equipped with a refractive index detector (Hewlett-Packard, Palo Alto, CA) to determine the concentration of xylitol that was present in the fermentation broth, using the methodology described in the General Methods section.

As shown in Figure 8, the rate and extent of xylitol production were much greater for wild type ZW1 than they were for the two ZW1/*ZMO0976* knockout mutants. For example, during the first 40 hours of the experiment the amount of xylitol in the fermentation broth for both AR1 and AR2 was below the level of detection for the HPLC, which is -0.1 g/L. And by the end of the experiment wild type ZW1 had generated ∼3- to 4-fold more xylitol than either of the ZW1/ *ZMO0976* knockout mutants; 0.736 g/L versus 0.254 g/L and 0.19 g/L for AR1 and AR2, respectively. It should be noted that the lower amounts of xylitol produced by AR1 and AR2 were not a consequence of lower cell density: the OD600 values for AR1 and AR2 at the 39-hr time point after all the glucose had been consumed were 7.29 and 6.98 compared to 6.25 for wild type ZW1.

The amount of xylitol generated by wild type ZW1 in the above experiment was similar to values previously reported for the same strain harboring the control plasmid pZB188/Kan under comparable experimental conditions (Fig. 14 A in US 7,741,084). However these values are at least 3-fold lower than the amount of xylitol that was generated when the xylose isomerase expression plasmid, pZB188/Kan-XylA, was introduced into wild type ZW1 (Fig. 14 B in US 7,741,084). The larger amounts of xylitol that are observed when xylose can be converted to xylulose by xylose isomerase is the consequence of glucose-fructose oxidoreductase (GFOR), which in turn is then able to convert xylulose to xylitol. As described in US 7,741,084, GFOR is the major contributor to xylitol formation *in vivo*, but only when glucose is present and a source of xylulose is available. The results in Figure 8 clearly demonstrate that the *ZMO0976* gene product is also a significant contributor to xylitol production *in vivo.* However, it is also evident that inactivation of this gene does not entirely eliminate xylitol production in vivo, even under conditions where the GFOR-mediated route for xylitol is inoperative due to the absence of xylose isomerase in the wild type strain, ZW1. To provide a direct demonstration that the *ZMO0976* gene product is able to catalyze the conversion of xylose to xylitol, cell-free extracts were prepared for wild type ZW1 and for AR1 and NADPH-dependent xylose reductase activities were measured. Preparation of the cell-free extracts and the spectrophotometric enzyme assay that was used for this experiment are described in the General Methods section. The specific activity of NADPH-dependent xylose reductase activity for wild type ZW1 was 14 mU/mg under the conditions employed. In marked contrast, NADPH-dependent xylose reductase activity was not detectable for the ZW1/*ZMO0976* knockout mutant. Indeed, the rate of NADPH disappearance in the cell-free extract prepared from the AR1 strain was slightly faster in the absence of xylose than in the presence of xylose. Thus it is safe to conclude from this experiment that elimination of the *ZMO0976* gene product in ZW1 reduced NADPH-dependent xylose reductase activity by greater than 90%.

### Example 6

### Inactivation of ZMO0976 allows immediate growth on xylose without adaptation and direct selection on xylose for a xylose-utilization pathway

To test the hypothesis that a functional *ZMO0976* gene constitutes a non-permissive condition for growth on xylose for recombinant strains of *Z. mobilis* that harbor the four genes that are required for xylose metabolism, plasmid pZB4 was introduced into wild type ZW1 and the ZW1/*ZMO976* knockout mutant, AR1. As described in US 5,514,583 Example 3, pZB4 is a multi-copy shuttle vector that replicates in *E. coli* and Z. *mobilis.* In addition to a tetracycline-resistance cassette that serves as the selectable marker, this plasmid contains the four genes that are needed to complete a xylose metabolic pathway for *Zymomonas,* which are expressed from two synthetic chimeric operons. One of the operons contains the *E*. *coli* xylose isomerase and xylulokinase coding regions under the control of the *Z*. *mobilis* Pgap promoter (P_{gap}-xylA/xylB operon), while the other consists of the *E. coli* transketolase and transaldolase coding regions that are driven by the *Z*. *mobilis* Peno promoter (P_{ENO}-tal/tkt operon).

Non-methylated pZB4 plasmid DNA was electroporated into ZW1 and AR1 as described in the General Methods section. After electroporation the entire 50-µl transformation reaction was added to 10 ml of MMG medium and the cells were allowed to recover for 5 hrs at 30 °C under static conditions. The cells were then harvested by centrifugation and 8 ml of the supernatant was removed and replaced with an equal volume of MMG medium (without MgS0₄) that contained 25 µg/ml of tetracycline. The resulting cultures were then incubated for -18 hrs at 33 °C to enrich for plasmid-bearing transformants. This enrichment step is very important since the transformation efficiency of pZB4 is extremely low, presumably due to the large size of this plasmid. Following the enrichment process the cells were collected by centrifugation and the cell pellets were resuspended in 800 µl of MMG (without MgS0₄). Next, a 100-µl aliquot of each cell suspension was plated onto an MMG agar plate (without MgS0₄) that contained tetracycline (20 µg/ml), and the plates were incubated at 33 °C in an anaerobic chamber. After 48 hrs there were -100 colonies on the plates for both the ZW1 and AR1 transformants and they were of similar size (2-3 mm) for both strains. Three ZW1/pZB4 colonies (-A, -B and -C) and three AR1/pZB4 colonies (-1, -2 and -3) were then randomly chosen for further characterization in the experiment described below without any additional manipulations. That all six strains harbored the pZB4 plasmid was confirmed by PCR analysis of resuspended cells using Primer TF-2 and Primer TR-9 (SEQ ID NOs:57 and 58). These oligonucleotides hybridize to the Peno-Tal/Tkt operon that is present in pZB4 and amplify a DNA fragment that is 1681 bp, and all six strains produced a DNA fragment with the correct size.

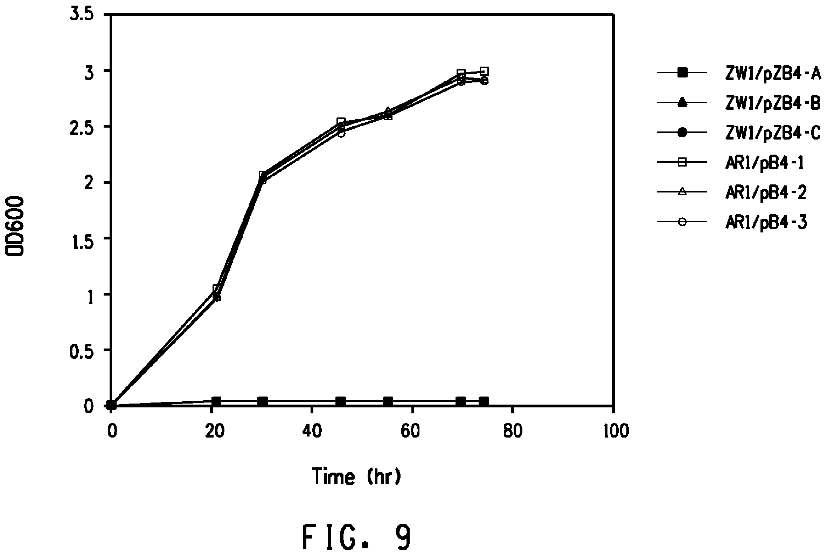

Figure 9 shows a shake flask experiment using mRM3-X10 (which contains 100 g/L xylose as the only sugar) plus 20 µg/ml of tetracycline as the test medium. The inocula used for this study were the six colonies from the glucose/tetracycline agar plates that were described above, which previously had not been exposed to xylose. Cultures were grown at 33 °C and the initial OD600 was ∼0.035. The three ZW1/pZB4 transformants that had a wild type *ZMO0976* gene failed to grow on xylose despite the fact they contain all four genes that are required for xylose metabolism (Figure 9). Indeed, even after a 3-day incubation period the OD600 of these cultures only increased about 60%, which constitutes less than one doubling. In striking contrast, when the three ZW1/ *ZMO976* knockout mutant colonies that harbored the pZB4 plasmid were transferred from the MMG/tetracycline plate into xylose-containing medium, growth began immediately without the need for a preliminary xylose-adaptation step (Figure 9).

Given the above results, experiments were performed to test whether it was possible to recover AR1/pZB4 transformants by plating them directly onto solid medium that contained xylose as the sole carbon source, without tetracycline. The pZB4 plasmid DNA was introduced into ZW1 and AR1 using the same protocol that is described above including the 18-hr enrichment step with tetracycline. Following this procedure, the cells were harvested by centrifugation, washed twice with 1.0 ml of MMX medium (same as MMG medium but contains 50 g/L xylose instead of glucose) and finally resuspended in 800 µl of the same medium. One hundred microliter aliquots of the resulting cell suspensions were then spread onto an MMX agar plate and an MMG agar plate (without MgS0₄) that contained tetracycline (20 µg/ml), and the plates were incubated at 33 °C under anaerobic conditions. After a 24-hr incubation period there were 132 and 118 colonies on the MMG/tetracycline plates for ZW1 and AR1, respectively. Thus the transformation efficiency with the pZB4 plasmid DNA was virtually identical for both strains. However, very different results were obtained when the same cell suspensions were plated directly onto MMX agar plates lacking tetracycline: After a 100-hr incubation period there were 85 colonies (∼1 mm) for AR1 and no colonies for the ZW1 parent strain that has a wild type *ZMO0976* gene.

The above results clearly demonstrate that a functional *ZMO0976* gene product presents a major obstacle for growth on xylose for recombinant strains of *Z*. *mobilis* that are genetically engineered for xylose metabolism. This enzyme is an NADPH-dependent aldose reductase that is able to convert xylose to xylitol. Although xylitol per se does *not* inhibit bacterial growth or cause cell death it is a well-known alternate substrate for *E. coli* xylulokinase, which phosphorylates it in the presence of ATP to form the toxic compound xylitol 5-phosphate. However, as shown in Figure 8, inactivation of the *ZMO0976* gene resulted in a 3- to 4-fold reduction in xylitol formation *in vivo*, and this allowed the AR1/pZB4 transformants to immediately grow on xylose when a xylose-utilization pathway was introduced. In other words, the AR1/pZB4 transformants are able to grow on xylose without an adaptation step because they generate less xylitol and hence form less xylitol 5-phosphate than the ZW1/pZB4 transformants. The experiment described above further demonstrates that inactivation of the *ZMO0976* gene can allow direct selection on xylose, provided the xylose pathway enzymes that are introduced can provide sufficient carbon flux to support bacterial growth when xylose is the only carbon source available.

### Example 7

### Adaptation of a ZW1/pZB4 transformant results in at least three different types of mutations

As shown in Figure 9, the ZW1/pZB4-A transformant that was obtained through tetracycline-selection on a glucose plate did not grow on xylose when it was inoculated into mRM3-X10 medium that contained 20 µg/ml tetracycline. Growth was also not observed when this strain was inoculated into MMX10 medium (same as MMX medium but contains 100 g/L xylose instead of 50 g/L xylose) that lacked tetracycline. Indeed, the latter experiment was performed eight different times using an initial OD of ∼0.2, and there was no increase in turbidity for any of the cultures even after a 6-day incubation period at 33 °C. However, we were able to adapt the ZW1/pZB4-A strain for growth on xylose by using a mixture of glucose and xylose as described in more detail below.

ZW1/pZB4-A glycerol stock was inoculated into 10-ml of mRM3-G5 medium that contained 20 µg/ml tetracycline and the initial OD was 0.1; mRM3-G5 is identical to mMR3-G10 but contains 50 g/l glucose instead of 100 g/L. After a 9-hr incubation period at 33 °C the OD increased to ∼2.0, and the cells were collected by centrifugation. The cell pellet was resuspended in 250 ml of 45 g/L xylose, 5 g/L glucose, 10 g/L yeast extract, 5 g/L of tryptone, 2.5 g/L of (NH₄)₂SO₄, 0.2 g/L K₂HPO₄, and 0.1 mM MgS0₄ and 20 µg/ml tetracycline to an initial OD of 0.08. Twenty three milliliter aliquots of the resuspended cells were then distributed to eight 50-ml loosely capped test tubes and the cultures were gently shaken at 33 °C for 86 hrs. During the incubation period the OD values increased to ∼1.0, and an aliquot of each culture was then added to 5 ml of mRM3-X10 medium. The new cultures were incubated at 33 °C for 28.5 hrs during which time their OD values increased from -0.04 to 0.7-1.1. Aliquots from each of the eight cultures were then individually plated onto an MMG agar plate (without MgS0₄) that contained 20 µg/ml tetracycline to isolate single colonies. After a 2-day incubation period at 33 °C, two colonies from each plate were randomly selected for further characterization; these colonies were named after the original eight cultures and are referred to below as strains 1A, 1B, 2A, 2B, 3A, 3B, 4A, 4B, 5A, 5B, 6A, 6B, 7A, 7B, 8A and 8B.

A preliminary experiment was performed to see how well the various strains grew on xylose. Each of the 16 colonies was separately inoculated into 5 ml of mRM3-X10 media and the cultures were incubated for 21.5 hrs at 33 °C. Based on the extent of growth during this experiment (increase in OD), one colony from each plate was chosen for further characterization and it was the one that grew the best in xylose. The selected strains were 1A, 2B, 3B, 5B, 6A, 6B, 7A and 8B, representing seven of the eight original cultures. Since strains 4A and 4B barely grew on xylose during the preliminary experiment, they were not included in the study described below, and both 6A and 6B were included.

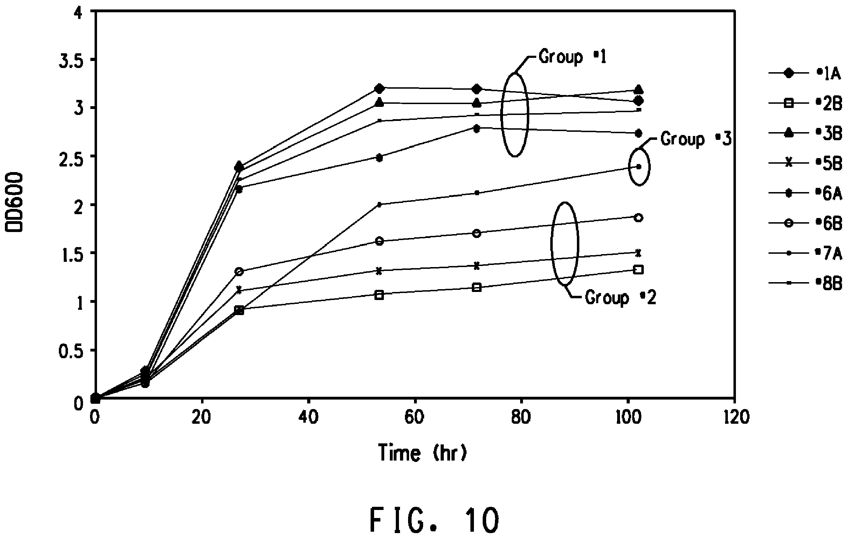

A shake flask experiment with mRM3-X10 as the growth medium was performed using the eight adapted strains that were selected above. The initial OD of the cultures was ∼0.05 and the temperature was 33 °C; the pre-cultures that were used as inocula for this experiment were also grown in mRM3-X10. Based on growth in xylose and HPLC analysis of the fermentation broth the strains appeared to fall into three different groups as shown in Figure 10. Group #1 strains (1A, 3B, 6A and 8B) all behaved similarly and grew faster on xylose than the other four strains. They also grew to a higher final OD. Group #2 strains (2B, 5B and 6B) also grew with similar kinetics, but their growth rates and final OD values were much lower than Group #1 strains. Although the Group #3 strain (7A) was the slowest grower, it eventually reached a higher OD than the Group #2 strains.

Table 1 shows endpoint values (102 hrs) for xylose, xylulose, ribulose, xylitol, glycerol, acetate and ethanol in the fermentation broth of the eight cultures described above as determined by HPLC analysis. Note that all four Group #1 strains generated large amounts of xylitol (>3 g/L), and this was also true for the slow growing Group #3 strain, 7A. In marked contrast, the amount of xylitol that was present in the fermentation broth for strains 2B, 5B and 6B was below the level of detection and could not be quantified. The three groups also had different patterns and levels for other by-products apart from xylitol. For example, the Group #1 and Group #3 strains generated significant amounts of acetate, but relatively low levels of ribulose and glycerol. On the other hand, the Group #2 strains produced large amounts of ribulose and glycerol, but barely made any acetate. Finally, only the Group #3 strain (7A) produced a large amount of xylulose (∼ 4 g/L) which is the natural substrate for the second enzyme in the engineered xylose pathway, namely xylulokinase. It was also interesting that although the Group #1 strains grew faster in xylose and to higher final ODs than the Group #2 strains, their rates of ethanol production were significantly slower than the other two groups.

**Table 1 Fermentation broth analysis of strains adapted for growth in xylose-containing medium.**

| Strain | Xylose | Xylulose | Ribu lose | Xylitol | Gycerol | Ace tate | EtOH | *ZM0976* Mutation | E. coli XylB Mutation |
|---|---|---|---|---|---|---|---|---|---|
| *Group* #*1* | | | | | | | | | |
| #1A | 55.06 | 0 | 0.99 | 3.39 | 0.68 | 0.74 | 18.55 | NO | YES |
| #3B | 56.52 | 0 | 0.97 | 3.26 | 0.69 | 0.74 | 17.08 | NO | YES |
| #6A | 56.95 | 0 | 1.05 | 2.98 | 0.75 | 0.49 | 18.15 | NO | YES |
| #8B | 56.30 | 0 | 0.94 | 3.25 | 0.69 | 0.71 | 17.72 | NO | YES |

| *Group #2* | | | | | | | | | |
|---|---|---|---|---|---|---|---|---|---|
| #2B | 46.64 | 0 | 4.08 | 0 | 3.19 | 0 | 21.59 | YES | NO |
| #5B | 46.10 | 0 | 4.45 | 0 | 3.63 | 0 | 21.73 | YES | NO |
| #6B | 41.13 | 0 | 4.50 | 0 | 3.56 | 0 | 23.25 | YES | NO |

| *Group #3* | | | | | | | | | |
|---|---|---|---|---|---|---|---|---|---|
| #7A | 49.79 | 3.97 | 0 | 4.53 | 1.06 | 1.07 | 19.06 | NO | *nd |

| | | | | | | | | | |
|---|---|---|---|---|---|---|---|---|---|
| nd = not determined | | | | | | | | | |

To determine whether any of the eight adapted strains had mutations in *ZMO0976,* the gene was PCR-amplified using Primer I and Primer G (SEQ ID NOS:55 and 53) and resuspended cells as a template. The DNA fragment that was generated contained the entire *ZMO0976* coding region plus ∼900 nucleotides upstream from the start codon and ∼200 nucleotides downstream from the stop codon. The resulting PCR products were subjected to DNA sequence analysis using six different primers (SEQ ID NOs:59 - 64).

Only the Group #2 strains had a mutation in the *ZMO0976* gene. Strain #5B had an 8-bp deletion in the open reading frame that resulted in a frame shift. The start of the deletion was 858 nucleotides downstream from the first nucleotide of the initiation codon. The two other Group #2 strains (2B and 6B) were identical siblings that had a G to T point mutation in the open reading frame at nucleotide 349 that converted amino acid residue E117 to a stop codon. These results are consistent with the HPLC data that is shown in Table 1, since the Group #2 strains were the ones that generated only trace amounts of xylitol.

The large amounts of xylitol that were found in the fermentation broth for the Group #1 and Group #3 strains strongly suggested that these strains had evolved a different mechanism for coping with xylitol toxicity. As already indicated it is xylitol 5-phosphate, *not* xylitol, that is responsible for the inhibitory effects on *Z*. *mobilis* growth and viability, and the former compound is generated by *E. coli* xylulokinase in a reaction that requires ATP and xylitol. It thus seemed possible that the Group #1 and Group #3 strains had acquired mutations in the *E. coli* xylulokinase gene (xylB), which is present in the pZB4 plasmid DNA that was introduced into these strains. To test this hypothesis the *E. coli* xylB gene (NCBI accession number NC_000913, Gene ID: 948133) was amplified from the eight adapted strains using two PCR primers (SEQ ID NOs:65 and 66) and resuspended cells as a template.

The DNA fragment that was generated contained the entire xylB coding region and -300 nucleotides downstream from the stop codon. The resulting PCR products were subjected to DNA sequence analysis using six different primers (SEQ ID NOs:67-72). Sequencing results showed that all of the Group #1 strains (1A, 3B, 6A and 8B) had a point mutation in the *E. coli* xylulokinase open reading frame, and it was the exact same modification indicating they were siblings. All four strains had a G to A replacement at nucleotide 224 (relative to the first nucleotide of the start codon) that resulted in a single amino acid substitution, a G75D mutation. Presumably this mutation either lowers the specific activity of xylulokinase or reduces its substrate specificity towards xylitol. Either scenario would result in the formation of less xylulose 5-phosphate, allowing these strains to grow in xylose during on-going *ZMO0976-*mediated xylitol production.

To determine the effect of the xylB point mutation on xylulokinase enzyme activity, cell-free extracts were prepared for one of the Group #1 strains (3B) and one of the unadapted ZW1/pZB4 strains (ZW1/pZB4-B); the latter has a wild type xylulokinase gene. The extracts were prepared essentially as described in the General Methods section but the cells were harvested at an OD of ∼1.0. The resulting extracts were used to measure xylulokinase activity in an enzyme-coupled reaction with lactate dehydrogenase and pyruvate kinase. The 1.0-ml reactions were conducted in a quartz cuvette at 20 °C and contained the following components: 50 mM Tris-HCI (pH 7.5), 5 mM MgCl₂, 50 mM KCI, 1 mM ATP, 1 mM EDTA, 1 mM DTT, 20 units of lactate dehydrogenase, 80 units of pyruvate kinase,1.5 mM phosphoenolpyruvate, 0.2 mM NADH, 5 mM D-xylulose and the equivalent of 2.5-10 µl of cell-free extract. The xylulose was added last after establishing baseline conditions. Control reactions were identical, but no xylulose was added. Enzyme activity was measured spectrophotometrically as a function of time at 340 nm using an extinction coefficient of 6220 M⁻¹cm⁻¹ to monitor the conversion of NADH to NAD. Enzyme activities are expressed as U/mg of cell-free extract protein after correcting for the rate of NADH oxidation in the absence of xylulose; 1 U = 1 micromole of xylulose 5-phophate formed per minute. The signal-to-noise ratio for xylulose-dependent NADH oxidation was greater than 60 when 10 µl of cell-free extract from the unadapted ZW1/pZB4 strain was used for the assay (i.e. the rate of NADH disappearance in the presence of xylulose compared to the control reaction).

The average xylulokinase specific activity for the unadapted ZW1/pZB4 strain was 3.67 U/mg under the conditions employed, based on three replicate determinations that varied by less than 7%. In marked contrast, the mean specific activity for the adapted 3B strain was only 0.21 U/mg, based on duplicate determinations that varied by 16%. Thus the G75D point mutation in the adapted strain 3B reduced the specific activity of E. coli xylukinase by ∼95% compared to the wild type gene. Although this mutation clearly allows the cells to survive during ongoing ZMO0916-mediated xylitol, it is clear from the xylose shake flask experiments that are shown in Figure 10 that it also has a detrimental effect on the rates of xylose utilization and ethanol production. More important, all four strains that have this mutation generate significant amounts of xylitol (Table I) which greatly reduces the metabolic yield for ethanol production.

The nature of the mutation for the Group 3 strain, 7A, remains to be determined. Although there were no mutations in the plasmid-born *E. coli* xylB open reading frame, the data shown in Table I and Figure 10 strongly suggests that this adapted strain also has a very low level of xylulokinase enzyme activity. Indeed, in addition to accumulating large amounts of xylitol, 7A was the only strain that produced detectable amounts of xylulose, indicating that *E. coli* xylulokinase was not able to phosphorylate xylulose as fast as xylose isomerase could produce it.

In summary, inactivation of the ZMO0976 gene greatly reduces xylitol production and the accumulation of toxic xylitol 5-phosphate. This strategy also eliminates the need for a preliminary adaptation step and allows immediate growth on xylose for recombinant *Z*. *mobilis* strains that have a xylose metabolism pathway. More important, intentional inactivation of ZMO0976 minimizes the possibility of acquiring other spontaneous mutations that can occur during the xylose adaptation process that help the organism cope with xylitol toxicity, such as the *E. coli* xylB mutation described above.

## Claims

1. A method of making a xylose-competent *Zymomonas* cell comprising:
a) providing a *Zymomonas* host cell;
b) creating a genetic modification in said cell in at least one endogenous gene encoding an aldose reductase enzyme of EC 1.1.1.21 having the ability to convert xylose to xylitol in the presence of NADPH; and
c) introducing into said cell a xylose utilization metabolic pathway;
wherein the order of steps (b) and (c) is not specified, and optionally occur concurrently, wherein said xylose-competent *Zymomonas* cell has aldose reductase activity reduced by greater than 90% as compared with a *Zymomonas* cell lacking the genetic modification of step (b), wherein said genetic modification is not created in a step of adaptation for growth in xylose by prolonged growth in medium containing xylose, and wherein said xylose-competent *Zymomonas* cell is able to grow in medium containing xylose as the only sugar.

2. The method of claim 1 further comprising selecting the xylose-competent *Zymomonas* cell using medium containing xylose as the only sugar.

3. The method of claim 1 or 2 wherein the *Zymomonas* host cell is a wild type cell.

4. The method of any one of claims 1 to 3, wherein no genetic modification in addition to the genetic modifications of (b) and (c) is made to allow growth of the *Zymomonas* host cell on xylose.

5. The method of any one of claims 1 to 4 wherein the *Zymomonas* host cell is a *Zymomonas* cell that has not previously been exposed to xylose.

6. The method of any one of claims 1 to 5 wherein the endogenous gene encoding aldose reductase (a) encodes a protein having at least about 95% sequence identity to the amino acid sequence as set forth in SEQ ID NO:2 and/or (b) has a coding region sequence having at least about 95% identity to the nucleotide sequence as set forth in SEQ ID NO:1.

7. The method of any one of claims 1 to 6 wherein the genetic modification of step (b) is selected from the group consisting of mutation, insertion, deletion, and combinations thereof.

8. The method of any one of claims 1 to 7 wherein the xylose utilization metabolic pathway comprises a series of polynucleotides encoding polypeptides, each having xylose isomerase, xylulokinase, transketolase, or transaldolase enzymatic activity.

9. The method of claim 8 wherein the polypeptide having xylose isomerase activity is a Group I xylose isomerase and is included in the class of enzymes identified by EC 5.3.1.5, wherein preferably the polynucleotide encoding the xylose isomerase polypeptide is isolated from *Actinoplanes missouriensis.*

10. The method of claim 9 wherein the polynucleotide encoding the xylose isomerase polypeptide is operably linked to a mutant glyceraldehyde-3-phosphate dehydrogenase gene promoter, wherein the mutant promoter has higher activity than the native promoter.

11. The method of any one of claims 1 to 10 wherein the xylose-competent *Zymomonas* cell further comprises (a) at least one genetic modification which reduces glucose-fructose oxidoreductase activity and/or (b) a genetic modification which reduces expression of the endogenous *himA* gene.

12. The method of any one of claims 1 to 11 wherein the xylose-competent *Zymomonas* cell further comprises polynucleotides encoding polypeptides for arabinose utilization, each having L-arabinose isomerase activity, L-ribulokinase activity, or L-ribulose-5-phosphate-4-epimerase activity, and optionally a polynucleotide encoding a polypeptide that is an arabinose-proton symporter.

13. The method of any one of claims 1 to 12 wherein the xylose-competent *Zymomonas* cell further comprises a genetic modification which increases ribose-5-phosphate isomerase activity.

14. The method of any one of claims 1 to 13 further comprising adapting the xylose-competent *Zymomonas* cell in xylose-containing medium wherein xylose utilization is improved.

15. A method for producing ethanol comprising growing a xylose-competent and ethanol-producing *Zymomonas* cell produced by the method of any one of claims 1 to 14 under conditions wherein ethanol is produced and optionally collecting said ethanol.

## Patentansprüche

1. Verfahren zur Herstellung einer Xylose-kompetenten *Zymomonas*-Zelle, umfassend:
a) Bereitstellen einer *Zymomonas*-Wirtszelle;
b) Erzeugen einer genetischen Modifikation in der Zelle in zumindest einem endogenen Gen, das für ein Aldosereduktase-Enzym mit EC 1.1.1.21 codiert, das die Fähigkeit besitzt, Xylose zu Xylit in Gegenwart von NADPH umzuwandeln; und
c) Einbringen, in die Zelle, eines Xyloseverwertungs-Stoffwechselwegs;
wobei die Reihenfolge der Schritte (b) und (c) nicht festgelegt ist, und sie gegebenenfalls gleichzeitig stattfinden, wobei die Xylose-kompetente *Zymomonas*-Zelle eine um mehr als 90% reduzierte Aldosereduktase-Aktivität gegenüber einer *Zymomonas-*Zelle aufweist, der die genetische Modifikation aus Schritt (b) fehlt, wobei die genetische Modifikation nicht in einem Schritt zur Anpassung an Wachstum in Xylose durch verlängertes Wachstum in xylosehaltigem Medium erzeugt wird, und wobei die Xylose-kompetente *Zymomonas*-Zelle dazu fähig ist, in Medium zu wachsen, das Xylose als den einzigen Zucker enthält.

2. Verfahren nach Anspruch 1, das weiterhin Auswählen der Xylose-kompetenten *Zymomonas-Zelle* unter Verwendung von Medium umfasst, das Xylose als den einzigen Zucker enthält.

3. Verfahren nach Anspruch 1 oder 2, wobei die *Zymomonas-Wirtszelle* eine Wildtyp-Zelle ist.

4. Verfahren nach einem der Ansprüche 1 bis 3, wobei keine genetische Modifikation zusätzlich zu den genetischen Modifikationen aus (b) und (c) vorgenommen wird, um Wachstum der *Zymomonas*-Wirtszelle auf Xylose zu ermöglichen.

5. Verfahren nach einem der Ansprüche 1 bis 4, wobei die *Zymomonas*-Wirtszelle eine *Zymomonas*-Zelle ist, die Xylose vorher nicht ausgesetzt worden ist.

6. Verfahren nach einem der Ansprüche 1 bis 5, wobei das für Aldosereduktase codierende endogene Gen (a) für ein Protein codiert, das zumindest ungefähr 95% Sequenzidentität mit der Aminosäuresequenz aufweist, wie sie in SEQ ID NO: 2 aufgeführt ist, und/oder (b) eine Codierungsregionsequenz aufweist, die zumindest ungefähr 95% Identität mit der Nukleotidsequenz aufweist, wie sie in SEQ ID NO: 1 aufgeführt ist.

7. Verfahren nach einem der Ansprüche 1 bis 6, wobei die genetische Modifikation aus Schritt (b) aus der Gruppe ausgewählt ist, die aus Mutation, Insertion, Deletion und Kombinationen davon besteht.

8. Verfahren nach einem der Ansprüche 1 bis 7, wobei der Xyloseverwertungs-Stoffwechselweg eine Reihe von Polynukleotiden umfasst, die für Polypeptide codieren, die jeweils Xylose-Isomerase-, Xylulokinase-, Transketolase- oder Transaldolase-Enzymaktivität aufweisen.

9. Verfahren nach Anspruch 8, wobei das Polypeptid, das Xylose-Isomerase-Aktivität aufweist, eine Gruppe-I-Xylose-Isomerase ist und in die durch EC 5.3.1.5 identifizierte Klasse von Enzymen aufgenommen ist, wobei das für das Xylose-Isomerase-Polypeptid codierende Polynukleotid vorzugsweise aus *Actinoplanes missouriensis* isoliert ist.

10. Verfahren nach Anspruch 9, wobei das für das Xylose-Isomerase-Polypeptid codierende Polynukleotid mit einem mutanten Glyceraldehyd-3-phosphat-Dehydrogenase-Gen-Promoter wirkverknüpft ist, wobei der mutante Promoter höhere Aktivität als der native Promoter aufweist.

11. Verfahren nach einem der Ansprüche 1 bis 10, wobei die Xylose-kompetente *Zymomonas*-Zelle weiterhin (a) zumindest eine genetische Modifikation umfasst, die Glukose-Fruktose-Oxidoreduktase-Aktivität reduziert, und/oder (b) eine genetische Modifikation umfasst, die Expression des endogenen *himA*-Gens reduziert.

12. Verfahren nach einem der Ansprüche 1 bis 11, wobei die Xylose-kompetente *Zymomonas*-Zelle weiterhin Polynukleotide, die für Polypeptide für Arabinoseverwertung codieren, die jeweils L-Arabinose-Isomerase-Aktivität, L-Ribulokinase-Aktivität oder L-Ribulose-5-phosphat-4-Epimerase-Aktivität aufweisen, und gegebenenfalls ein Polynukleotid umfasst, das für ein Polypeptid codiert, das ein Arabinose-Protonen-Symporter ist.

13. Verfahren nach einem der Ansprüche 1 bis 12, wobei die Xylose-kompetente *Zymomonas*-Zelle weiterhin eine genetische Modifikation umfasst, die Ribose-5-phosphat-Isomerase-Aktivität erhöht.

14. Verfahren nach einem der Ansprüche 1 bis 13, das weiterhin Anpassen der Xylose-kompetenten *Zymomonas*-Zelle in xylosehaltigem Medium umfasst, wobei Xyloseverwertung verbessert wird.

15. Verfahren zur Produktion von Ethanol, umfassend Anzüchten einer Xylose-kompetenten und Ethanol produzierenden *Zymomonas*-Zelle, hergestellt durch das Verfahren nach einem der Ansprüche 1 bis 14, unter Bedingungen, bei denen Ethanol produziert wird, und gegebenenfalls Sammeln des Ethanols.

## Revendications

1. Méthode pour la fabrication d'une cellule de *Zymomonas* compétente avec du xylose comprenant:
a) la fourniture d'une cellule hôte de *Zymomonas*;
b) la création d'une modification génétique dans ladite cellule dans au moins un gène endogène codant pour une enzyme aldose réductase de EC 1.1.1.21 ayant la capacité de convertir le xylose en xylitol en présence de NADPH; et
c) l'introduction dans ladite cellule d'une voie métabolique d'utilisation de xylose;
dans laquelle l'ordre des étapes (b) et (c) n'est pas spécifié et optionnellement elles ont lieu simultanément, dans laquelle ladite cellule de *Zymomonas* compétente avec du xylose a une activité d'aldose réductase réduite de plus de 90% lorsqu'elle est comparée à une cellule de *Zymomonas* dénuée de la modification génétique de l'étape (b), dans laquelle ladite modification génétique n'est pas créée dans une étape d'adaptation pour une croissance dans du xylose par une croissance prolongée dans un milieu contenant du xylose et dans laquelle ladite cellule de *Zymomonas* compétente avec du xylose est capable de croître dans un milieu contenant du xylose comme seul sucre.

2. Méthode selon la revendication 1 comprenant en outre la sélection de la cellule de *Zymomonas* compétente avec du xylose en utilisant un milieu contenant du xylose comme seul sucre.

3. Méthode selon la revendication 1 ou 2, dans laquelle la cellule hôte de *Zymomonas* est une cellule de type sauvage.

4. Méthode selon l'une quelconque des revendications 1 à 3, dans laquelle il n'est pas fait de modification génétique en plus des modifications génétiques de (b) et (c) pour permettre la croissance de la cellule hôte de *Zymomonas* sur du xylose.

5. Méthode selon l'une quelconque des revendications 1 à 4, dans laquelle la cellule hôte de *Zymomonas* est une cellule de *Zymomonas* qui n'a pas été antérieurement exposée à du xylose.

6. Méthode selon l'une quelconque des revendications 1 à 5, dans laquelle le gène endogène codant pour une aldose réductase (a) code pour une protéine possédant au moins environ 95% d'identité de séquence avec la séquence d'acides aminés telle que présentée dans la SEQ ID NO:2 et/ou (b) a une séquence de région de codage possédant au moins environ 95% d'identité avec la séquence de nucléotides telle que présentée dans la SEQ ID NO:1.

7. Méthode selon l'une quelconque des revendications 1 à 6, dans laquelle la modification génétique de l'étape (b) est choisie dans le groupe constitué d'une mutation, d'une insertion, d'une délétion et d'une combinaison de celles-ci.

8. Méthode selon l'une quelconque des revendications 1 à 7, dans laquelle la voie métabolique d'utilisation de xylose comprend une série de polynucléotides codant pour des polypeptides, chacun ayant une activité enzymatique de xylose isomérase, de xylulokinase, de transcétolase ou de transaldolase.

9. Méthode selon la revendication 8, dans laquelle le polypeptide ayant une activité de xylose isomérase est une xylose isomérase du Groupe I et elle est incluse dans la classe d'enzymes identifiée par EC 5.3.1.5, dans laquelle de préférence le polynucléotide codant pour le polypeptide de xylose isomérase est isolé à partir de *Actinoplanes missouriensis.*

10. Méthode selon la revendication 9, dans laquelle le polynucléotide codant pour le polypeptide de xylose isomérase est lié de manière fonctionnelle à un promoteur de gène de glycéraldéhyde-3-phosphate déshydrogénase mutant, dans laquelle le promoteur mutant a une activité plus élevée que celle du promoteur naturel.

11. Méthode selon l'une quelconque des revendications 1 à 10, dans laquelle la cellule de *Zymomonas* compétente avec du xylose comprend en outre (a) au moins une modification génétique qui réduit l'activité de glucose-fructose oxydoréductase et/ou (b) une modification génétique qui réduit l'expression du gène *himA* endogène.

12. Méthode selon l'une quelconque des revendications 1 à 11, dans laquelle la cellule de *Zymomonas* compétente avec du xylose comprend en outre des polynucléotides codant pour des polypeptides pour une utilisation d'arabinose, chacun ayant une activité de L-arabinose isomérase, une activité de L-ribulokinase ou une activité de L-ribulose-5-phosphate-4-épimérase et optionnellement un polynucléotide codant pour un polypeptide qui est un symport d'arabinose-protons.

13. Méthode selon l'une quelconque des revendications 1 à 12, dans laquelle la cellule de *Zymomonas* compétente avec du xylose comprend en outre une modification génétique qui augmente l'activité de ribose-5-phosphate isomérase.

14. Méthode selon l'une quelconque des revendications 1 à 13 comprenant en outre l'adaptation de la cellule de *Zymomonas* compétente avec du xylose dans un milieu contenant du xylose, dans laquelle l'utilisation de xylose est améliorée.

15. Méthode pour la production d'éthanol comprenant la croissance d'une cellule de *Zymomonas* compétente avec du xylose et produisant de l'éthanol produite par la méthode selon l'une quelconque des revendications 1 à 14 dans des conditions dans lesquelles de l'éthanol est produit et optionnellement la récupération dudit éthanol.


### SEQUENCE LISTING

<110> E. I. du Pont de Nemours and Company
<120> GENE INACTIVATION ALLOWING IMMEDIATE GROWTH ON XYLOSE MEDIUM BY ENGINEERED ZYMOMONAS
<130> CL5570PCT
<160> 72
<170> PatentIn version 3.5
<210> 1
   <211> 1023
   <212> DNA
   <213> Zymomonas mobilis
<400> 1
   atgaacactt ctacgcaaaa acccgctcat ttcgacaaga tttcgatcaa agggattgat      60
   aaatccgcaa cccgtgtagc gttaggcaca tgggctattg gtggctggat gtggggcggc     120
   actgatgacg atgcctccat taaaaccatt catcgggcga ttgatcttgg tatcaatatc     180
   atcgacaccg ctccggctta tggccgtggc catgctgaag aagtcgttgg taaagccatc     240
   aaaggtcaac gcgataattt gattattgcg accaaagtcg gccttgattg gactttaacc     300
   cccgaccaat cgatgcgccg taacagttca gccagccgta tcaaaaaaga aatcgaagat     360
   tctctgcgcc gccttggcac tgattatatc gacctttatc aggtgcattg gccggatccg     420
   ctggttccga ttgaagaaac cgcaacaata ttggaagccc tcagaaaaga aggcaaaatc     480
   cgttctatcg gcgtttccaa ttattccgtt cagcagatgg acgagttcaa gaaatatgcc     540
   gagctggccg tttcgcagtc gccttataat ctgtttgaac gcgaaataga caaagacatc     600
   ctgccctatg ccaagaaaaa cgatctggtc gttttaggct atggtgcgct ttgccgtggt     660
   ttactttctg gcagaatgac ggcggatcgt gcctttacag gcgatgattt acggaaaaca     720
   gacccgaaat tccagaaacc gcgctttgaa cattatctgg ccgcggttga agaactgaag     780
   aaactcgcca aagagcatta caataaatcg gtgttggctt tggctatccg ctggatgttg     840
   gagcaagggc ccactttagc actttggggc gctcgcaagc cggaacagat cgacggtatt     900
   gatgaagttt ttggctggca gatatcggat gaagatctga aacagattga tgctattctg     960
   gccaagaata tccccaatcc tatcggtgca gaatttatgg cacccccgcc acgcgataaa    1020
   taa                                                                  1023
<210> 2
   <211> 340
   <212> PRT
   <213> Zymomonas mobilis
<400> 2
   Met Asn Thr Ser Thr Gln Lys Pro Ala His Phe Asp Lys Ile Ser Ile
   1               5                   10                  15
   Lys Gly Ile Asp Lys Ser Ala Thr Arg Val Ala Leu Gly Thr Trp Ala
               20                  25                  30
   Ile Gly Gly Trp Met Trp Gly Gly Thr Asp Asp Asp Ala Ser Ile Lys
           35                  40                  45
   Thr Ile His Arg Ala Ile Asp Leu Gly Ile Asn Ile Ile Asp Thr Ala
       50                  55                  60
   Pro Ala Tyr Gly Arg Gly His Ala Glu Glu Val Val Gly Lys Ala Ile
   65                  70                  75                  80
   Lys Gly Gln Arg Asp Asn Leu Ile Ile Ala Thr Lys Val Gly Leu Asp
                   85                  90                  95
   Trp Thr Leu Thr Pro Asp Gln Ser Met Arg Arg Asn Ser Ser Ala Ser
               100                 105                 110
   Arg Ile Lys Lys Glu Ile Glu Asp Ser Leu Arg Arg Leu Gly Thr Asp
           115                 120                 125
   Tyr Ile Asp Leu Tyr Gln Val His Trp Pro Asp Pro Leu Val Pro Ile
       130                 135                 140
   Glu Glu Thr Ala Thr Ile Leu Glu Ala Leu Arg Lys Glu Gly Lys Ile
   145                 150                 155                 160
   Arg Ser Ile Gly Val Ser Asn Tyr Ser Val Gln Gln Met Asp Glu Phe
                   165                 170                 175
   Lys Lys Tyr Ala Glu Leu Ala Val Ser Gln Ser Pro Tyr Asn Leu Phe
               180                 185                 190
   Glu Arg Glu Ile Asp Lys Asp Ile Leu Pro Tyr Ala Lys Lys Asn Asp
           195                 200                 205
   Leu Val Val Leu Gly Tyr Gly Ala Leu Cys Arg Gly Leu Leu Ser Gly
       210                 215                 220
   Arg Met Thr Ala Asp Arg Ala Phe Thr Gly Asp Asp Leu Arg Lys Thr
   225                 230                 235                 240
   Asp Pro Lys Phe Gln Lys Pro Arg Phe Glu His Tyr Leu Ala Ala Val
                   245                 250                 255
   Glu Glu Leu Lys Lys Leu Ala Lys Glu His Tyr Asn Lys Ser Val Leu
               260                 265                 270
   Ala Leu Ala Ile Arg Trp Met Leu Glu Gln Gly Pro Thr Leu Ala Leu
           275                 280                 285
   Trp Gly Ala Arg Lys Pro Glu Gln Ile Asp Gly Ile Asp Glu Val Phe
       290                 295                 300
   Gly Trp Gln Ile Ser Asp Glu Asp Leu Lys Gln Ile Asp Ala Ile Leu
   305                 310                 315                 320
   Ala Lys Asn Ile Pro Asn Pro Ile Gly Ala Glu Phe Met Ala Pro Pro
                   325                 330                 335
   Pro Arg Asp Lys
               340
<210> 3
   <211> 305
   <212> DNA
   <213> Zymomonas mobilis
<400> 3
   gttcgatcaa caacccgaat cctatcgtaa tgatgttttg cccgatcagc ctcaatcgac     60
   aattttacgc gtttcgatcg aagcagggac gacaattggc tgggaacggt atactggaat    120
   aaatggtctt cgttatggta ttgatgtttt tggtgcatcg gccccggcga atgatctata    180
   tgctcatttc ggcttgaccg cagtcggcat cacgaacaag gtgttggccg cgatcgccgg    240
   taagtcggca cgttaaaaaa tagctatgga atataatagc tacttaataa gttaggagaa    300
   taaac                                                                305
<210> 4
   <211> 304
   <212> DNA
   <213> artificial sequence
<220>
   <223> mutated promoter
<400> 4
   gttcgatcaa caacccgaat cctatcgtaa tgatgttttg cccgatcagc ctcaatcgac    60
   aattttacgc gtttcgatcg aagcagggac gacaattggc tgggaacggt atacttgaat   120
   aaatggtctt cgttatggta ttgatgtttt tggtgcatcg gccccggcga atgatctata   180
   tgctcatttc ggcttgaccg cagtcggcat cacgaacaag gtgttggccg cgatcgccgg   240
   taagtcggca cgttaaaaaa tagctatgga atataatagc tactaataag ttaggagaat   300
   aaac                                                                304
<210> 5
   <211> 304
   <212> DNA
   <213> artificial sequence
<220>
   <223> mutant promoter
<400> 5
   gttcgatcaa caacccgaat cctatcgtaa tgatgttttg cccgatcagc ctcaatcgac    60
   aattttacgc gtttcgatcg aagcagggac gacaattggc tgggaacggt atactggaat   120
   aaatggtctt cgttatggta ttgatgtttt tggtgcatcg gccccggcga atgatctata   180
   tgctcatttc ggcttgaccg cagtcggcat cacgaataag gtgttggccg cgatcgccgg   240
   taagtcggca cgttaaaaaa tagctatgga atataatagc tactaataag ttaggagaat   300
   aaac                                                                304
<210> 6
   <211> 304
   <212> DNA
   <213> artificial sequence
<220>
   <223> double mutation promoter
<400> 6
   gttcgatcaa caacccgaat cctatcgtaa tgatgttttg cccgatcagc ctcaatcgac    60
   aattttacgc gtttcgatcg aagcagggac gacaattggc tgggaacggt atacttgaat   120
   aaatggtctt cgttatggta ttgatgtttt tggtgcatcg gccccggcga atgatctata   180
   tgctcatttc ggcttgaccg cagtcggcat cacgaataag gtgttggccg cgatcgccgg   240
   taagtcggca cgttaaaaaa tagctatgga atataatagc tactaataag ttaggagaat   300
   aaac                                                                304
<210> 7
   <211> 305
   <212> DNA
   <213> artificial sequence
<220>
   <223> promoter with mutation
<400> 7
   gttcgatcaa caacccgaat cctatcgtaa tgatgttttg cccgatcagc ctcaatcgac 60
   aattttacgc gtttcgatcg aagcagggac gacaattggc tgggaacggt atacttgaat 120
   aaatggtctt cgttatggta ttgatgtttt tggtgcatcg gccccggcga atgatctata 180
   tgctcatttc ggcttgaccg cagtcggcat cacgaacaag gtgttggccg cgatcgccgg 240
   taagtcggca cgttaaaaaa tagctatgga atataatagc tacttaataa gttaggagaa 300
   taaac 305
<210> 8
   <211> 305
   <212> DNA
   <213> artificial sequence
<220>
   <223> promoter with mutation
<400> 8
   gttcgatcaa caacccgaat cctatcgtaa tgatgttttg cccgatcagc ctcaatcgac 60
   aattttacgc gtttcgatcg aagcagggac gacaattggc tgggaacggt atactggaat 120
   aaatggtctt cgttatggta ttgatgtttt tggtgcatcg gccccggcga atgatctata 180
   tgctcatttc ggcttgaccg cagtcggcat cacgaataag gtgttggccg cgatcgccgg 240
   taagtcggca cgttaaaaaa tagctatgga atataatagc tacttaataa gttaggagaa 300
   taaac 305
<210> 9
   <211> 305
   <212> DNA
   <213> artificial sequence
<220>
   <223> promoter with mutations
<400> 9
   gttcgatcaa caacccgaat cctatcgtaa tgatgttttg cccgatcagc ctcaatcgac 60
   aattttacgc gtttcgatcg aagcagggac gacaattggc tgggaacggt atacttgaat 120
   aaatggtctt cgttatggta ttgatgtttt tggtgcatcg gccccggcga atgatctata 180
   tgctcatttc ggcttgaccg cagtcggcat cacgaataag gtgttggccg cgatcgccgg 240
   taagtcggca cgttaaaaaa tagctatgga atataatagc tacttaataa gttaggagaa 300
   taaac 305
<210> 10
   <211> 305
   <212> DNA
   <213> artificial sequence
<220>
   <223> promoter with mutation
<400> 10
   gttcgatcaa caacccgaat cctatcgtaa tgatgttttg cccgatcagc ctcaatcgac 60
   aattttacgc gtttcgatcg aagcagggac gacaattggc tgggaacggt atacttgaat 120
   aaatggtctt cgttatggta ttgatgtttt tggtgcatcg gccccggcga atgatctata 180
   tgctcatttc ggcttgaccg cagtcggcat cacgaacaag gtgttggccg cgatcgccgg 240
   taagtcggca cgttaaaaaa tagctatgga atatagtagc tacttaataa gttaggagaa 300
   taaac 305
<210> 11
   <211> 305
   <212> DNA
   <213> artificial sequence
<220>
   <223> promoter with mutation
<400> 11
   gttcgatcaa caacccgaat cctatcgtaa tgatgttttg cccgatcagc ctcaatcgac 60
   aattttacgc gtttcgatcg aagcagggac gacaattggc tgggaacggt atactggaat 120
   aaatggtctt cgttatggta ttgatgtttt tggtgcatcg gccccggcga atgatctata 180
   tgctcatttc ggcttgaccg cagtcggcat cacgaataag gtgttggccg cgatcgccgg 240
   taagtcggca cgttaaaaaa tagctatgga atatagtagc tacttaataa gttaggagaa 300
   taaac 305
<210> 12
   <211> 305
   <212> DNA
   <213> artificial sequence
<220>
   <223> promoter with mutations
<400> 12
   gttcgatcaa caacccgaat cctatcgtaa tgatgttttg cccgatcagc ctcaatcgac       60
   aattttacgc gtttcgatcg aagcagggac gacaattggc tgggaacggt atacttgaat      120
   aaatggtctt cgttatggta ttgatgtttt tggtgcatcg gccccggcga atgatctata      180
   tgctcatttc ggcttgaccg cagtcggcat cacgaataag gtgttggccg cgatcgccgg      240
   taagtcggca cgttaaaaaa tagctatgga atatagtagc tacttaataa gttaggagaa      300
   taaac                                                                  305
<210> 13
   <211> 1182
   <212> DNA
   <213> artificial sequence
<220>
   <223> codon optimized coding region of Actinoplanes missouriensis xylose isomerase for expression in Zymomonas
<400> 13
   atgagtgtcc aagctacacg cgaagataaa tttagctttg gcttgtggac agttgggtgg 60
   caggcccgtg acgcctttgg tgatgccacg cgtaccgccc ttgatccggt cgaagcagtt 120
   cataaacttg ccgaaatcgg agcctatggc attacatttc atgatgatga cttggtcccc 180
   ttcggttcgg atgctcagac acgggatggt atcattgctg gtttcaaaaa agcccttgat 240
   gaaaccggtt tgatcgttcc tatggttacc acaaatttgt ttacgcatcc ggtttttaaa 300
   gacggcgggt ttacctctaa cgatcgttct gtgcgccggt atgctatccg gaaagttttg 360
   agacagatgg atcttggtgc ggaactgggg gccaaaacgc ttgtcttatg gggcggcaga 420
   gaaggtgcgg aatatgattc agcaaaagat gttagtgccg ccttggatcg ttatcgcgaa 480
   gcactgaatc ttctggcaca atatagcgaa gacagaggct atggacttcg ttttgcaatt 540
   gaaccgaaac ctaatgaacc acgtggcgat attctgctgc ctaccgcagg ccatgctatt 600
   gcctttgtgc aagaattaga acggcccgaa ctttttggca tcaatccaga aaccgggcat 660
   gaacagatgt caaacttgaa ttttacccag gggattgctc aggctttgtg gcataaaaaa 720
   ctttttcata ttgatttgaa tggacaacat ggtcccaagt ttgatcagga ccttgtcttt 780
   ggtcatggtg accttttaaa tgcctttagc ctggtcgatt tgttagaaaa tggtccagat 840
   ggtgccccgg cttatgatgg cccgcgccat tttgattata aaccatctcg tactgaagat 900
   tatgatggcg tttgggaatc agcgaaagcc aatatccgta tgtatctttt attaaaagaa 960
   cgtgccaaag cgtttagagc tgatccggaa gttcaggaag cactggcagc aagcaaagtt 1020
   gccgaattga aaaccccaac gttgaatcct ggtgaaggct atgcagaact gttagcagat 1080
   cgcagtgctt ttgaagatta tgatgccgat gctgttggtg cgaaaggttt tggtttcgtg 1140
   aaattgaacc aattggccat tgaacattta ttaggtgccc gc 1182
<210> 14
   <211> 945
   <212> DNA
   <213> Zymomonas mobilis
<400> 14
   atgaacactt ctacgcaaaa acccgctcat ttcgacaaga tttcgatcaa agggattgat 60
   aaatccgcaa cccgtgtagc gttaggcaca tgggctattg gtggctggat gtggggcggc 120
   actgatgacg atgcctccat taaaaccatt catcgggcga ttgatcttgg tatcaatatc 180
   atcgacaccg ctccggctta tggccgtggc catgctgaag aagtcgttgg taaagccatc 240
   aaaggtcaac gcgataattt gattattgcg accaaagtcg gccttgattg gactttaacc 300
   cccgaccaat cgatgcgccg taacagttca gccagccgta tcaaaaaaga aatcgaagat 360
   tctctgcgcc gccttggcac tgattatatc gacctttatc aggtgcattg gccggatccg     420
   ctggttccga ttgaagaaac cgcaacaata ttggaagccc tcagaaaaga aggcaaaatc     480
   cgttctatcg gcgtttccaa ttattccgtt cagcagatgg acgagttcaa gaaatatgcc     540
   gagctgctgg tcgttttagg ctatggtgcg ctttgccgtg gtttactttc tggcagaatg     600
   acggcggatc gtgcctttac aggcgatgat ttacggaaaa cagacccgaa attccagaaa     660
   ccgcgctttg aacattatct ggccgcggtt gaagaactga agaaactcgc caaagagcat     720
   tacaataaat cggtgttggc tttggctatc cgctggatgt tggagcaagg gcccacttta     780
   gcactttggg gcgctcgcaa gccggaacag atcgacggta ttgatgaagt ttttggctgg     840
   cagatatcgg atgaagatct gaaacagatt gatgctattc tggccaagaa tatccccaat     900
   cctatcggtg cagaatttat ggcacccccg ccacgcgata aataa                     945
<210> 15
   <211> 10250
   <212> DNA
   <213> artificial sequence
<220>
   <223> constructed plasmid
<400> 15
   ctagtgttcg atcaacaacc cgaatcctat cgtaatgatg ttttgcccga tcagcctcaa      60
   tcgacaattt tacgcgtttc gatcgaagca gggacgacaa ttggctggga acggtatact     120
   tgaataaatg gtcttcgtta tggtattgat gtttttggtg catcggcccc ggcgaatgat     180
   ctatatgctc atttcggctt gaccgcagtc ggcatcacga acaaggtgtt ggccgcgatc     240
   gccggtaagt cggcacgtta aaaaatagct atggaatata atagctacta ataagttagg     300
   agaataaaca tgagtgtcca agctacacgc gaagataaat ttagctttgg cttgtggaca     360
   gttgggtggc aggcccgtga cgcctttggt gatgccacgc gtaccgccct tgatccggtc     420
   gaagcagttc ataaacttgc cgaaatcgga gcctatggca ttacatttca tgatgatgac     480
   ttggtcccct tcggttcgga tgctcagaca cgggatggta tcattgctgg tttcaaaaaa     540
   gcccttgatg aaaccggttt gatcgttcct atggttacca caaatttgtt tacgcatccg     600
   gtttttaaag acggcgggtt tacctctaac gatcgttctg tgcgccggta tgctatccgg     660
   aaagttttga gacagatgga tcttggtgcg gaactggggg ccaaaacgct tgtcttatgg     720
   ggcggcagag aaggtgcgga atatgattca gcaaaagatg ttagtgccgc cttggatcgt     780
   tatcgcgaag cactgaatct tctggcacaa tatagcgaag acagaggcta tggacttcgt     840
   tttgcaattg aaccgaaacc taatgaacca cgtggcgata ttctgctgcc taccgcaggc     900
   catgctattg cctttgtgca agaattagaa cggcccgaac tttttggcat caatccagaa     960
   accgggcatg aacagatgtc aaacttgaat tttacccagg ggattgctca ggctttgtgg    1020
   cataaaaaac tttttcatat tgatttgaat ggacaacatg gtcccaagtt tgatcaggac 1080
   cttgtctttg gtcatggtga ccttttaaat gcctttagcc tggtcgattt gttagaaaat 1140
   ggtccagatg gtgccccggc ttatgatggc ccgcgccatt ttgattataa accatctcgt 1200
   actgaagatt atgatggcgt ttgggaatca gcgaaagcca atatccgtat gtatctttta 1260
   ttaaaagaac gtgccaaagc gtttagagct gatccggaag ttcaggaagc actggcagca 1320
   agcaaagttg ccgaattgaa aaccccaacg ttgaatcctg gtgaaggcta tgcagaactg 1380
   ttagcagatc gcagtgcttt tgaagattat gatgccgatg ctgttggtgc gaaaggtttt 1440
   ggtttcgtga aattgaacca attggccatt gaacatttat taggtgcccg ctagtctaga 1500
   tgactgtata aaaccacagc caatcaaacg aaaccaggct atactcaagc ctggtttttt 1560
   gatggatttt cagcgtggcg caggcaggtt ttatcttaac ccgacactgg cgggacaccc 1620
   cgcaagggac agaagtctcc ttctggctgg cgacggacaa cgggccaagc ttccagttac 1680
   tcaatacgta acaataatca gtttatccta actatagaat cgcatgagaa gcgataacgt 1740
   ttcaccataa gcaatatatt cattgcaaca gtggaattgc cttatgcgtc aaggaaggat 1800
   agatcattga cggactgagt tcaaaaagag actggtctaa aagattttaa gaaaggtttc 1860
   gatatgtata tcgggataga tcttggcacc tcgggcgtaa aagttatttt gctcaacgag 1920
   cagggtgagg tggttgctgc gcaaacggaa aagctgaccg tttcgcgccc gcatccactc 1980
   tggtcggaac aagacccgga acagtggtgg caggcaactg atcgcgcaat gaaagctctg 2040
   ggcgatcagc attctctgca ggacgttaaa gcattgggta ttgccggcca gatgcacgga 2100
   gcaaccttgc tggatgctca gcaacgggtg ttacgccctg ccattttgtg gaacgacggg 2160
   cgctgtgcgc aagagtgcac tttgctggaa gcgcgagttc cgcaatcgcg ggtgattacc 2220
   ggcaacctga tgatgcccgg atttactgcg cctaaattgc tatgggttca gcggcatgag 2280
   ccggagatat tccgtcaaat cgacaaagta ttattaccga aagattactt gcgtctgcgt 2340
   atgacggggg agtttgccag cgatatgtct gacgcagctg gcaccatgtg gctggatgtc 2400
   gcaaagcgtg actggagtga cgtcatgctg caggcttgcg acttatctcg tgaccagatg 2460
   cccgcattat acgaaggcag cgaaattact ggtgctttgt tacctgaagt tgcgaaagcg 2520
   tggggtatgg cgacggtgcc agttgtcgca ggcggtggcg acaatgcagc tggtgcagtt 2580
   ggtgtgggaa tggttgatgc taatcaggca atgttatcgc tggggacgtc gggggtctat 2640
   tttgctgtca gcgaagggtt cttaagcaag ccagaaagcg ccgtacatag cttttgccat 2700
   gcgctaccgc aacgttggca tttaatgtct gtgatgctga gtgcagcgtc gtgtctggat 2760
   tgggccgcga aattaaccgg cctgagcaat gtcccagctt taatcgctgc agctcaacag 2820
   gctgatgaaa gtgccgagcc agtttggttt ctgccttatc tttccggcga gcgtacgcca 2880
   cacaataatc cccaggcgaa gggggttttc tttggtttga ctcatcaaca tggccccaat 2940
   gaactggcgc gagcagtgct ggaaggcgtg ggttatgcgc tggcagatgg catggatgtc 3000
   gtgcatgcct gcggtattaa accgcaaagt gttacgttga ttgggggcgg ggcgcgtagt 3060
   gagtactggc gtcagatgct ggcggatatc agcggtcagc agctcgatta ccgtacgggg 3120
   ggggatgtgg ggccagcact gggcgcagca aggctggcgc agatcgcggc gaatccagag 3180
   aaatcgctca ttgaattgtt gccgcaacta ccgttagaac agtcgcatct accagatgcg 3240
   cagcgttatg ccgcttatca gccacgacga gaaacgttcc gtcgcctcta tcagcaactt 3300
   ctgccattaa tggcgtaaac gttatcccct gcctgaccgg gtgggggata attcacatct 3360
   atatatctca gtaattaatt aatatttagt acgaatttat tctgaaaatc atttgttaat 3420
   ggcatttttc agttttgtct ttcgttggtt actcgtaatg tatcgctggt agatatggag 3480
   atcgttatga aaacctcaaa gactgtggca aaactattat ttgttgtcgg ggcgctggtt 3540
   tatctggttg ggctatggat ctcatgccca ttgttaagtg gaaaaggcta ttttcttggc 3600
   gtgttaatga cagcaacttt tggcaactat gcgaattcgc gatcgcataa cttcgtataa 3660
   tgtatgctat acgaagttat gcggccgcag cacaggatga cgcctaacaa ttcattcaag 3720
   ccgacaccgc ttcgcggcgc ggcttaattc aggagttaaa catcatgagg gaagcggtga 3780
   tcgccgaagt atcgactcaa ctatcagagg tagttggcgt catcgagcgc catctcgaac 3840
   cgacgttgct ggccgtacat ttgtacggct ccgcagtgga tggcggcctg aagccacaca 3900
   gtgatattga tttgctggtt acggtgactg taaggcttga tgaaacaacg cggcgagctt 3960
   tgatcaacga ccttttggaa acttcggctt cccctggaga gagcgagatt ctccgcgctg 4020
   tagaagtcac cattgttgtg cacgacgaca tcattccgtg gcgttatcca gctaagcgcg 4080
   aactgcaatt tggagaatgg cagcgcaatg acattcttgc aggtatcttc gagccagcca 4140
   cgatcgacat tgatctggct atcttgctga caaaagcaag agaacatagc gttgccttgg 4200
   taggtccagc ggcggaggaa ctctttgatc cggttcctga acaggatcta tttgaggcgc 4260
   taaatgaaac cttaacgcta tggaactcgc cgcccgactg ggctggcgat gagcgaaatg 4320
   tagtgcttac gttgtcccgc atttggtaca gcgcagtaac cggcaaaatc gcgccgaagg 4380
   atgtcgctgc cgactgggca atggagcgcc tgccggccca gtatcagccc gtcatacttg 4440
   aagctaggca ggcttatctt ggacaagaag atcgcttggc ctcgcgcgca gatcagttgg 4500
   aagaatttgt tcactacgtg aaaggcgaga tcaccaaggt agtcggcaaa taatgtctaa 4560
   caattcgttc aagccgacgc cgcttcgcgg cgcggcttaa ctcaagcgtt agagagctgg 4620
   ggaagactat gcgcgatctg ttgaaggtgg ttctaagcct cgtacttgcg atggcatcgg 4680
   ggcaggcact tgctgacctg ccttaattaa ataacttcgt ataatgtatg ctatacgaag 4740
   ttatggccgg cccgttactt gctgggtgaa gaaccgatcg aagtccgtgc ttacacctac 4800
   agcgatccga atgatgaacg tttcgttgaa gtcgaagatc gtattatttg gcagatgcgc 4860
   ttcagaagcg gtgctctgtc tcatggtgca tcttcttatt cgaccacgac gacttcacgt 4920
   ttctcggtgc agggcgacaa agctgttctg ttgatggatc cggctaccgg atattatcag 4980
   aatttgattt ctgtccagac cccaggccat gctaaccagt cgatgatgcc acagttcatc 5040
   atgccagcga acaaccagtt ctctgcacag ttggatcatc tggctgaagc cgtcatcaat 5100
   aacaaaccag ttcgtagccc gggtgaagaa ggtatgcagg atgtgcgcct gattcaggcc 5160
   atttatgaag cagctcgtac cggtcgcccc gtcaacacgg attggggtta tgtccgtcag 5220
   ggtggttatt gattctgact taacctattt gggttaaaca gacttatttt tcctgtttta 5280
   ggaaaatagt taaaaaggcg tcattggttc ttccaatgac gccttttttt ataaacaaaa 5340
   aaatcctttt gtcggtttta taaaaatact tcatattttg ataagccgtc ttaaaaatat 5400
   aataaatttt tataatattt atccgatcaa aggacccctt tatgctagaa gtcattatat 5460
   cggcattact accgattata attactttaa tgataggttt tttcgctggc tggcgtggtg 5520
   aatttacggc aaatcaagcc tcgaccttga ataaaatggt cttacgctat gccttaccta 5580
   tgactttatt ctctgggatt ttatcacttc ccaaaacaca gattttatcg tcgggttctg 5640
   ccgcaattat tttactttta gccatggctg gcggctatct aattacactt gggataggat 5700
   attttgtctg ccagcgccca gtgaatgaat ctgctctttt agctctttct gttagcgcac 5760
   ctgcagttcc ttttgttggc ataacagttc tagggcattt atttggcact gccagcacga 5820
   tattggtttc aatatgtagc ctgatgatga acctcgtcca ggttcccgtt acctttttct 5880
   tttgtcagcg tattctccaa aaaagaatac tgacaaaata gccacggata gttctttttt 5940
   ttctcatatc agacatgctt ttaccgaacc tattgttatt gcccctattc tggctcttat 6000
   ctgtgtcagt ctctctattc ctttccctga aaccttaaaa tcttctttaa tgctactagg 6060
   aaaagcgacc ggaggcgttg cgcttttctc ttctggtata attttatttt ctcgaaaagt 6120
   tattttaagt aaaacagtag catctttagt tttatcaaaa aatattatta ttccaacagc 6180
   ggtattggtt cttgcgtcgg tacccaattc gccctatagt gagtcgtatt acgcgcgctc 6240
   actggccgtc gttttacaac gtcgtgactg ggaaaaccct ggcgttaccc aacttaatcg 6300
   ccttgcagca catccccctt tcgccagctg gcgtaatagc gaagaggccc gcaccgatcg 6360
   cccttcccaa cagttgcgca gcctgaatgg cgaatgggac gcgccctgta gcggcgcatt 6420
   aagcgcggcg ggtgtggtgg ttacgcgcag cgtgaccgct acacttgcca gcgccctagc 6480
   gcccgctcct ttcgctttct tcccttcctt tctcgccacg ttcgccggct ttccccgtca 6540
   agctctaaat cgggggctcc ctttagggtt ccgatttagt gctttacggc acctcgaccc 6600
   caaaaaactt gattagggtg atggttcacg tagtgggcca tcgccctgat agacggtttt 6660
   tcgccctttg acgttggagt ccacgttctt taatagtgga ctcttgttcc aaactggaac 6720
   aacactcaac cctatctcgg tctattcttt tgatttataa gggattttgc cgatttcggc 6780
   ctattggtta aaaaatgagc tgatttaaca aaaatttaac gcgaatttta acaaaatatt 6840
   aacgcttaca atttaggtgg cacttttcgg ggaaatgtgc gcggaacccc tatttgttta 6900
   tttttctaaa tacattcaaa tatgtatccg ctcatgagac aataaccctg ataaatgctt 6960
   caataatatt gaaaaaggaa gagtatgagt attcaacatt tccgtgtcgc ccttattccc 7020
   ttttttgcgg cattttgcct tcctgttttt gctcacccag aaacgctggt gaaagtaaaa 7080
   gatgctgaag atcagttggg tgcacgagtg ggttacatcg aactggatct caacagcggt 7140
   aagatccttg agagttttcg ccccgaagaa cgttttccaa tgatgagcac ttttaaagtt 7200
   ctgctatgtg gcgcggtatt atcccgtatt gacgccgggc aagagcaact cggtcgccgc 7260
   atacactatt ctcagaatga cttggttgag tactcaccag tcacagaaaa gcatcttacg 7320
   gatggcatga cagtaagaga attatgcagt gctgccataa ccatgagtga taacactgcg 7380
   gccaacttac ttctgacaac gatcggagga ccgaaggagc taaccgcttt tttgcacaac 7440
   atgggggatc atgtaactcg ccttgatcgt tgggaaccgg agctgaatga agccatacca 7500
   aacgacgagc gtgacaccac gatgcctgta gcaatggcaa caacgttgcg caaactatta 7560
   actggcgaac tacttactct agcttcccgg caacaattaa tagactggat ggaggcggat 7620
   aaagttgcag gaccacttct gcgctcggcc cttccggctg gctggtttat tgctgataaa 7680
   tctggagccg gtgagcgtgg gtctcgcggt atcattgcag cactggggcc agatggtaag 7740
   ccctcccgta tcgtagttat ctacacgacg gggagtcagg caactatgga tgaacgaaat 7800
   agacagatcg ctgagatagg tgcctcactg attaagcatt ggtaactgtc agaccaagtt 7860
   tactcatata tactttagat tgatttaaaa cttcattttt aatttaaaag gatctaggtg 7920
   aagatccttt ttgataatct catgaccaaa atcccttaac gtgagttttc gttccactga 7980
   gcgtcagacc ccgtagaaaa gatcaaagga tcttcttgag atcctttttt tctgcgcgta 8040
   atctgctgct tgcaaacaaa aaaaccaccg ctaccagcgg tggtttgttt gccggatcaa 8100
   gagctaccaa ctctttttcc gaaggtaact ggcttcagca gagcgcagat accaaatact 8160
   gtccttctag tgtagccgta gttaggccac cacttcaaga actctgtagc accgcctaca 8220
   tacctcgctc tgctaatcct gttaccagtg gctgctgcca gtggcgataa gtcgtgtctt 8280
   accgggttgg actcaagacg atagttaccg gataaggcgc agcggtcggg ctgaacgggg 8340
   ggttcgtgca cacagcccag cttggagcga acgacctaca ccgaactgag atacctacag 8400
   cgtgagctat gagaaagcgc cacgcttccc gaagggagaa aggcggacag gtatccggta 8460
   agcggcaggg tcggaacagg agagcgcacg agggagcttc cagggggaaa cgcctggtat 8520
   ctttatagtc ctgtcgggtt tcgccacctc tgacttgagc gtcgattttt gtgatgctcg 8580
   tcaggggggc ggagcctatg gaaaaacgcc agcaacgcgg cctttttacg gttcctggcc    8640
   ttttgctggc cttttgctca catgttcttt cctgcgttat cccctgattc tgtggataac    8700
   cgtattaccg cctttgagtg agctgatacc gctcgccgca gccgaacgac cgagcgcagc    8760
   gagtcagtga gcgaggaagc ggaagagcgc ccaatacgca aaccgcctct ccccgcgcgt    8820
   tggccgattc attaatgcag ctggcacgac aggtttcccg actggaaagc gggcagtgag    8880
   cgcaacgcaa ttaatgtgag ttagctcact cattaggcac cccaggcttt acactttatg    8940
   cttccggctc gtatgttgtg tggaattgtg agcggataac aatttcacac aggaaacagc    9000
   tatgaccatg attacgccaa gcgcgcaatt aaccctcact aaagggaaca aaagctggag    9060
   ctcgtccaga aaagacagca ttccttctca ataaagaaat attatttttt gtttttgaaa    9120
   aatttttcca aaatctagaa tgctacatta aatatacaaa aatattatta tacaaataag    9180
   gcttttaaat acccatattt tttagaattt ctttacaaag aaacatgtta aatatagatt    9240
   tagagattaa tatcagccat ttttatcaaa aattcttttt ttgttttata atattatgct    9300
   gcaaaactaa taaaaacgcc ctttcgaaat taacgatcac ccacaagaaa taattatctg    9360
   acagcgctta ccaatcaatt attgccgaac gcagagtccc gtattaggac ggtcaacaat    9420
   ctaaaccgtt tttcagaaaa tattgcttta taagcctcaa aacttaaaag ctgcggtatt    9480
   ttaatatacc aaaattttct ggaaaagccg gcgaatcaga taacagttcc gcacaggtga    9540
   gaaccacgac ggatcttctc tgaattgttg gttagttaag aaagaaacaa ggattatgac    9600
   gaacaaaatc tcgtcttcag ataatctttc caatgctgtt tcagcaacgg atgacaacgc    9660
   ttcccgtacg ccaaatctga cccgtcgcgc tctcgttggt ggtggtgttg gactggccgc    9720
   agctggcgcc ttagccagtg gtcttcaggc agcgacgctt cctgctggtg ccagccaggt    9780
   tccgaccacg cctgcaggtc gcccgatgcc ttacgcgatc cgcccgatgc cggaagatcg    9840
   tcgtttcggt tatgctatcg tcggtctggg taaatatgcc cttaaccaga ttttaccggg    9900
   ttttgccgga tgccagcatt cccgcatcga agctttggtc agcggtaacg ctgaaaaagc    9960
   taaaatcgtt gccgctgaat atggcgtcga tccccgtaaa atttatgatt acagcaactt    10020
   cgacaagatc gctaaagatc caaaaatcga cgctgtttac atcattttgc caaactcttt    10080
   gcatgctgaa tttgctatcc gtgctttcaa agccggcaag catgttatgt gtgaaaagcc    10140
   gatggcaacc tctgttgctg attgtcagcg gatgatcgat gcagccaagg ctgctaataa    10200
   aaagctgatg atcggttacc gttgccacta tgatccaatg aaccgtgcaa              10250
<210> 16
   <211> 1186
   <212> DNA
   <213> artificial sequence
<220>
   <223> sonstructed fragment
<400> 16
   gtccagaaaa gacagcattc cttctcaata aagaaatatt attttttgtt tttgaaaaat      60
   ttttccaaaa tctagaatgc tacattaaat atacaaaaat attattatac aaataaggct     120
   tttaaatacc catatttttt agaatttctt tacaaagaaa catgttaaat atagatttag     180
   agattaatat cagccatttt tatcaaaaat tctttttttg ttttataata ttatgctgca     240
   aaactaataa aaacgccctt tcgaaattaa cgatcaccca caagaaataa ttatctgaca     300
   gcgcttacca atcaattatt gccgaacgca gagtcccgta ttaggacggt caacaatcta     360
   aaccgttttt cagaaaatat tgctttataa gcctcaaaac ttaaaagctg cggtatttta     420
   atataccaaa attttctgga aaagccggcg aatcagataa cagttccgca caggtgagaa     480
   ccacgacgga tcttctctga attgttggtt agttaagaaa gaaacaagga ttatgacgaa     540
   caaaatctcg tcttcagata atctttccaa tgctgtttca gcaacggatg acaacgcttc     600
   ccgtacgcca aatctgaccc gtcgcgctct cgttggtggt ggtgttggac tggccgcagc     660
   tggcgcctta gccagtggtc ttcaggcagc gacgcttcct gctggtgcca gccaggttcc     720
   gaccacgcct gcaggtcgcc cgatgcctta cgcgatccgc ccgatgccgg aagatcgtcg     780
   tttcggttat gctatcgtcg gtctgggtaa atatgccctt aaccagattt taccgggttt     840
   tgccggatgc cagcattccc gcatcgaagc tttggtcagc ggtaacgctg aaaaagctaa     900
   aatcgttgcc gctgaatatg gcgtcgatcc ccgtaaaatt tatgattaca gcaacttcga     960
   caagatcgct aaagatccaa aaatcgacgc tgtttacatc attttgccaa actctttgca    1020
   tgctgaattt gctatccgtg ctttcaaagc cggcaagcat gttatgtgtg aaaagccgat    1080
   ggcaacctct gttgctgatt gtcagcggat gatcgatgca gccaaggctg ctaataaaaa    1140
   gctgatgatc ggttaccgtt gccactatga tccaatgaac cgtgca                    1186
<210> 17
   <211> 1302
   <212> DNA
   <213> Zymomonas mobilis
<400> 17
   atgacgaaca aaatctcgtc ttcagataat ctttccaatg ctgtttcagc aacggatgac 60
   aacgcttccc gtacgccaaa tctgacccgt cgcgctctcg ttggtggtgg tgttggactg 120
   gccgcagctg gcgccttagc cagtggtctt caggcagcga cgcttcctgc tggtgccagc 180
   caggttccga ccacgcctgc aggtcgcccg atgccttacg cgatccgccc gatgccggaa 240
   gatcgtcgtt tcggttatgc tatcgtcggt ctgggtaaat atgcccttaa ccagatttta 300
   ccgggttttg ccggatgcca gcattcccgc atcgaagctt tggtcagcgg taacgctgaa 360
   aaagctaaaa tcgttgccgc tgaatatggc gtcgatcccc gtaaaattta tgattacagc 420
   aacttcgaca agatcgctaa agatccaaaa atcgacgctg tttacatcat tttgccaaac 480
   tctttgcatg ctgaatttgc tatccgtgct ttcaaagccg gcaagcatgt tatgtgtgaa 540
   aagccgatgg caacctctgt tgctgattgt cagcggatga tcgatgcagc caaggctgct 600
   aataaaaagc tgatgatcgg ttaccgttgc cactatgatc caatgaaccg tgcagcggta 660
   aaattgatcc gtgaaaacca gttgggtaaa ctgggcatgg ttaccaccga caactcagac 720
   gttatggatc agaacgatcc tgcacagcag tggcgtctgc gtcgtgaact cgccggtggc 780
   ggttctttga tggatatcgg tatttatggc ttgaacggta cccgttactt gctgggtgaa 840
   gaaccgatcg aagtccgtgc ttacacctac agcgatccga atgatgaacg tttcgttgaa 900
   gtcgaagatc gtattatttg gcagatgcgc ttcagaagcg gtgctctgtc tcatggtgca 960
   tcttcttatt cgaccacgac gacttcacgt ttctcggtgc agggcgacaa agctgttctg 1020
   ttgatggatc cggctaccgg atattatcag aatttgattt ctgtccagac cccaggccat 1080
   gctaaccagt cgatgatgcc acagttcatc atgccagcga acaaccagtt ctctgcacag 1140
   ttggatcatc tggctgaagc cgtcatcaat aacaaaccag ttcgtagccc gggtgaagaa 1200
   ggtatgcagg atgtgcgcct gattcaggcc atttatgaag cagctcgtac cggtcgcccc 1260
   gtcaacacgg attggggtta tgtccgtcag ggtggttatt ga 1302
<210> 18
   <211> 1446
   <212> DNA
   <213> artificial sequence
<220>
   <223> constructed fragment
<400> 18
   cgttacttgc tgggtgaaga accgatcgaa gtccgtgctt acacctacag cgatccgaat 60
   gatgaacgtt tcgttgaagt cgaagatcgt attatttggc agatgcgctt cagaagcggt 120
   gctctgtctc atggtgcatc ttcttattcg accacgacga cttcacgttt ctcggtgcag 180
   ggcgacaaag ctgttctgtt gatggatccg gctaccggat attatcagaa tttgatttct 240
   gtccagaccc caggccatgc taaccagtcg atgatgccac agttcatcat gccagcgaac 300
   aaccagttct ctgcacagtt ggatcatctg gctgaagccg tcatcaataa caaaccagtt 360
   cgtagcccgg gtgaagaagg tatgcaggat gtgcgcctga ttcaggccat ttatgaagca 420
   gctcgtaccg gtcgccccgt caacacggat tggggttatg tccgtcaggg tggttattga 480
   ttctgactta acctatttgg gttaaacaga cttatttttc ctgttttagg aaaatagtta 540
   aaaaggcgtc attggttctt ccaatgacgc ctttttttat aaacaaaaaa atccttttgt 600
   cggttttata aaaatacttc atattttgat aagccgtctt aaaaatataa taaattttta 660
   taatatttat ccgatcaaag gaccccttta tgctagaagt cattatatcg gcattactac 720
   cgattataat tactttaatg ataggttttt tcgctggctg gcgtggtgaa tttacggcaa 780
   atcaagcctc gaccttgaat aaaatggtct tacgctatgc cttacctatg actttattct 840
   ctgggatttt atcacttccc aaaacacaga ttttatcgtc gggttctgcc gcaattattt 900
   tacttttagc catggctggc ggctatctaa ttacacttgg gataggatat tttgtctgcc 960
   agcgcccagt gaatgaatct gctcttttag ctctttctgt tagcgcacct gcagttcctt 1020
   ttgttggcat aacagttcta gggcatttat ttggcactgc cagcacgata ttggtttcaa 1080
   tatgtagcct gatgatgaac ctcgtccagg ttcccgttac ctttttcttt tgtcagcgta 1140
   ttctccaaaa aagaatactg acaaaatagc cacggatagt tctttttttt ctcatatcag 1200
   acatgctttt accgaaccta ttgttattgc ccctattctg gctcttatct gtgtcagtct 1260
   ctctattcct ttccctgaaa ccttaaaatc ttctttaatg ctactaggaa aagcgaccgg 1320
   aggcgttgcg cttttctctt ctggtataat tttattttct cgaaaagtta ttttaagtaa 1380
   aacagtagca tctttagttt tatcaaaaaa tattattatt ccaacagcgg tattggttct 1440
   tgcgtc 1446
<210> 19
   <211> 1661
   <212> DNA
   <213> artificial sequence
<220>
   <223> constructed fragment
<400> 19
   gttcgatcaa caacccgaat cctatcgtaa tgatgttttg cccgatcagc ctcaatcgac      60
   aattttacgc gtttcgatcg aagcagggac gacaattggc tgggaacggt atacttgaat     120
   aaatggtctt cgttatggta ttgatgtttt tggtgcatcg gccccggcga atgatctata     180
   tgctcatttc ggcttgaccg cagtcggcat cacgaacaag gtgttggccg cgatcgccgg     240
   taagtcggca cgttaaaaaa tagctatgga atataatagc tactaataag ttaggagaat     300
   aaacatgagt gtccaagcta cacgcgaaga taaatttagc tttggcttgt ggacagttgg     360
   gtggcaggcc cgtgacgcct ttggtgatgc cacgcgtacc gcccttgatc cggtcgaagc     420
   agttcataaa cttgccgaaa tcggagccta tggcattaca tttcatgatg atgacttggt     480
   ccccttcggt tcggatgctc agacacggga tggtatcatt gctggtttca aaaaagccct     540
   tgatgaaacc ggtttgatcg ttcctatggt taccacaaat ttgtttacgc atccggtttt     600
   taaagacggc gggtttacct ctaacgatcg ttctgtgcgc cggtatgcta tccggaaagt     660
   tttgagacag atggatcttg gtgcggaact gggggccaaa acgcttgtct tatggggcgg     720
   cagagaaggt gcggaatatg attcagcaaa agatgttagt gccgccttgg atcgttatcg     780
   cgaagcactg aatcttctgg cacaatatag cgaagacaga ggctatggac ttcgttttgc     840
   aattgaaccg aaacctaatg aaccacgtgg cgatattctg ctgcctaccg caggccatgc     900
   tattgccttt gtgcaagaat tagaacggcc cgaacttttt ggcatcaatc cagaaaccgg     960
   gcatgaacag atgtcaaact tgaattttac ccaggggatt gctcaggctt tgtggcataa    1020
   aaaacttttt catattgatt tgaatggaca acatggtccc aagtttgatc aggaccttgt    1080
   ctttggtcat ggtgaccttt taaatgcctt tagcctggtc gatttgttag aaaatggtcc    1140
   agatggtgcc ccggcttatg atggcccgcg ccattttgat tataaaccat ctcgtactga    1200
   agattatgat ggcgtttggg aatcagcgaa agccaatatc cgtatgtatc ttttattaaa    1260
   agaacgtgcc aaagcgttta gagctgatcc ggaagttcag gaagcactgg cagcaagcaa    1320
   agttgccgaa ttgaaaaccc caacgttgaa tcctggtgaa ggctatgcag aactgttagc    1380
   agatcgcagt gcttttgaag attatgatgc cgatgctgtt ggtgcgaaag gttttggttt    1440
   cgtgaaattg aaccaattgg ccattgaaca tttattaggt gcccgctagt ctagatgact    1500
   gtataaaacc acagccaatc aaacgaaacc aggctatact caagcctggt tttttgatgg    1560
   attttcagcg tggcgcaggc aggttttatc ttaacccgac actggcggga caccccgcaa    1620
   gggacagaag tctccttctg gctggcgacg gacaacgggc c                        1661
<210> 20
   <211> 1960
   <212> DNA
   <213> artificial sequence
<220>
   <223> constructed fragment containing a 191 bp Peno, a 1,455-bp E. coli xylB coding sequence (ECxylB) and a 314-bp E.coli xylB 3'UTR
<400> 20
   ccagttactc aatacgtaac aataatcagt ttatcctaac tatagaatcg catgagaagc 60
   gataacgttt caccataagc aatatattca ttgcaacagt ggaattgcct tatgcgtcaa 120
   ggaaggatag atcattgacg gactgagttc aaaaagagac tggtctaaaa gattttaaga 180
   aaggtttcga tatgtatatc gggatagatc ttggcacctc gggcgtaaaa gttattttgc 240
   tcaacgagca gggtgaggtg gttgctgcgc aaacggaaaa gctgaccgtt tcgcgcccgc 300
   atccactctg gtcggaacaa gacccggaac agtggtggca ggcaactgat cgcgcaatga 360
   aagctctggg cgatcagcat tctctgcagg acgttaaagc attgggtatt gccggccaga 420
   tgcacggagc aaccttgctg gatgctcagc aacgggtgtt acgccctgcc attttgtgga 480
   acgacgggcg ctgtgcgcaa gagtgcactt tgctggaagc gcgagttccg caatcgcggg 540
   tgattaccgg caacctgatg atgcccggat ttactgcgcc taaattgcta tgggttcagc 600
   ggcatgagcc ggagatattc cgtcaaatcg acaaagtatt attaccgaaa gattacttgc 660
   gtctgcgtat gacgggggag tttgccagcg atatgtctga cgcagctggc accatgtggc 720
   tggatgtcgc aaagcgtgac tggagtgacg tcatgctgca ggcttgcgac ttatctcgtg 780
   accagatgcc cgcattatac gaaggcagcg aaattactgg tgctttgtta cctgaagttg 840
   cgaaagcgtg gggtatggcg acggtgccag ttgtcgcagg cggtggcgac aatgcagctg 900
   gtgcagttgg tgtgggaatg gttgatgcta atcaggcaat gttatcgctg gggacgtcgg 960
   gggtctattt tgctgtcagc gaagggttct taagcaagcc agaaagcgcc gtacatagct 1020
   tttgccatgc gctaccgcaa cgttggcatt taatgtctgt gatgctgagt gcagcgtcgt 1080
   gtctggattg ggccgcgaaa ttaaccggcc tgagcaatgt cccagcttta atcgctgcag 1140
   ctcaacaggc tgatgaaagt gccgagccag tttggtttct gccttatctt tccggcgagc 1200
   gtacgccaca caataatccc caggcgaagg gggttttctt tggtttgact catcaacatg 1260
   gccccaatga actggcgcga gcagtgctgg aaggcgtggg ttatgcgctg gcagatggca 1320
   tggatgtcgt gcatgcctgc ggtattaaac cgcaaagtgt tacgttgatt gggggcgggg 1380
   cgcgtagtga gtactggcgt cagatgctgg cggatatcag cggtcagcag ctcgattacc 1440
   gtacgggggg ggatgtgggg ccagcactgg gcgcagcaag gctggcgcag atcgcggcga 1500
   atccagagaa atcgctcatt gaattgttgc cgcaactacc gttagaacag tcgcatctac 1560
   cagatgcgca gcgttatgcc gcttatcagc cacgacgaga aacgttccgt cgcctctatc 1620
   agcaacttct gccattaatg gcgtaaacgt tatcccctgc ctgaccgggt gggggataat 1680
   tcacatctat atatctcagt aattaattaa tatttagtac gaatttattc tgaaaatcat 1740
   ttgttaatgg catttttcag ttttgtcttt cgttggttac tcgtaatgta tcgctggtag 1800
   atatggagat cgttatgaaa acctcaaaga ctgtggcaaa actattattt gttgtcgggg 1860
   cgctggttta tctggttggg ctatggatct catgcccatt gttaagtgga aaaggctatt 1920
   ttcttggcgt gttaatgaca gcaacttttg gcaactatgc 1960
<210> 21
   <211> 1014
   <212> DNA
   <213> artificial sequence
<220>
   <223> constructed fragment with spec resistance gene bounded by lox recombination sites
<400> 21
   agcacaggat gacgcctaac aattcattca agccgacacc gcttcgcggc gcggcttaat      60
   tcaggagtta aacatcatga gggaagcggt gatcgccgaa gtatcgactc aactatcaga     120
   ggtagttggc gtcatcgagc gccatctcga accgacgttg ctggccgtac atttgtacgg     180
   ctccgcagtg gatggcggcc tgaagccaca cagtgatatt gatttgctgg ttacggtgac     240
   tgtaaggctt gatgaaacaa cgcggcgagc tttgatcaac gaccttttgg aaacttcggc     300
   ttcccctgga gagagcgaga ttctccgcgc tgtagaagtc accattgttg tgcacgacga     360
   catcattccg tggcgttatc cagctaagcg cgaactgcaa tttggagaat ggcagcgcaa     420
   tgacattctt gcaggtatct tcgagccagc cacgatcgac attgatctgg ctatcttgct     480
   gacaaaagca agagaacata gcgttgcctt ggtaggtcca gcggcggagg aactctttga     540
   tccggttcct gaacaggatc tatttgaggc gctaaatgaa accttaacgc tatggaactc     600
   gccgcccgac tgggctggcg atgagcgaaa tgtagtgctt acgttgtccc gcatttggta     660
   cagcgcagta accggcaaaa tcgcgccgaa ggatgtcgct gccgactggg caatggagcg     720
   cctgccggcc cagtatcagc ccgtcatact tgaagctagg caggcttatc ttggacaaga     780
   agatcgcttg gcctcgcgcg cagatcagtt ggaagaattt gttcactacg tgaaaggcga     840
   gatcaccaag gtagtcggca aataatgtct aacaattcgt tcaagccgac gccgcttcgc     900
   ggcgcggctt aactcaagcg ttagagagct ggggaagact atgcgcgatc tgttgaaggt     960
   ggttctaagc ctcgtacttg cgatggcatc ggggcaggca cttgctgacc tgcc           1014
<210> 22
   <211> 12198
   <212> DNA
   <213> artificial sequence
<220>
   <223> constructed plasmid
<400> 22
   ctagtgttcg atcaacaacc cgaatcctat cgtaatgatg ttttgcccga tcagcctcaa      60
   tcgacaattt tacgcgtttc gatcgaaaca gggacgacaa ttggctggga acggtatact     120
   ggaataaatg gtcttcgtta tggtattgat gtttttggtg catcggcccc ggcgaatgat     180
   ctatatgctc atttcggctt gaccgcagtc ggcatcacga acaaggtgtt ggccgcgatc     240
   gccggtaagt cggcacgtta aaaaatagct atggaatata atagctacta ataagttagg     300
   agaataaaca tgacggacaa attgacctcc cttcgtcagt acaccaccgt agtggccgac     360
   actggggaca tcgcggcaat gaagctgtat caaccgcagg atgccacaac caacccttct     420
   ctcattctta acgcagcgca gattccggaa taccgtaagt tgattgatga tgctgtcgcc     480
   tgggcgaaac agcagagcaa cgatcgcgcg cagcagatcg tggacgcgac cgacaaactg     540
   gcagtaaata ttggtctgga aatcctgaaa ctggttccgg gccgtatctc aactgaagtt     600
   gatgcgcgcc tttcctatga caccgaagcg tcaattgcga aagcaaaacg cctgatcaaa     660
   ctctacaacg atgctggtat tagcaacgat cgtattctga tcaaactggc ttctacctgg     720
   cagggtatcc gtgctgcaga acagctggaa aaagaaggca tcaactgtaa cctgaccctg     780
   ctgttctcct tcgctcaggc tcgtgcttgt gcggaagcgg gcgtgttcct gatctcgccg 840
   tttgttggcc gtattcttga ctggtacaaa gcgaataccg ataagaaaga gtacgctccg 900
   gcagaagatc cgggcgtggt ttctgtatct gaaatctacc agtactacaa agagcacggt 960
   tatgaaaccg tggttatggg cgcaagcttc cgtaacatcg gcgaaattct ggaactggca 1020
   ggctgcgacc gtctgaccat cgcaccggca ctgctgaaag agctggcgga gagcgaaggg 1080
   gctatcgaac gtaaactgtc ttacaccggc gaagtgaaag cgcgtccggc gcgtatcact 1140
   gagtccgagt tcctgtggca gcacaaccag ggtccaatgg cagtagataa actggcggaa 1200
   ggtatccgta agtttgctat tgaccaggaa aaactggaaa aaatgatcgg cgatctgctg 1260
   taatctagac gatctggagt caaaatgtcc tcacgtaaag agcttgccaa tgctattcgt 1320
   gcgctgagca tggacgcagt acagaaagcc aaatccggtc acccgggtgc ccctatgggt 1380
   atggctgaca ttgccgaagt cctgtggcgt gatttcctga aacacaaccc gcagaatccg 1440
   tcctgggctg accgtgaccg cttcgtgctg tccaacggcc acggctccat gctgatctac 1500
   agcctgctgc acctcaccgg ttacgatctg ccgatggaag aactgaaaaa cttccgtcag 1560
   ctgcactcta aaactccggg tcacccggaa gtgggttaca ccgctggtgt ggaaaccacc 1620
   accggtccgc tgggtcaggg tattgccaac gcagtcggta tggcgattgc agaaaaaacg 1680
   ctggcggcgc agtttaaccg tccgggccac gacattgtcg accactacac ctacgccttc 1740
   atgggcgacg gctgcatgat ggaaggcatc tcccacgaag tttgctctct ggcgggtacg 1800
   ctgaagctgg gtaaactgat tgcattctac gatgacaacg gtatttctat cgatggtcac 1860
   gttgaaggct ggttcaccga cgacaccgca atgcgtttcg aagcttacgg ctggcacgtt 1920
   attcgcgaca tcgacggtca tgacgcggca tctatcaaac gcgcagtaga agaagcgcgc 1980
   gcagtgactg acaaaccttc cctgctgatg tgcaaaacca tcatcggttt cggttccccg 2040
   aacaaagccg gtacccacga ctcccacggt gcgccgctgg gcgacgctga aattgccctg 2100
   acccgcgaac aactgggctg gaaatatgcg ccgttcgaaa tcccgtctga aatctatgct 2160
   cagtgggatg cgaaagaagc aggccaggcg aaagaatccg catggaacga gaaattcgct 2220
   gcttacgcga aagcttatcc gcaggaagcc gctgaattta cccgccgtat gaaaggcgaa 2280
   atgccgtctg acttcgacgc taaagcgaaa gagttcatcg ctaaactgca ggctaatccg 2340
   gcgaaaatcg ccagccgtaa agcgtctcag aatgctatcg aagcgttcgg tccgctgttg 2400
   ccggaattcc tcggcggttc tgctgacctg gcgccgtcta acctgaccct gtggtctggt 2460
   tctaaagcaa tcaacgaaga tgctgcgggt aactacatcc actacggtgt tcgcgagttc 2520
   ggtatgaccg cgattgctaa cggtatctcc ctgcacggtg gcttcctgcc gtacacctcc 2580
   accttcctga tgttcgtgga atacgcacgt aacgccgtac gtatggctgc gctgatgaaa 2640
   cagcgtcagg tgatggttta cacccacgac tccatcggtc tgggcgaaga cggcccgact 2700
   caccagccgg ttgagcaggt cgcttctctg cgcgtaaccc cgaacatgtc tacatggcgt 2760
   ccgtgtgacc aggttgaatc cgcggtcgcg tggaaatacg gtgttgagcg tcaggacggc 2820
   ccgaccgcac tgatcctctc ccgtcagaac ctggcgcagc aggaacgaac tgaagagcaa 2880
   ctggcaaaca tcgcgcgcgg tggttatgtg ctgaaagact gcgccggtca gccggaactg 2940
   attttcatcg ctaccggttc agaagttgaa ctggctgttg ctgcctacga aaaactgact 3000
   gccgaaggcg tgaaagcgcg cgtggtgtcc atgccgtcta ccgacgcatt tgacaagcag 3060
   gatgctgctt accgtgaatc cgtactgccg aaagcggtta ctgcacgcgt tgctgtagaa 3120
   gcgggtattg ctgactactg gtacaagtat gttggcctga acggtgctat cgtcggtatg 3180
   accaccttcg gtgaatctgc tccggcagag ctgctgtttg aagagttcgg cttcactgtt 3240
   gataacgttg ttgcgaaagc aaaagaactg ctgtaattag catttcgggt aaaaaaggtc 3300
   gcttcggcga ccttttttat taccttgata atgtccgttt gcgcggcgcg ccccagttac 3360
   tcaatacgta acaataatca gtttatccta actatagaat cgcatgagaa gcgataacgt 3420
   ttcaccataa gcaatatatt cattgcaaca gtggaattgc cttatgcgtc aaggaaggat 3480
   agatcattga cggactgagt tcaaaaagag actggtctaa aagattttaa gaaaggtttc 3540
   gatatgacct ctgctgtgcc atcaaatacg aaaaaaaagc tggtgattgc ttccgatcac 3600
   gcagcatttg agttgaaatc aaccttgatt acttggctga aagagcttgg tcatgaggtc 3660
   gaagaccttg gccctcatga aaaccattca gtcgattatc ccgattacgg ttataagctg 3720
   gctgtcgcta tcgcagaaaa aaccgctgat ttcggtattg ctttatgtgg ctcgggaatc 3780
   ggtatctcga tcgctgtcaa tcgccatccg gctgcccgtt gcgctttgat tacggataac 3840
   cttaccgccc gtttggcaag agaacataac aatgccaatg ttatcgctat gggtgcgaga 3900
   ttgatcggca ttgaaaccgc taaggattgt atttcagctt tccttgcaac gccgtttgga 3960
   ggtgaacgtc atgttcgccg tatcgataaa ctttcgaatc ctcagttcaa tatctagata 4020
   agttaggaga ataaacatga gtaaattacc cctgattgct ccctctatcc tttcggcgga 4080
   ttttgcccat ttgggagatg aggtcgcggc gatagatcag gccggtgccg attggatcca 4140
   tattgatgtg atggatggcc atttcgtgcc gaatatcacc ataggcccca tggttgtgaa 4200
   ggctttgcgt ccctatagcc aaaagccttt tgatgtccat ttgatgattg cgcctgtcga 4260
   tcaatatatc gaggcttttt ctgaagcggg tgctgatatt atcagtttcc atcccgaagc 4320
   gggcgcgcat ccccatcgca ctattcagca tatcaaatca ttgggcaaaa aagcgggatt 4380
   agtttttaat ccggcgaccc ctttaagctg gcttgattat ctaatggatg atcttgatct 4440
   gattatggtg atgagcgtta accccggttt tggcggccaa aaatttatca aaacccaatt 4500
   agaaaagatt aaagatatcc gtcaaagaat taccgcctct gggcgggata tccgcttgga 4560
   agtggatggc ggaattgatg ccacgactgc accgcttgcc gtcgaagccg gtgccgatgt 4620
   tttggtcgcg ggaacggcca gctttaaagg cggcgcaaca tgttacaccg ataatatcag 4680
   gatattgcgt aaatcatgat taattaactc gaggcggcct gaacgtactg caagtcctga 4740
   cgtcactgtg cagtccgttg gcccggttat cggtagcgat accgggcatt tttttaagga 4800
   acgatcgata gaattcgcgg gccggccaag cttgaattca tggttttggt gccaatgtta 4860
   tcgcctataa accgcatcca gaccccgaat tggcgaaaaa ggtcggtttc cgcttcacct 4920
   ctctcgatga agtgatcgag accagcgaca tcatttcgct tcactgtccg ctcacgccag 4980
   aaaatcatca catgattaat gaagaaacac tggcaagggc aaaaaaaggc ttttacctcg 5040
   tcaataccag tcgcggcggc ttggttgata ccaaggcggt gattaaatcg ctgaaagcca 5100
   aacatctcgg cggttatgcg gcggatgttt acgaagagga ggggccttta ttcttcgaaa 5160
   atcacgctga cgatattatc gaagatgata ttctcgaaag gttgatcgct ttcccgaatg 5220
   tggttttcac gggacatcag gcctttttga cgaaagaggc cttatcaaac attgctcaca 5280
   gtattctaca agatatcagc gatgccgaag ctggaaaaga aatgccggat gcgcttgttt 5340
   agtagacaag cgacaattaa ccttttgaag atcataatga tcaaattttt gggttaattc 5400
   ggtagttatg gcataggcta ttacgcgcta attgatatca aaaaaaagca tagccggaca 5460
   tcataccggc tatgtttttt attaggaaaa aatttccttt caccttgctt agccatcgcc 5520
   gcattattta atcaatatgc cgagtttttc ttgaaatccc tatcttacac caaggccaac 5580
   aagggaatca tccatactcg gtgtcctatc ctatgacttt ttaaattttc tccaaattta 5640
   ctaaaatcac gccatctcag cggctgctat tttcaaaaag cgcctctcaa aaccgctttt 5700
   tcctgctcaa atatcggatc ccaaaattcc ctcaaaaaag gcagggtatt ttttacaaaa 5760
   tcgcccctaa tatctctcaa tccgctgcct tgttcatatg tttttgcaaa tgatttttat 5820
   taaacttttt taggcgtatt tttatcaaga aaatttaaat aatcacattt ttattatttt 5880
   agatttaagt attgatacaa gtgatatcta taaatgtttt tataactttc tggatcgtaa 5940
   tcggctggca atcgttttcc ctatattcgc aagatgtatg tcagccgcgc ggccgcctaa 6000
   ttccggatga gcattcatca ggcgggcaag aatgtgaata aaggccggat aaaacttgtg 6060
   cttatttttc tttacggtct ttaaaaaggc cgtaatatcc agctgaacgg tctggttata 6120
   ggtacattga gcaactgact gaaatgcctc aaaatgttct ttacgatgcc attgggatat 6180
   atcaacggtg gtatatccag tgattttttt ctccatttta gcttccttag ctcctgaaaa 6240
   tctcgataac tcaaaaaata cgcccggtag tgatcttatt tcattatggt gaaagttgga 6300
   acctcttacg tgccgatcaa cgtctcattt tcgccaaaag ttggcccagg gcttcccggt 6360
   atcaacaggg acaccaggat ttatttattc tgcgaagtga tcttccgtca caggtattta 6420
   ttcggcgcaa agtgcgtcgg gtgatgctgc caacttactg atttagtgta tgatggtgtt 6480
   tttgaggtgc tccagtggct tctgtttcta tcagctgtcc ctcctgttca gctactgacg 6540
   gggtggtgcg taacggcaaa agcaccgccg gacatcagcg ctagcggagt gtatactggc 6600
   ttactatgtt ggcactgatg agggtgtcag tgaagtgctt catgtggcag gagaaaaaag 6660
   gctgcaccgg tgcgtcagca gaatatgtga tacaggatat attccgcttc ctcgctcact 6720
   gactcgctac gctcggtcgt tcgactgcgg cgagcggaaa tggcttacga acggggcgga 6780
   gatttcctgg aagatgccag gaagatactt aacagggaag tgagagggcc gcggcaaagc 6840
   cgtttttcca taggctccgc ccccctgaca agcatcacga aatctgacgc tcaaatcagt 6900
   ggtggcgaaa cccgacagga ctataaagat accaggcgtt tccccctggc ggctccctcg 6960
   tgcgctctcc tgttcctgcc tttcggttta ccggtgtcat tccgctgtta tggccgcgtt 7020
   tgtctcattc cacgcctgac actcagttcc gggtaggcag ttcgctccaa gctggactgt 7080
   atgcacgaac cccccgttca gtccgaccgc tgcgccttat ccggtaacta tcgtcttgag 7140
   tccaacccgg aaagacatgc aaaagcacca ctggcagcag ccactggtaa ttgatttaga 7200
   ggagttagtc ttgaagtcat gcgccggtta aggctaaact gaaaggacaa gttttggtga 7260
   ctgcgctcct ccaagccagt tacctcggtt caaagagttg gtagctcaga gaaccttcga 7320
   aaaaccgccc tgcaaggcgg ttttttcgtt ttcagagcaa gagattacgc gcagaccaaa 7380
   acgatctcaa gaagatcatc ttattaatca gataaaatat ttctagattt cagtgcaatt 7440
   tatctcttca aatgtagcac ctgaagtcag ccccatacga tataagttgt aattctcatg 7500
   tttgacagct tatcatcgat gtgacggaag atcacttcgc agaataaata aatcctggtg 7560
   tccctgttga taccgggaag ccctgggcca acttttggcg aaaatgagac gttgatcggc 7620
   acgtaagagg ttccaacttt caccataatg aaataagatc actaccgggc gtattttttg 7680
   agttatcgag attttcagga gctaaggaag ctaaaatgga gaaaaaaatc actggatata 7740
   ccaccgttga tatatcccaa tggcatcgta aagaacattt tgaggcattt cagtcagttg 7800
   ctcaatgtac ctataaccag accgttcagc tggatattac ggccttttta aagaccgtaa 7860
   agaaaaataa gcacaagttt tatccggcct ttattcacat tcttgcccgc ctgatgaatg 7920
   ctcatccgga attccgtatg gcaatgaaag acggtgagct ggtgatatgg gatagtgttc 7980
   acccttgtta caccgttttc catgagcaaa ctgaaacgtt ttcatcgctc tggagtgaat 8040
   accacgacga tttccggcag tttctacaca tatattcgca agatgtggcg tgttacggtg 8100
   aaaacctggc ctatttccct aaagggttta ttgagaatat gtttttcgtc tcagccaatc 8160
   cctgggtgag tttcaccagt tttgatttaa acgtggccaa tatggacaac ttcttcgccc 8220
   ccgttttcac catgggcaaa tattatacgc aaggcgacaa ggtgctgatg ccgctggcga 8280
   ttcaggttca tcatgccgtt tgtgatggct tccatgtcgg cagaatgctt aatgaattac 8340
   aacagtactg cgatgagtgg cagggcgggg cgtaattttt ttaaggcagt tattggtgcc 8400
   cttaaacgcc tggttgctac gcctgaataa gtcgaccttt gtagtcttgg cctgttgtgt 8460
   gcatgagcaa atcaatggca ccaccccctc ctttttgagc tgaatggtca taaaatttat 8520
   aattatctat cgtaattcgg aatctatgtt cagggtctcg ccattgcttt ttgtctgctg 8580
   ggtcaagttc catgcctaag gtttttaaga catcagaaag aggtattgca cgcatgctat 8640
   cagcttttct tctagctaat gacagggctt cctctgctct atctgctcgt tttttttctt 8700
   ccacatatct cgccgctttg tcagccagcg gctgtattac ggaaagtgcc gatttttggg 8760
   cttttaggcg ttctttttct gcccattctt ccttatttgt aaaaattgag ggtgggatgg 8820
   gtgcctgaat cttgggatct agctgtaaag ttttgttgat atttccgtaa tgtctttgga 8880
   ctctttgatg cgttgctttt gaacctttta cgcctctggc cagccctaga ggctccatag 8940
   aagccgcata atccgtctgg agggcagaaa gggcttttcg accatcaaac catctcgatg 9000
   cgtttaaacg gcctgtatcg gggtctctag gcaccataaa gccggttaag tggggtgttg 9060
   tttcatcagc atgtagctga agagatacaa ggttgttttc tccaaaggtt tgttccgccc 9120
   attgctgggt gattgttttc cagtgttcga gtttttcagg agtggcctgt tttgaccatt 9180
   ctggagacat accaaagaac agttctatgg cctgcacacc gttttttcta agaggctttc 9240
   ccgtttcttt ctgaatttta ttcagcatag atttaacatc tgctgatggg tcagtagagc 9300
   ctttgagtat ttcgtttagt tcttttctat ctgggtcagc gttttgtgtt tcgcggcctc 9360
   gcgtcatatg caggctcgcg gctttaatcg tgccaactgt tttatgtttt tcaaacctaa 9420
   agattgcata gttcggcatg ttttaactgc tttaatttga gaaaagacca gaggaaataa 9480
   tccagcctat atttctttcc ctagtagcga actggaattg tttttccgaa ggaaaaaagc 9540
   aattccgtag tgagtactga atttattctg attcgtcttg cttttggagc gtctttttgc 9600
   gttctataac tgttgtgaaa gctacgcggt cgccattgaa aacgaaatta ggattaataa 9660
   aataccatcc ttggcgaaca tgctttgcaa tgattttagc tttttctaat tcggctagac 9720
   ctcttgcaaa ggtagcttga gatagtgcca gttttttttc ttgtgcgtta agaaagtcct 9780
   ctaaaacgaa tttgtctaaa gggacgaggt ctttgctgat gcctttgtct tgaagtatcc 9840
   aaaccagaac gctgaaagct tttattccag cggctcctag ttcaaaagtt agcgcgatat 9900
   tggtgctaaa taattttaca aattcttcac tatcaacacg tctgtaagtc gtcacatgag 9960
   tgccttgcat ctcaccagtg gcttgattga ccagaatgtt atcatctcgt cctaatcgag 10020
   ataactgaac cctctgactt ttaactggca caaccatacc ttcgatgaaa ggattctcgt 10080
   catatctgat tggctgcttt ctcaattttg tcgccatatt tgataaacct ttaatcaaaa 10140
   aaaccacatt ttttgattat acctattcat cgaatgaggc aaggtctatc aattttaccc 10200
   ctttttttga tagacggttt aatcaatatt gatagacccc ttcacagatt ctgaaaatcg 10260
   acttccctat tttagggata ttttcacgat tccctttctt agttcttcct agtggggaaa 10320
   ttcgttgaat cctgcctcgg aaaaaccatg agaaagctgt tggttatata cacgggcaaa 10380
   gccaccctat ttttagctac tggggaaaga gataaggcag ggtatttgta aaattaaaac 10440
   cggatttttc gctttacggt ttgtttaggc gcaactgtct ttttaagacc gcgtttaacc 10500
   atcaaaagat cgttccaatc ttttccgtgt atcatctgtt ctttaggtgg gagccagttt 10560
   tcaacttttt ttgttggaaa cgcggcttta atcgctccga ctaatagcga tgctgctctt 10620
   tgtcctacag catcccaatc ataggcaata tggacagaag atgccttttc aacgattttt 10680
   cggagagttt tagtaagaga cgttcttacg ccgctggtgc ttaataattt tacgccagct 10740
   ttaatttttt ctgggcttaa aaagccgact actgaaatcg cgtctatcgc actttcagcg 10800
   atataaagat catacttttc gtcatttttt acattgatgc tgccagtaaa atgggcttcg 10860
   cgactgcttc ccaaggctaa ccctttaaaa ccactgcttg ttccgcgtaa ttctgcgccc 10920
   tgaagtgtat ctttatcgtc atacatcaag aaggctacat taccgcgatc atctgttcgg 10980
   atagagtcag gaatattgtt aaatgatatt cctcgggcag cgttgggtcc tggccacggg 11040
   tgcgcatgat cgtgctcctg tcgttgagga cccggctagg ctggcggggt tgccttactg 11100
   gttagcagaa tgaatcaccg atacgcgagc gaacgtgaag cgactgctgc tgcaaaacgt 11160
   ctgcgacctg agcaacaaca tgaatggtct tcggtttccg tgtttcgtaa agtctggaaa 11220
   cgcggaagtc ccctacgtgc tgctgaagtt gcccgcaaca gagagtggaa ccaaccggtg 11280
   ataccacgat actatgactg agagtcaacg ccatgggagc tcgtttttct atccccatca 11340
   cctcggtttt gttgacaaaa aaaggtggcc actaaattgg ctttccgcac cgatgggatg 11400
   atttttattc tttgctattc ttcgctcttt gcccaattca ttaaaagcgg aaatcatcac 11460
   caaagataga agacgcagcc ttcaccattt cagattgccc ttctcgggca ttttctgctg 11520
   ctagaatcct cttaaaaata ttaaattcca ctctattggt aatatgtttc cctctttagg 11580
   gaacaaataa agcccttctt tgttctataa aagttagctt accgatttta caaaaaataa 11640
   taccgcttca ttcaatcggt aatacatatc ttttttcttc aaaaaacttt tcaagagggt 11700
   gtctatgcgc gtcgcaatat tcagttccaa aaactatgac catcattcta ttgaaaaaga 11760
   aaatgaacat tatggccatg accttgtttt tctgaatgag cggcttacca aagagacagc 11820
   agaaaaagcc aaagacgcag aagctgtttg tatctttgtg aatgacgaag ccaatgccga 11880
   agtgctggaa attttggcag gcttaggcat caagttggtt gctcttcgtt gcgccggtta 11940
   taacaatgtc gatctcgatg cggccaaaaa gctgaatatc aaggttgtgc gcgtgcctgc 12000
   ctattcgccc tattcggttg ccgaatatgc agtagggatg ttgctcaccc tgaatcggca 12060
   aatttcacgc ggtttgaagc gggttcggga aaataacttc tccttggaag gtttgattgg 12120
   ccttgatgtg catgacaaaa cagtcggcat tatcggtgtt ggtcatatcg ggagcgtctt 12180
   tgcccatatt atgaccca 12198
<210> 23
   <211> 875
   <212> DNA
   <213> artificial sequence
<220>
   <223> constructed fragment for targeting integration into the ldh locus of Z. mobilis
<400> 23
   gtttttctat ccccatcacc tcggttttgt tgacaaaaaa aggtggccac taaattggct 60
   ttccgcaccg atgggatgat ttttattctt tgctattctt cgctctttgc ccaattcatt 120
   aaaagcggaa atcatcacca aagatagaag acgcagcctt caccatttca gattgccctt 180
   ctcgggcatt ttctgctgct agaatcctct taaaaatatt aaattccact ctattggtaa 240
   tatgtttccc tctttaggga acaaataaag cccttctttg ttctataaaa gttagcttac 300
   cgattttaca aaaaataata ccgcttcatt caatcggtaa tacatatctt ttttcttcaa 360
   aaaacttttc aagagggtgt ctatgcgcgt cgcaatattc agttccaaaa actatgacca 420
   tcattctatt gaaaaagaaa atgaacatta tggccatgac cttgtttttc tgaatgagcg 480
   gcttaccaaa gagacagcag aaaaagccaa agacgcagaa gctgtttgta tctttgtgaa 540
   tgacgaagcc aatgccgaag tgctggaaat tttggcaggc ttaggcatca agttggttgc 600
   tcttcgttgc gccggttata acaatgtcga tctcgatgcg gccaaaaagc tgaatatcaa 660
   ggttgtgcgc gtgcctgcct attcgcccta ttcggttgcc gaatatgcag tagggatgtt 720
   gctcaccctg aatcggcaaa tttcacgcgg tttgaagcgg gttcgggaaa ataacttctc 780
   cttggaaggt ttgattggcc ttgatgtgca tgacaaaaca gtcggcatta tcggtgttgg 840
   tcatatcggg agcgtctttg cccatattat gaccc 875
<210> 24
   <211> 1149
   <212> DNA
   <213> artificial sequence
<220>
   <223> constructed fragment for integration into the Z. mobilis ldh locus
<400> 24
   atggttttgg tgccaatgtt atcgcctata aaccgcatcc agaccccgaa ttggcgaaaa      60
   aggtcggttt ccgcttcacc tctctcgatg aagtgatcga gaccagcgac atcatttcgc     120
   ttcactgtcc gctcacgcca gaaaatcatc acatgattaa tgaagaaaca ctggcaaggg     180
   caaaaaaagg cttttacctc gtcaatacca gtcgcggcgg cttggttgat accaaggcgg     240
   tgattaaatc gctgaaagcc aaacatctcg gcggttatgc ggcggatgtt tacgaagagg     300
   aggggccttt attcttcgaa aatcacgctg acgatattat cgaagatgat attctcgaaa     360
   ggttgatcgc tttcccgaat gtggttttca cgggacatca ggcctttttg acgaaagagg     420
   ccttatcaaa cattgctcac agtattctac aagatatcag cgatgccgaa gctggaaaag     480
   aaatgccgga tgcgcttgtt tagtagacaa gcgacaatta accttttgaa gatcataatg     540
   atcaaatttt tgggttaatt cggtagttat ggcataggct attacgcgct aattgatatc     600
   aaaaaaaagc atagccggac atcataccgg ctatgttttt tattaggaaa aaatttcctt     660
   tcaccttgct tagccatcgc cgcattattt aatcaatatg ccgagttttt cttgaaatcc     720
   ctatcttaca ccaaggccaa caagggaatc atccatactc ggtgtcctat cctatgactt     780
   tttaaatttt ctccaaattt actaaaatca cgccatctca gcggctgcta ttttcaaaaa     840
   gcgcctctca aaaccgcttt ttcctgctca aatatcggat cccaaaattc cctcaaaaaa     900
   ggcagggtat tttttacaaa atcgccccta atatctctca atccgctgcc ttgttcatat     960
   gtttttgcaa atgattttta ttaaactttt ttaggcgtat ttttatcaag aaaatttaaa    1020
   taatcacatt tttattattt tagatttaag tattgataca agtgatatct ataaatgttt    1080
   ttataacttt ctggatcgta atcggctggc aatcgttttc cctatattcg caagatgtat    1140
   gtcagccgc                                                            1149
<210> 25
   <211> 996
   <212> DNA
   <213> Zymomonas mobilis
<400> 25
   atgcgcgtcg caatattcag ttccaaaaac tatgaccatc attctattga aaaagaaaat 60
   gaacattatg gccatgacct tgtttttctg aatgagcggc ttaccaaaga gacagcagaa 120
   aaagccaaag acgcagaagc tgtttgtatc tttgtgaatg acgaagccaa tgccgaagtg 180
   ctggaaattt tggcaggctt aggcatcaag ttggttgctc ttcgttgcgc cggttataac 240
   aatgtcgatc tcgatgcggc caaaaagctg aatatcaagg ttgtgcgcgt gcctgcctat 300
   tcgccctatt cggttgccga atatgcagta gggatgttgc tcaccctgaa tcggcaaatt 360
   tcacgcggtt tgaagcgggt tcgggaaaat aacttctcct tggaaggttt gattggcctt 420
   gatgtgcatg acaaaacagt cggcattatc ggtgttggtc atatcgggag cgtctttgcc 480
   catattatga cccatggttt tggtgccaat gttatcgcct ataaaccgca tccagacccc 540
   gaattggcga aaaaggtcgg tttccgcttc acctctctcg atgaagtgat cgagaccagc 600
   gacatcattt cgcttcactg tccgctcacg ccagaaaatc atcacatgat taatgaagaa 660
   acactggcaa gggcaaaaaa aggcttttac ctcgtcaata ccagtcgcgg cggcttggtt 720
   gataccaagg cggtgattaa atcgctgaaa gccaaacatc tcggcggtta tgcggcggat 780
   gtttacgaag aggaggggcc tttattcttc gaaaatcacg ctgacgatat tatcgaagat 840
   gatattctcg aaaggttgat cgctttcccg aatgtggttt tcacgggaca tcaggccttt 900
   ttgacgaaag aggccttatc aaacattgct cacagtattc tacaagatat cagcgatgcc 960
   gaagctggaa aagaaatgcc ggatgcgctt gtttag 996
<210> 26
   <211> 3339
   <212> DNA
   <213> artificial sequence
<220>
   <223> constructed fragment containing a 304-bp T-mutant of the Z. mobilis GAP promoter (PgapT), a 954-bp E. coli Tal coding region (ECTal), a 1,992-bp E. coli Tkt coding region, and a 68-bp E. coli Tkt 3'UTR (ECTkt 3'UTR)
<400> 26
   gttcgatcaa caacccgaat cctatcgtaa tgatgttttg cccgatcagc ctcaatcgac      60
   aattttacgc gtttcgatcg aaacagggac gacaattggc tgggaacggt atactggaat     120
   aaatggtctt cgttatggta ttgatgtttt tggtgcatcg gccccggcga atgatctata     180
   tgctcatttc ggcttgaccg cagtcggcat cacgaacaag gtgttggccg cgatcgccgg     240
   taagtcggca cgttaaaaaa tagctatgga atataatagc tactaataag ttaggagaat     300
   aaacatgacg gacaaattga cctcccttcg tcagtacacc accgtagtgg ccgacactgg     360
   ggacatcgcg gcaatgaagc tgtatcaacc gcaggatgcc acaaccaacc cttctctcat     420
   tcttaacgca gcgcagattc cggaataccg taagttgatt gatgatgctg tcgcctgggc     480
   gaaacagcag agcaacgatc gcgcgcagca gatcgtggac gcgaccgaca aactggcagt     540
   aaatattggt ctggaaatcc tgaaactggt tccgggccgt atctcaactg aagttgatgc     600
   gcgcctttcc tatgacaccg aagcgtcaat tgcgaaagca aaacgcctga tcaaactcta     660
   caacgatgct ggtattagca acgatcgtat tctgatcaaa ctggcttcta cctggcaggg     720
   tatccgtgct gcagaacagc tggaaaaaga aggcatcaac tgtaacctga ccctgctgtt     780
   ctccttcgct caggctcgtg cttgtgcgga agcgggcgtg ttcctgatct cgccgtttgt     840
   tggccgtatt cttgactggt acaaagcgaa taccgataag aaagagtacg ctccggcaga     900
   agatccgggc gtggtttctg tatctgaaat ctaccagtac tacaaagagc acggttatga     960
   aaccgtggtt atgggcgcaa gcttccgtaa catcggcgaa attctggaac tggcaggctg    1020
   cgaccgtctg accatcgcac cggcactgct gaaagagctg gcggagagcg aaggggctat    1080
   cgaacgtaaa ctgtcttaca ccggcgaagt gaaagcgcgt ccggcgcgta tcactgagtc    1140
   cgagttcctg tggcagcaca accagggtcc aatggcagta gataaactgg cggaaggtat 1200
   ccgtaagttt gctattgacc aggaaaaact ggaaaaaatg atcggcgatc tgctgtaatc 1260
   tagacgatct ggagtcaaaa tgtcctcacg taaagagctt gccaatgcta ttcgtgcgct 1320
   gagcatggac gcagtacaga aagccaaatc cggtcacccg ggtgccccta tgggtatggc 1380
   tgacattgcc gaagtcctgt ggcgtgattt cctgaaacac aacccgcaga atccgtcctg 1440
   ggctgaccgt gaccgcttcg tgctgtccaa cggccacggc tccatgctga tctacagcct 1500
   gctgcacctc accggttacg atctgccgat ggaagaactg aaaaacttcc gtcagctgca 1560
   ctctaaaact ccgggtcacc cggaagtggg ttacaccgct ggtgtggaaa ccaccaccgg 1620
   tccgctgggt cagggtattg ccaacgcagt cggtatggcg attgcagaaa aaacgctggc 1680
   ggcgcagttt aaccgtccgg gccacgacat tgtcgaccac tacacctacg ccttcatggg 1740
   cgacggctgc atgatggaag gcatctccca cgaagtttgc tctctggcgg gtacgctgaa 1800
   gctgggtaaa ctgattgcat tctacgatga caacggtatt tctatcgatg gtcacgttga 1860
   aggctggttc accgacgaca ccgcaatgcg tttcgaagct tacggctggc acgttattcg 1920
   cgacatcgac ggtcatgacg cggcatctat caaacgcgca gtagaagaag cgcgcgcagt 1980
   gactgacaaa ccttccctgc tgatgtgcaa aaccatcatc ggtttcggtt ccccgaacaa 2040
   agccggtacc cacgactccc acggtgcgcc gctgggcgac gctgaaattg ccctgacccg 2100
   cgaacaactg ggctggaaat atgcgccgtt cgaaatcccg tctgaaatct atgctcagtg 2160
   ggatgcgaaa gaagcaggcc aggcgaaaga atccgcatgg aacgagaaat tcgctgctta 2220
   cgcgaaagct tatccgcagg aagccgctga atttacccgc cgtatgaaag gcgaaatgcc 2280
   gtctgacttc gacgctaaag cgaaagagtt catcgctaaa ctgcaggcta atccggcgaa 2340
   aatcgccagc cgtaaagcgt ctcagaatgc tatcgaagcg ttcggtccgc tgttgccgga 2400
   attcctcggc ggttctgctg acctggcgcc gtctaacctg accctgtggt ctggttctaa 2460
   agcaatcaac gaagatgctg cgggtaacta catccactac ggtgttcgcg agttcggtat 2520
   gaccgcgatt gctaacggta tctccctgca cggtggcttc ctgccgtaca cctccacctt 2580
   cctgatgttc gtggaatacg cacgtaacgc cgtacgtatg gctgcgctga tgaaacagcg 2640
   tcaggtgatg gtttacaccc acgactccat cggtctgggc gaagacggcc cgactcacca 2700
   gccggttgag caggtcgctt ctctgcgcgt aaccccgaac atgtctacat ggcgtccgtg 2760
   tgaccaggtt gaatccgcgg tcgcgtggaa atacggtgtt gagcgtcagg acggcccgac 2820
   cgcactgatc ctctcccgtc agaacctggc gcagcaggaa cgaactgaag agcaactggc 2880
   aaacatcgcg cgcggtggtt atgtgctgaa agactgcgcc ggtcagccgg aactgatttt 2940
   catcgctacc ggttcagaag ttgaactggc tgttgctgcc tacgaaaaac tgactgccga 3000
   aggcgtgaaa gcgcgcgtgg tgtccatgcc gtctaccgac gcatttgaca agcaggatgc 3060
   tgcttaccgt gaatccgtac tgccgaaagc ggttactgca cgcgttgctg tagaagcggg     3120
   tattgctgac tactggtaca agtatgttgg cctgaacggt gctatcgtcg gtatgaccac     3180
   cttcggtgaa tctgctccgg cagagctgct gtttgaagag ttcggcttca ctgttgataa     3240
   cgttgttgcg aaagcaaaag aactgctgta attagcattt cgggtaaaaa aggtcgcttc     3300
   ggcgaccttt tttattacct tgataatgtc cgtttgcgc                            3339
<210> 27
   <211> 304
   <212> DNA
   <213> artificial sequence
<220>
   <223> mutant Pgap promoter with a "G" to an "A" change at position 83 in SEQ ID NO:21
<400> 27
   gttcgatcaa caacccgaat cctatcgtaa tgatgttttg cccgatcagc ctcaatcgac       60
   aattttacgc gtttcgatcg aaacagggac gacaattggc tgggaacggt atactggaat      120
   aaatggtctt cgttatggta ttgatgtttt tggtgcatcg gccccggcga atgatctata      180
   tgctcatttc ggcttgaccg cagtcggcat cacgaacaag gtgttggccg cgatcgccgg      240
   taagtcggca cgttaaaaaa tagctatgga atataatagc tactaataag ttaggagaat      300
   aaac                                                                   304
<210> 28
   <211> 1443
   <212> DNA
   <213> artificial sequence
<220>
   <223> constructed fragment containing a 191 bp Peno, a 471 bp Z. mobilis Rpi coding sequence (ZMRpi), a 663 bp Z. mobilis Rpe coding sequence (ZMRpe), and a 35 bp E.coli xylA 3'UTR (ECxylA 3'UTR)
<400> 28
   ccagttactc aatacgtaac aataatcagt ttatcctaac tatagaatcg catgagaagc       60
   gataacgttt caccataagc aatatattca ttgcaacagt ggaattgcct tatgcgtcaa      120
   ggaaggatag atcattgacg gactgagttc aaaaagagac tggtctaaaa gattttaaga      180
   aaggtttcga tatgacctct gctgtgccat caaatacgaa aaaaaagctg gtgattgctt      240
   ccgatcacgc agcatttgag ttgaaatcaa ccttgattac ttggctgaaa gagcttggtc      300
   atgaggtcga agaccttggc cctcatgaaa accattcagt cgattatccc gattacggtt      360
   ataagctggc tgtcgctatc gcagaaaaaa ccgctgattt cggtattgct ttatgtggct      420
   cgggaatcgg tatctcgatc gctgtcaatc gccatccggc tgcccgttgc gctttgatta      480
   cggataacct taccgcccgt ttggcaagag aacataacaa tgccaatgtt atcgctatgg      540
   gtgcgagatt gatcggcatt gaaaccgcta aggattgtat ttcagctttc cttgcaacgc      600
   cgtttggagg tgaacgtcat gttcgccgta tcgataaact ttcgaatcct cagttcaata      660
   tctagataag ttaggagaat aaacatgagt aaattacccc tgattgctcc ctctatcctt      720
   tcggcggatt ttgcccattt gggagatgag gtcgcggcga tagatcaggc cggtgccgat      780
   tggatccata ttgatgtgat ggatggccat ttcgtgccga atatcaccat aggccccatg      840
   gttgtgaagg ctttgcgtcc ctatagccaa aagccttttg atgtccattt gatgattgcg      900
   cctgtcgatc aatatatcga ggctttttct gaagcgggtg ctgatattat cagtttccat      960
   cccgaagcgg gcgcgcatcc ccatcgcact attcagcata tcaaatcatt gggcaaaaaa     1020
   gcgggattag tttttaatcc ggcgacccct ttaagctggc ttgattatct aatggatgat     1080
   cttgatctga ttatggtgat gagcgttaac cccggttttg gcggccaaaa atttatcaaa     1140
   acccaattag aaaagattaa agatatccgt caaagaatta ccgcctctgg gcgggatatc     1200
   cgcttggaag tggatggcgg aattgatgcc acgactgcac cgcttgccgt cgaagccggt     1260
   gccgatgttt tggtcgcggg aacggccagc tttaaaggcg gcgcaacatg ttacaccgat     1320
   aatatcagga tattgcgtaa atcatgatta attaactcga ggcggcctga acgtactgca     1380
   agtcctgacg tcactgtgca gtccgttggc ccggttatcg gtagcgatac cgggcatttt     1440
   ttt                                                                   1443
<210> 29
   <211> 12704
   <212> DNA
   <213> artificial sequence
<220>
   <223> constructed plasmid
<400> 29
   ctagtgttcg atcaacaacc cgaatcctat cgtaatgatg ttttgcccga tcagcctcaa      60
   tcgacaattt tacgcgtttc gatcgaagca gggacgacaa ttggctggga acggtatact     120
   ggaataaatg gtcttcgtta tggtattgat gtttttggtg catcggcccc ggcgaatgat     180
   ctatatgctc atttcggctt gaccgcagtc ggcatcacga acaaggtgtt ggccgcgatc     240
   gccggtaagt cggcacgtta aaaaatagct atggaatata atagctacta ataagttagg     300
   agaataaaca tgacggacaa attgacctcc cttcgtcagt acaccaccgt agtggccgac     360
   actggggaca tcgcggcaat gaagctgtat caaccgcagg atgccacaac caacccttct     420
   ctcattctta acgcagcgca gattccggaa taccgtaagt tgattgatga tgctgtcgcc     480
   tgggcgaaac agcagagcaa cgatcgcgcg cagcagatcg tggacgcgac cgacaaactg     540
   gcagtaaata ttggtctgga aatcctgaaa ctggttccgg gccgtatctc aactgaagtt     600
   gatgcgcgtc tttcctatga caccgaagcg tcaattgcga aagcaaaacg cctgatcaaa 660
   ctctacaacg atgctggtat tagcaacgat cgtattctga tcaaactggc ttctacctgg 720
   cagggtatcc gtgctgcaga acagctggaa aaagaaggca tcaactgtaa cctgaccctg 780
   ctgttctcct tcgctcaggc tcgtgcttgt gcggaagcgg gcgtgttcct gatctcgccg 840
   tttgttggcc gtattcttga ctggtacaaa gcgaataccg ataagaaaga gtacgctccg 900
   gcagaagatc cgggcgtggt ttctgtatct gaaatctacc agtactacaa agagcacggt 960
   tatgaaaccg tggttatggg cgcaagcttc cgtaacatcg gcgaaattct ggaactggca 1020
   ggctgcgacc gtctgaccat cgcaccggca ctgctgaaag agctggcgga gagcgaaggg 1080
   gctatcgaac gtaaactgtc ttacaccggc gaagtgaaag cgcgtccggc gcgtatcact 1140
   gagtccgagt tcctgtggca gcacaaccag gatccaatgg cagtagataa actggcggaa 1200
   ggtatccgta agtttgctat tgaccaggaa aaactggaaa aaatgatcgg cgatctgctg 1260
   taatctagac gatctggagt caaaatgtcc tcacgtaaag agcttgccaa tgctattcgt 1320
   gcgctgagca tggacgcagt acagaaagcc aaatccggtc acccgggggc ccctatgggt 1380
   atggctgaca ttgccgaagt cctgtggcgt gatttcctga aacacaaccc gcagaatccg 1440
   tcctgggctg accgtgaccg cttcgtgctg tccaacggcc acggctccat gctgatctac 1500
   agcctgctgc acctcaccgg ttacgatctg ccgatggaag aactgaaaaa cttccgtcag 1560
   ctgcactcta aaactccggg tcacccggaa gtgggttaca ccgctggtgt ggaaaccacc 1620
   accggtccgc tgggtcaggg tattgccaac gcagtcggta tggcgattgc agaaaaaacg 1680
   ctggcggcgc agtttaaccg tccgggccac gacattgtcg accactacac ctacgccttc 1740
   atgggcgacg gctgcatgat ggaaggcatc tcccacgaag tttgctctct ggcgggtacg 1800
   ctgaagctgg gtaaactgat tgcattctac gatgacaacg gtatttctat cgatggtcac 1860
   gttgaaggct ggttcaccga cgacaccgca atgcgtttcg aagcttacgg ctggcacgtt 1920
   attcgcgaca tcgacggtca tgacgcggca tctatcaaac gcgcagtaga agaagcgcgc 1980
   gcagtgactg acaaaccttc cctgctgatg tgcaaaacca tcatcggttt cggttccccg 2040
   aacaaagccg gtacccacga ctcccacggt gcgccgctgg gcgacgctga aattgccctg 2100
   acccgcgaac aactgggctg gaaatatgcg ccgttcgaaa tcccgtctga aatctatgct 2160
   cagtgggatg cgaaagaagc aggccaggcg aaagaatccg catggaacga gaaattcgct 2220
   gcttacgcga aagcttatcc gcaggaagcc gctgaattta cccgccgtat gaaaggcgaa 2280
   atgccgtctg acttcgacgc taaagcgaaa gagttcatcg ctaaactgca ggctaatccg 2340
   gcgaaaatcg ccagccgtaa agcgtctcag aatgctatcg aagcgttcgg tccgctgttg 2400
   ccggaattcc tcggcggttc tgctgacctg gcgccgtcta acctgaccct gtggtctggt 2460
   tctaaagcaa tcaacgaaga tgctgcgggt aactacatcc actacggtgt tcgcgagttc 2520
   ggtatgaccg cgattgctaa cggtatctcc ctgcacggtg gcttcctgcc gtacacctcc 2580
   accttcctga tgttcgtgga atacgcacgt aacgccgtac gtatggctgc gctgatgaaa 2640
   cagcgtcagg tgatggttta cacccacgac tccatcggtc tgggcgaaga cgggccgact 2700
   caccagccgg ttgagcaggt cgcttctctg cgcgtaaccc cgaacatgtc tacatggcgt 2760
   ccgtgtgacc aggttgaatc cgcggtcgcg tggaaatacg gtgttgagcg tcaggacggc 2820
   ccgaccgcac tgatcctctc ccgtcagaac ctggcgcagc aggaacgaac tgaagagcaa 2880
   ctggcaaaca tcgcgcgcgg tggttatgtg ctgaaagact gcgccggtca gccggaactg 2940
   attttcatcg ctaccggttc agaagttgaa ctggctgttg ctgcctacga aaaactgact 3000
   gccgaaggcg tgaaagcgcg cgtggtgtcc atgtcgtcta ccgacgcatt tgacaagcag 3060
   gatgctgctt accgtgaatc cgtactgccg aaagcggtta ctgcacgcgt tgctgtagaa 3120
   gcgggtattg ctgactactg gtacaagtat gttggcctga acggtgctat cgtcggtatg 3180
   accaccttcg gtgaatctgc tccggcagag ctgctgtttg aagagttcgg cttcactgtt 3240
   gataacgttg ttgcgaaagc aaaagaactg ctgtaattag catttcgggt aaaaaaggtc 3300
   gcttcggcga ccttttttat taccttgata atgtccgttt gcgcggcgcg ccccagttac 3360
   tcaatacgta acaataatca gtttatccta actatagaat cgcatgagaa gcgataacgt 3420
   ttcaccataa gcaatatatt cattgcaaca gtggaattgc cttatgcgtc aaggaaggat 3480
   agatcattga cggactgagt tcaaaaagag actcgtctaa aagattttaa gaaaggtttc 3540
   gatatgacct ctgctgtgcc atcaaatacg aaaaaaaagc tggtgattgc ttccgatcac 3600
   gcagcatttg agttgaaatc aaccttgatt acttggctga aagagcttgg tcatgaggtc 3660
   gaagaccttg gccctcatga aaaccattca gtcgattatc ccgattacgg ttataagctg 3720
   gctgtcgcta tcgcagaaaa aaccgctgat ttcggtattg ctttatgtgg ctcgggaatc 3780
   ggtatctcga tcgctgtcaa tcgccatccg gctgcccgtt gcgctttgat tacggataac 3840
   cttaccgccc gtttggcaag agaacataac aatgccaatg ttatcgctat gggtgcgaga 3900
   ttgatcggca ttgaaaccgc taaggattgt atttcagctt tccttgcaac gccgtttgga 3960
   ggtgaacgtc atgttcgccg tatcgataaa ctttcgaatc ctcagttcaa tatctagata 4020
   agttaggaga ataaacatga gtaaattacc cctgattgct ccctctatcc tttcggcgga 4080
   ttttgcccat ttgggagatg aggtcgcggc gatagatcag gccggtgccg attggatcca 4140
   tattgatgtg atggatggcc atttcgtgcc gaatatcacc ataggcccca tggttgtgaa 4200
   ggctttgcgt ccctatagcc aaaagccttt tgatgtccat ttgatgattg cgcctgtcga 4260
   tcaatatatc gaggcttttt ctgaagcggg tgctgatatt atcagtttcc atcccgaagc 4320
   gggcgcgcat ccccatcgca ctattcagca tatcaaatca ttgggcaaaa aagcgggatt 4380
   agtttttaat ccggcgaccc ctttaagctg gcttgattat ctaatggatg atcttgatct 4440
   gattatggtg atgagcgtta accccggttt tggcggccaa aaatttatca aaacccaatt 4500
   agaaaagatt aaagatatcc gtcaaagaat taccgcctct gggcgggata tccgcttgga 4560
   agtggatggc ggaattgatg ccacgactgc accgcttgcc gtcgaagccg gtgccgatgt 4620
   tttggtcgcg ggaacggcca gctttaaagg cggcgcaaca tgttacaccg ataatatcag 4680
   gatattgcgt aaatcatgat taattaactc gaggcggcct gaacgtactg caagtcctga 4740
   cgtcactgtg cagtccgttg gcccggttat cggtagcgat accgggcatt tttttaagga 4800
   acgatcgata gaattcgcgg ccggcccggc aagacgtgat atggaaccgg aatttgctcc 4860
   ggcattcctg cgcaaagata gctaatatct ttcatatttt gtatcgaaaa aggagggtct 4920
   ttaaagatcc tccttttttt tgcataaaaa gaaggccata gaacaaacag tgataaagac 4980
   agtctcaaac tgtcttttta tagaaaatac cagaatattg tatctggggg aggatgcatg 5040
   gtcttaatcc ggaatacccc ggtcatgcac aggatgttag agcttttgcc tttatggcaa 5100
   aataaaccat ggctcgggaa tatctgcgct ttgatttttg taggatgtgc cttccttgtc 5160
   cgtagtatta ttgggcattt tttaccggca ggttatcctt tcgtgacctt tatgccgaca 5220
   atgcttgtgg ttactttcct ctttgggaca agaccgggta ttatcgcggc tattcttagc 5280
   ttgatggttg cgccttattt tatcgaagaa ggaagccgat ttaacggtgt attggtctgg 5340
   tttctttgcc tgctagaaac agtcactgat atgggattgg tgattgcgct acagcaaggt 5400
   aattaccgcc tccagaaaaa gcgtgcctat aatcagatgc tggctgaacg caatgagttg 5460
   ctgtttcatg aattacagca tcgcatttca aataacttac aggttattgc gtcattattg 5520
   cggatgcaaa gccgcagcat caccgatgaa aaagccaagg aagctattga tgcctctgtt 5580
   cgtcggattc atatgatcgg tgaattacag cgggcgcttt atattaaaaa cgggaatcag 5640
   cttggggcaa aattgatcct tgatcgcttg atcaaagagg tcattgcgtc cagtaatctc 5700
   ccgaacatcc gctataaaat agaagctgaa gacctgatct taccgtcaga tatggcaatc 5760
   cctttagcgc ttgtatctgc tgaatccgtt tcaaacgcgt tagagcatgg ctttaaaggc 5820
   gatcataaag acgcgtttat tgaaattaag cttcaaaaaa ttagcgggca aatcgaactt 5880
   accatttcca ataatggcaa acctcttccc caaggctttt cccttgaaaa ggtcgatagc 5940
   ttaggcctga aaattgcggc tatgtttgcc cgacaattca aaggaaaatt caccttaagt 6000
   aatcagccta accgttatgt ggtttctagc cttattttgc cttgcggtta ggcggccgcc 6060
   taattccgga tgagcattca tcaggcgggc aagaatgtga ataaaggccg gataaaactt 6120
   gtgcttattt ttctttacgg tctttaaaaa ggccgtaata tccagctgaa cggtctggtt 6180
   ataggtacat tgagcaactg actgaaatgc ctcaaaatgt tctttacgat gccattggga 6240
   tatatcaacg gtggtatatc cagtgatttt tttctccatt ttagcttcct tagctcctga 6300
   aaatctcgat aactcaaaaa atacgcccgg tagtgatctt atttcattat ggtgaaagtt 6360
   ggaacctctt acgtgccgat caacgtctca ttttcgccaa aagttggccc agggcttccc 6420
   ggtatcaaca gggacaccag gatttattta ttctgcgaag tgatcttccg tcacaggtat 6480
   ttattcggcg caaagtgcgt cgggtgatgc tgccaactta ctgatttagt gtatgatggt 6540
   gtttttgagg tgctccagtg gcttctgttt ctatcagctg tccctcctgt tcagctactg 6600
   acggggtggt gcgtaacggc aaaagcaccg ccggacatca gcgctagcgg agtgtatact 6660
   ggcttactat gttggcactg atgagggtgt cagtgaagtg cttcatgtgg caggagaaaa 6720
   aaggctgcac cggtgcgtca gcagaatatg tgatacagga tatattccgc ttcctcgctc 6780
   actgactcgc tacgctcggt cgttcgactg cggcgagcgg aaatggctta cgaacggggc 6840
   ggagatttcc tggaagatgc caggaagata cttaacaggg aagtgagagg gccgcggcaa 6900
   agccgttttt ccataggctc cgcccccctg acaagcatca cgaaatctga cgctcaaatc 6960
   agtggtggcg aaacccgaca ggactataaa gataccaggc gtttccccct ggcggctccc 7020
   tcgtgcgctc tcctgttcct gcctttcggt ttaccggtgt cattccgctg ttatggccgc 7080
   gtttgtctca ttccacgcct gacactcagt tccgggtagg cagttcgctc caagctggac 7140
   tgtatgcacg aaccccccgt tcagtccgac cgctgcgcct tatccggtaa ctatcgtctt 7200
   gagtccaacc cggaaagaca tgcaaaagca ccactggcag cagccactgg taattgattt 7260
   agaggagtta gtcttgaagt catgcgccgg ttaaggctaa actgaaagga caagttttgg 7320
   tgactgcgct cctccaagcc agttacctcg gttcaaagag ttggtagctc agagaacctt 7380
   cgaaaaaccg ccctgcaagg cggttttttc gttttcagag caagagatta cgcgcagacc 7440
   aaaacgatct caagaagatc atcttattaa tcagataaaa tatttctaga tttcagtgca 7500
   atttatctct tcaaatgtag cacctgaagt cagccccata cgatataagt tgtaattctc 7560
   atgtttgaca gcttatcatc gatgtgacgg aagatcactt cgcagaataa ataaatcctg 7620
   gtgtccctgt tgataccggg aagccctggg ccaacttttg gcgaaaatga gacgttgatc 7680
   ggcacgtaag aggttccaac tttcaccata atgaaataag atcactaccg ggcgtatttt 7740
   ttgagttatc gagattttca ggagctaagg aagctaaaat ggagaaaaaa atcactggat 7800
   ataccaccgt tgatatatcc caatggcatc gtaaagaaca ttttgaggca tttcagtcag 7860
   ttgctcaatg tacctataac cagaccgttc agctggatat tacggccttt ttaaagaccg 7920
   taaagaaaaa taagcacaag ttttatccgg cctttattca cattcttgcc cgcctgatga 7980
   atgctcatcc ggaattccgt atggcaatga aagacggtga gctggtgata tgggatagtg 8040
   ttcacccttg ttacaccgtt ttccatgagc aaactgaaac gttttcatcg ctctggagtg 8100
   aataccacga cgatttccgg cagtttctac acatatattc gcaagatgtg gcgtgttacg 8160
   gtgaaaacct ggcctatttc cctaaagggt ttattgagaa tatgtttttc gtctcagcca 8220
   atccctgggt gagtttcacc agttttgatt taaacgtggc caatatggac aacttcttcg 8280
   cccccgtttt caccatgggc aaatattata cgcaaggcga caaggtgctg atgccgctgg 8340
   cgattcaggt tcatcatgcc gtttgtgatg gcttccatgt cggcagaatg cttaatgaat 8400
   tacaacagta ctgcgatgag tggcagggcg gggcgtaatt tttttaaggc agttattggt 8460
   gcccttaaac gcctggttgc tacgcctgaa taagtcgacc tttgtagtct tggcctgttg 8520
   tgtgcatgag caaatcaatg gcaccacccc ctcctttttg agctgaatgg tcataaaatt 8580
   tataattatc tatcgtaatt cggaatctat gttcagggtc tcgccattgc tttttgtctg 8640
   ctgggtcaag ttccatgcct aaggttttta agacatcaga aagaggtatt gcacgcatgc 8700
   tatcagcttt tcttctagct aatgacaggg cttcctctgc tctatctgct cgtttttttt 8760
   cttccacata tctcgccgct ttgtcagcca gcggctgtat tacggaaagt gccgattttt 8820
   gggcttttag gcgttctttt tctgcccatt cttccttatt tgtaaaaatt gagggtggga 8880
   tgggtgcctg aatcttggga tctagctgta aagttttgtt gatatttccg taatgtcttt 8940
   ggactctttg atgcgttgct tttgaacctt ttacgcctct ggccagccct agaggctcca 9000
   tagaagccgc ataatccgtc tggagggcag aaagggcttt tcgaccatca aaccatctcg 9060
   atgcgtttaa acggcctgta tcggggtctc taggcaccat aaagccggtt aagtggggtg 9120
   ttgtttcatc agcatgtagc tgaagagata caaggttgtt ttctccaaag gtttgttccg 9180
   cccattgctg ggtgattgtt ttccagtgtt cgagtttttc aggagtggcc tgttttgacc 9240
   attctggaga cataccaaag aacagttcta tggcctgcac accgtttttt ctaagaggct 9300
   ttcccgtttc tttctgaatt ttattcagca tagatttaac atctgctgat gggtcagtag 9360
   agcctttgag tatttcgttt agttcttttc tatctgggtc agcgttttgt gtttcgcggc 9420
   ctcgcgtcat atgcaggctc gcggctttaa tcgtgccaac tgttttatgt ttttcaaacc 9480
   taaagattgc atagttcggc atgttttaac tgctttaatt tgagaaaaga ccagaggaaa 9540
   taatccagcc tatatttctt tccctagtag cgaactggaa ttgtttttcc gaaggaaaaa 9600
   agcaattccg tagtgagtac tgaatttatt ctgattcgtc ttgcttttgg agcgtctttt 9660
   tgcgttctat aactgttgtg aaagctacgc ggtcgccatt gaaaacgaaa ttaggattaa 9720
   taaaatacca tccttggcga acatgctttg caatgatttt agctttttct aattcggcta 9780
   gacctcttgc aaaggtagct tgagatagtg ccagtttttt ttcttgtgcg ttaagaaagt 9840
   cctctaaaac gaatttgtct aaagggacga ggtctttgct gatgcctttg tcttgaagta 9900
   tccaaaccag aacgctgaaa gcttttattc cagcggctcc tagttcaaaa gttagcgcga 9960
   tattggtgct aaataatttt acaaattctt cactatcaac acgtctgtaa gtcgtcacat 10020
   gagtgccttg catctcacca gtggcttgat tgaccagaat gttatcatct cgtcctaatc 10080
   gagataactg aaccctctga cttttaactg gcacaaccat accttcgatg aaaggattct 10140
   cgtcatatct gattggctgc tttctcaatt ttgtcgccat atttgataaa cctttaatca 10200
   aaaaaaccac attttttgat tatacctatt catcgaatga ggcaaggtct atcaatttta 10260
   cccctttttt tgatagacgg tttaatcaat attgatagac cccttcacag attctgaaaa 10320
   tcgacttccc tattttaggg atattttcac gattcccttt cttagttctt cctagtgggg 10380
   aaattcgttg aatcctgcct cggaaaaacc atgagaaagc tgttggttat atacacgggc 10440
   aaagccaccc tatttttagc tactggggaa agagataagg cagggtattt gtaaaattaa 10500
   aaccggattt ttcgctttac ggtttgttta ggcgcaactg tctttttaag accgcgttta 10560
   accatcaaaa gatcgttcca atcttttccg tgtatcatct gttctttagg tgggagccag 10620
   ttttcaactt tttttgttgg aaacgcggct ttaatcgctc cgactaatag cgatgctgct 10680
   ctttgtccta cagcatccca atcataggca atatggacag aagatgcctt ttcaacgatt 10740
   tttcggagag ttttagtaag agacgttctt acgccgctgg tgcttaataa ttttacgcca 10800
   gctttaattt tttctgggct taaaaagccg actactgaaa tcgcgtctat cgcactttca 10860
   gcgatataaa gatcatactt ttcgtcattt tttacattga tgctgccagt aaaatgggct 10920
   tcgcgactgc ttcccaaggc taacccttta aaaccactgc ttgttccgcg taattctgcg 10980
   ccctgaagtg tatctttatc gtcatacatc aagaaggcta cattaccgcg atcatctgtt 11040
   cggatagagt caggaatatt gttaaatgat attcctcggg cagcgttggg tcctggccac 11100
   gggtgcgcat gatcgtgctc ctgtcgttga ggacccggct aggctggcgg ggttgcctta 11160
   ctggttagca gaatgaatca ccgatacgcg agcgaacgtg aagcgactgc tgctgcaaaa 11220
   cgtctgcgac ctgagcaaca acatgaatgg tcttcggttt ccgtgtttcg taaagtctgg 11280
   aaacgcggaa gtcccctacg tgctgctgaa gttgcccgca acagagagtg gaaccaaccg 11340
   gtgataccac gatactatga ctgagagtca acgccatggg agctccctat cgtctgactc 11400
   gcaaggctga acgtgttgac gccttgagca aggccaaagc ggttcttgac gaagccttcc 11460
   cagaagctga tccgacagaa aagctgcgca tccagaagct tgcgaagaag ctggaagcaa 11520
   aaatcgtccg caccgccatt ctgaaagaag gccggagaat tgacggacgc gatctgaaaa 11580
   cagttcgccc gatccgctct caggttggat tcttgccccg cacgcatggt tctgccctgt 11640
   ttacgcgtgg tgaaacacag gctttggttt caaccaccct tggaacggcg gatgctgaac 11700
   agatgatcga cggtttaacc ggccttcatt atgaacgctt catgctgcat tacaacttcc 11760
   ccccatattc ggtcggtgaa gttggtcgtt ttggtgctcc gggtcgtcgt gaaatcggcc 11820
   atggtaaact ggcatggcgt gcgcttcatc cggttttgcc gagcaaggct gatttcccgt 11880
   ataccatccg tgttttgtcg gatatcaccg aatctaatgg ttcctcttcc atggcaaccg 11940
   tttgcggtgg ctgccttgca ttgatggatg ccggtgttcc cttaacgcgt ccggtttccg 12000
   gtatcgccat gggtcttatt ctggaaaaag acggcttcgc tattttgtcc gatatcatgg 12060
   gtgatgaaga tcacttgggt gatatggact ttaaggtcgc cggtaccgaa aaaggtatca 12120
   ccagcctcca gatggacatc aaggttgctg gcattaccga agaaatcatg cagaaagctt 12180
   tggaacaggc taaaggtggc cgtgctcata tcttgggtga aatgtccaaa gcgctgggtg 12240
   aagtccgctc cgaaatttct aatttggcac cgcgcattga aacaatgagc gtaccaaaag 12300
   acaaaatccg tgatgttatc ggaacgggcg gaaaagttat ccgtgaaatc gtggcgacca 12360
   caggtgccaa ggtcgatatc gaagatgacg gcacggttcg tctgtcttct tctgatccgg 12420
   ccaatattga agcagcccgt gaatggatca atggtattgt tgaagaaccg gaagtaggca 12480
   aaatctataa cggtaaagtc gtcaatatcg ttgatttcgg tgccttcgta aacttcatgg 12540
   gtggccgtga cggcttggta catgtttcgg aaatcaagaa cgaacgtgtc aacaaggtca 12600
   gcgatgtcct gtccgaaggt caggaagtca aagtcaaggt tcttgaaatt gacaaccgtg 12660
   gcaaggttcg cctgtctatg cgtgttgtcg atcaggaaac cgga 12704
<210> 30
   <211> 1318
   <212> DNA
   <213> artificial sequence
<220>
   <223> constructed fragment for integration into Z. m pnp gene
<400> 30
   cctatcgtct gactcgcaag gctgaacgtg ttgacgcctt gagcaaggcc aaagcggttc      60
   ttgacgaagc cttcccagaa gctgatccga cagaaaagct gcgcatccag aagcttgcga     120
   agaagctgga agcaaaaatc gtccgcaccg ccattctgaa agaaggccgg agaattgacg     180
   gacgcgatct gaaaacagtt cgcccgatcc gctctcaggt tggattcttg ccccgcacgc     240
   atggttctgc cctgtttacg cgtggtgaaa cacaggcttt ggtttcaacc acccttggaa     300
   cggcggatgc tgaacagatg atcgacggtt taaccggcct tcattatgaa cgcttcatgc     360
   tgcattacaa cttcccccca tattcggtcg gtgaagttgg tcgttttggt gctccgggtc     420
   gtcgtgaaat cggccatggt aaactggcat ggcgtgcgct tcatccggtt ttgccgagca     480
   aggctgattt cccgtatacc atccgtgttt tgtcggatat caccgaatct aatggttcct     540
   cttccatggc aaccgtttgc ggtggctgcc ttgcattgat ggatgccggt gttcccttaa     600
   cgcgtccggt ttccggtatc gccatgggtc ttattctgga aaaagacggc ttcgctattt     660
   tgtccgatat catgggtgat gaagatcact tgggtgatat ggactttaag gtcgccggta     720
   ccgaaaaagg tatcaccagc ctccagatgg acatcaaggt tgctggcatt accgaagaaa     780
   tcatgcagaa agctttggaa caggctaaag gtggccgtgc tcatatcttg ggtgaaatgt     840
   ccaaagcgct gggtgaagtc cgctccgaaa tttctaattt ggcaccgcgc attgaaacaa     900
   tgagcgtacc aaaagacaaa atccgtgatg ttatcggaac gggcggaaaa gttatccgtg     960
   aaatcgtggc gaccacaggt gccaaggtcg atatcgaaga tgacggcacg gttcgtctgt    1020
   cttcttctga tccggccaat attgaagcag cccgtgaatg gatcaatggt attgttgaag    1080
   aaccggaagt aggcaaaatc tataacggta aagtcgtcaa tatcgttgat ttcggtgcct    1140
   tcgtaaactt catgggtggc cgtgacggct tggtacatgt ttcggaaatc aagaacgaac    1200
   gtgtcaacaa ggtcagcgat gtcctgtccg aaggtcagga agtcaaagtc aaggttcttg    1260
   aaattgacaa ccgtggcaag gttcgcctgt ctatgcgtgt tgtcgatcag gaaaccgg      1318
<210> 31
   <211> 1225
   <212> DNA
   <213> artificial sequence
<220>
   <223> constructed fragment for integration into the Z. m pnp gene
<400> 31
   cggcaagacg tgatatggaa ccggaatttg ctccggcatt cctgcgcaaa gatagctaat      60
   atctttcata ttttgtatcg aaaaaggagg gtctttaaag atcctccttt tttttgcata     120
   aaaagaaggc catagaacaa acagtgataa agacagtctc aaactgtctt tttatagaaa     180
   ataccagaat attgtatctg ggggaggatg catggtctta atccggaata ccccggtcat      240
   gcacaggatg ttagagcttt tgcctttatg gcaaaataaa ccatggctcg ggaatatctg     300
   cgctttgatt tttgtaggat gtgccttcct tgtccgtagt attattgggc attttttacc     360
   ggcaggttat cctttcgtga cctttatgcc gacaatgctt gtggttactt tcctctttgg     420
   gacaagaccg ggtattatcg cggctattct tagcttgatg gttgcgcctt attttatcga     480
   agaaggaagc cgatttaacg gtgtattggt ctggtttctt tgcctgctag aaacagtcac     540
   tgatatggga ttggtgattg cgctacagca aggtaattac cgcctccaga aaaagcgtgc     600
   ctataatcag atgctggctg aacgcaatga gttgctgttt catgaattac agcatcgcat     660
   ttcaaataac ttacaggtta ttgcgtcatt attgcggatg caaagccgca gcatcaccga     720
   tgaaaaagcc aaggaagcta ttgatgcctc tgttcgtcgg attcatatga tcggtgaatt     780
   acagcgggcg ctttatatta aaaacgggaa tcagcttggg gcaaaattga tccttgatcg     840
   cttgatcaaa gaggtcattg cgtccagtaa tctcccgaac atccgctata aaatagaagc     900
   tgaagacctg atcttaccgt cagatatggc aatcccttta gcgcttgtat ctgctgaatc     960
   cgtttcaaac gcgttagagc atggctttaa aggcgatcat aaagacgcgt ttattgaaat    1020
   taagcttcaa aaaattagcg ggcaaatcga acttaccatt tccaataatg gcaaacctct    1080
   tccccaaggc ttttcccttg aaaaggtcga tagcttaggc ctgaaaattg cggctatgtt    1140
   tgcccgacaa ttcaaaggaa aattcacctt aagtaatcag cctaaccgtt atgtggtttc    1200
   tagccttatt ttgccttgcg gttag                                          1225
<210> 32
   <211> 25
   <212> DNA
   <213> artificial sequence
<220>
   <223> primer
<400> 32
   cggcttcaat cggattgtta gcagg 25
<210> 33
   <211> 38
   <212> DNA
   <213> artificial sequence
<220>
   <223> primer
<400> 33
   cgtgtagctt ggacactcat gtttattctc ctaactta 38
<210> 34
   <211> 20
   <212> DNA
   <213> artificial sequence
<220>
   <223> synthetic primer
<400> 34
   ccagtatcag cccgtcatac 20
<210> 35
   <211> 20
   <212> DNA
   <213> artificial sequence
<220>
   <223> synthetic primer
<400> 35
   ccagcatggt tgtgatggct 20
<210> 36
   <211> 20
   <212> DNA
   <213> artificial sequence
<220>
   <223> synthetic primer
<400> 36
   gccttgggct tttaaagcct 20
<210> 37
   <211> 22
   <212> DNA
   <213> artificial sequence
<220>
   <223> synthetic primer
<400> 37
   gagaagggtt ggttgtggca tc 22
<210> 38
   <211> 22
   <212> DNA
   <213> artificial sequence
<220>
   <223> synthetic primer
<400> 38
   gtggatggcg gaattgatgc ca 22
<210> 39
   <211> 26
   <212> DNA
   <213> artificial sequence
<220>
   <223> synthetic primer
<400> 39
   tctcggagag atagaggtca gtcgac 26
<210> 40
   <211> 23
   <212> DNA
   <213> artificial sequence
<220>
   <223> synthetic primer
<400> 40
   cagctatgat gacagcgcat tgg 23
<210> 41
   <211> 23
   <212> DNA
   <213> artificial sequence
<220>
   <223> synthetic primer
<400> 41
   gggcggttcg atccatagaa agg 23
<210> 42
   <211> 2247
   <212> DNA
   <213> Zymomonas mobilis
<400> 42
   atgttcgata ttaaacgcca ggaaatcgat tggggcggaa aaaaactgac actggaaacc 60
   ggacaggttg cccgtcaggc agatggcgcc gtcattgcga ccttaggtga aacggtcgta 120
   ttatgcgcgg taacggcagc aaaaacggta aaagaaggtc aggatttctt tcctttgacc 180
   gtccattatc aggaaaaata ttcagcagca ggccgtattc ccggtggctt tttcaagcgt 240
   gaacgtggcg caaccgaacg ggaaacgctg atttcacggt taatcgaccg tccaatccgt 300
   cctctgtttc cggaaggttt ctataacgaa accttggtca ttgcgcaggt catgtcctat 360
   gacggcgaga atgaaccgga tatcttggcg atgatcgctg cttctgcggc tcttgctctt 420
   tccggtgtgc ctttcttggg ccccatcggt gctgcccgtg tgggttatca agatggcgag 480
   ttcattctta acccgacctt ggaacagctt gaaaaaagtg atcttgatct ggttgtcggg 540
   gctacccgtg atgccgtgat gatggttgaa tcggaagcga atgagcttcc cgaagaagtc 600
   atgctcaatg ccgtttcttt tgcgcatgaa tctttacagc cggttatcaa agctatcatc 660
   aatctggcag aacaggccgc taaagagcct tgggaactgg tcagctatga tgacagcgca 720
   ttggctgcca aagtcgaaga actctgctac gacaatttcg ataaggccta tcgtctgact 780
   cgcaaggctg aacgtgttga cgccttgagc aaggccaaag cggttcttga cgaagccttc 840
   ccagaagctg atccgacaga aaagctgcgc atccagaagc ttgcgaagaa gctggaagca 900
   aaaatcgtcc gcaccgccat tctgaaagaa ggccggagaa ttgacggacg cgatctgaaa 960
   acagttcgcc cgatccgctc tcaggttgga ttcttgcccc gcacgcatgg ttctgccctg 1020
   tttacgcgtg gtgaaacaca ggctttggtt tcaaccaccc ttggaacggc ggatgctgaa 1080
   cagatgatcg acggtttaac cggccttcat tatgaacgct tcatgctgca ttacaacttc 1140
   cccccatatt cggtcggtga agttggtcgt tttggtgctc cgggtcgtcg tgaaatcggc 1200
   catggtaaac tggcatggcg tgcgcttcat ccggttttgc cgagcaaggc tgatttcccg 1260
   tataccatcc gtgttttgtc ggatatcacc gaatctaatg gttcctcttc catggcaacc 1320
   gtttgcggtg gctgccttgc attgatggat gccggtgttc ccttaacgcg tccggtttcc 1380
   ggtatcgcca tgggtcttat tctggaaaaa gacggcttcg ctattttgtc cgatatcatg 1440
   ggtgatgaag atcacttggg tgatatggac tttaaggtcg ccggtaccga aaaaggtatc 1500
   accagcctcc agatggacat caaggttgct ggcattaccg aagaaatcat gcagaaagct 1560
   ttggaacagg ctaaaggtgg ccgtgctcat atcttgggtg aaatgtccaa agcgctgggt 1620
   gaagtccgct ccgaaatttc taatttggca ccgcgcattg aaacaatgag cgtaccaaaa 1680
   gacaaaatcc gtgatgttat cggaacgggc ggaaaagtta tccgtgaaat cgtggcgacc 1740
   acaggtgcca aggtcgatat cgaagatgac ggcacggttc gtctgtcttc ttctgatccg 1800
   gccaatattg aagcagcccg tgaatggatc aatggtattg ttgaagaacc ggaagtaggc 1860
   aaaatctata acggtaaagt cgtcaatatc gttgatttcg gtgccttcgt aaacttcatg 1920
   ggtggccgtg acggcttggt acatgtttcg gaaatcaaga acgaacgtgt caacaaggtc 1980
   agcgatgtcc tgtccgaagg tcaggaagtc aaagtcaagg ttcttgaaat tgacaaccgt 2040
   ggcaaggttc gcctgtctat gcgtgttgtc gatcaggaaa ccggcgcaga gctggatgat 2100
   aaccgtccgc cacgtgagaa cgcagaacgt cgcggtggtg agcgtcctcg tcgtgatcgg 2160
   ggccctcgtc gggaatctgg cgatcgtccg gcaagacgtg atatggaacc ggaatttgct 2220
   ccggcattcc tgcgcaaaga tagctaa 2247
<210> 43
   <211> 474
   <212> DNA
   <213> artificial sequence
<220>
   <223> start codon changed to ATG
<400> 43
   atgacctctg ctgtgccatc aaatacgaaa aaaaagctgg tgattgcttc cgatcacgca 60
   gcatttgagt tgaaatcaac cttgattact tggctgaaag agcttggtca tgaggtcgaa 120
   gaccttggcc ctcatgaaaa ccattcagtc gattatcccg attacggtta taagctggct 180
   gtcgctatcg cagaaaaaac cgctgatttc ggtattgctt tatgtggctc gggaatcggt 240
   atctcgatcg ctgtcaatcg ccatccggct gcccgttgcg ctttgattac ggataacctt 300
   accgcccgtt tggcaagaga acataacaat gccaatgtta tcgctatggg tgcgagattg 360
   atcggcattg aaaccgctaa ggattgtatt tcagctttcc ttgcaacgcc gtttggaggt 420
   gaacgtcatg ttcgccgtat cgataaactt tcgaatcctc agttcaatat ctag 474
<210> 44
   <211> 39
   <212> DNA
   <213> artificial sequence
<220>
   <223> primer
<400> 44
   catcttactg cggccgcgtg acggaagatc acttcgcag 39
<210> 45
   <211> 37
   <212> DNA
   <213> artificial sequence
<220>
   <223> primer
<400> 45
   tcactcattt aattaactta ttcaggcgta gcaccag 37
<210> 46
   <211> 1002
   <212> DNA
   <213> artificial sequence
<220>
   <223> constructed fragment that contains a Cmr-cassette that is flanked by two wild type loxP sites
<400> 46
   ccataacttc gtatagcata cattatacga agttatttaa ttaacttatt caggcgtagc      60
   aaccaggcgt ttaagggcac caataactgc cttaaaaaaa ttacgccccg ccctgccact     120
   catcgcagta ctgttgtaat tcattaagca ttctgccgac atggaagcca tcacaaacgg     180
   catgatgaac ctgaatcgcc agcggcatca gcaccttgtc gccttgcgta taatatttgc     240
   ccatggtgaa aacgggggcg aagaagttgt ccatattggc cacgtttaaa tcaaaactgg     300
   tgaaactcac ccagggattg gctgagacga aaaacatatt ctcaataaac cctttaggga     360
   aataggccag gttttcaccg taacacgcca catcttgcga atatatgtgt agaaactgcc     420
   ggaaatcgtc gtggtattca ctccagagcg atgaaaacgt ttcagtttgc tcatggaaaa     480
   cggtgtaaca agggtgaaca ctatcccata tcaccagctc accgtctttc attgccatac     540
   ggaattccgg atgagcattc atcaggcggg caagaatgtg aataaaggcc ggataaaact     600
   tgtgcttatt tttctttacg gtctttaaaa aggccgtaat atccagctga acggtctggt     660
   tataggtaca ttgagcaact gactgaaatg cctcaaaatg ttctttacga tgccattggg     720
   atatatcaac ggtggtatat ccagtgattt ttttctccat tttagcttcc ttagctcctg     780
   aaaatctcga taactcaaaa aatacgcccg gtagtgatct tatttcatta tggtgaaagt     840
   tggaacctct tacgtgccga tcaacgtctc attttcgcca aaagttggcc cagggcttcc     900
   cggtatcaac agggacacca ggatttattt attctgcgaa gtgatcttcc gtcacgcggc     960
   cgcataactt cgtatagcat acattatacg aagttatgcg at                       1002
<210> 47
   <211> 36
   <212> DNA
   <213> artificial sequence
<220>
   <223> primer
<400> 47
   ctactcatcc tgcaggcttc tcggtgatcg tgttgc 36
<210> 48
   <211> 38
   <212> DNA
   <213> Artificial sequence
<220>
   <223> primer
<400> 48
   tcactcatgg ccggccgaac agatcgacgg tattgatg 38
<210> 49
   <211> 37
   <212> DNA
   <213> Artificial sequence
<220>
   <223> primer
<400> 49
   catcttactg cgatcgcgat caatcgcccg atgaatg 37
<210> 50
   <211> 36
   <212> DNA
   <213> Artificial sequence
<220>
   <223> primer
<400> 50
   catcttactg gcgcgcctcg ccgtattgta tcgctg 36
<210> 51
   <211> 20
   <212> DNA
   <213> Artificial sequence
<220>
   <223> primer
<400> 51
   ctacttcact tcatgaccgg 20
<210> 52
   <211> 35
   <212> DNA
   <213> Artificial sequence
<220>
   <223> primer
<400> 52
   agtcatgcag gcctctgatg aatgctcatc cggaa 35
<210> 53
   <211> 20
   <212> DNA
   <213> Artificial sequence
<220>
   <223> primer
<400> 53
   gtctgacgtt gatcctgatc 20
<210> 54
   <211> 38
   <212> DNA
   <213> artificial sequence
<220>
   <223> primer
<400> 54
   tcactcatgg ccggcctgcg tataatattt gcccatgg 38
<210> 55
   <211> 21
   <212> DNA
   <213> artificial sequence
<220>
   <223> primer
<400> 55
   gttcctgctt tgcttttgtg g 21
<210> 56
   <211> 22
   <212> DNA
   <213> artificial sequence
<220>
   <223> primer
<400> 56
   cccggaagct atcaaaattt tg 22
<210> 57
   <211> 18
   <212> DNA
   <213> artificial sequence
<220>
   <223> primer
<400> 57
   caccgtagtg gccgacac 18
<210> 58
   <211> 21
   <212> DNA
   <213> artificial sequence
<220>
   <223> primer
<400> 58
   gatggttttg cacatcagca g 21
<210> 59
   <211> 20
   <212> DNA
   <213> artificial sequence
<220>
   <223> primer
<400> 59
   tctgcaccga taggattggg 20
<210> 60
   <211> 21
   <212> DNA
   <213> artificial sequence
<220>
   <223> primer
<400> 60
   gatgtctttg tctatttcgc g 21
<210> 61
   <211> 20
   <212> DNA
   <213> artificial sequence
<220>
   <223> primer
<400> 61
   ccaacgactt cttcagcatg 20
<210> 62
   <211> 20
   <212> DNA
   <213> artificial sequence
<220>
   <223> primer
<400> 62
   cccaatccta tcggtgcaga 20
<210> 63
   <211> 21
   <212> DNA
   <213> artificial sequence
<220>
   <223> primer
<400> 63
   cgcgaaatag acaaagacat c 21
<210> 64
   <211> 20
   <212> DNA
   <213> artificial sequence
<220>
   <223> primer
<400> 64
   catgctgaag aagtcgttgg 20
<210> 65
   <211> 23
   <212> DNA
   <213> artificial sequence
<220>
   <223> primer
<400> 65
   ccaaacaagc ttgcatagtt gcc 23
<210> 66
   <211> 20
   <212> DNA
   <213> artificial sequence
<220>
   <223> primer
<400> 66
   gcagatggtg gttgagcata 20
<210> 67
   <211> 20
   <212> DNA
   <213> artificial sequence
<220>
   <223> primer
<400> 67
   tgccattttg tggaacgacg 20
<210> 68
   <211> 19
   <212> DNA
   <213> artificial sequence
<220>
   <223> primer
<400> 68
   tgcagttggt gtgggaatg 19
<210> 69
   <211> 21
   <212> DNA
   <213> artificial sequence
<220>
   <223> primer
<400> 69
   ggtttgactc atcaacatgg c 21
<210> 70
   <211> 20
   <212> DNA
   <213> artificial sequence
<220>
   <223> primer
<400> 70
   cgtcgttcca caaaatggca 20
<210> 71
   <211> 19
   <212> DNA
   <213> artificial sequence
<220>
   <223> primer
<400> 71
   cattcccaca ccaactgca 19
<210> 72
   <211> 21
   <212> DNA
   <213> artificial sequence
<220>
   <223> primer
<400> 72
   gccatgttga tgagtcaaac c 21